# EUROPEAN PATENT SPECIFICATION

(11) **EP 4 262 883 B1**
(45) Date of publication and mention of the grant of the patent: **28.05.2025**
(21) Application number: 21907979.5
(22) Date of filing: 20.12.2021
(51) Int. Cl.: A61K 48/00, C12N 15/09, C12N 15/64

(54) **PEG LIPIDS AND LIPID NANOPARTICLES**
PEG-LIPIDE UND LIPIDNANOPARTIKEL
LIPIDES PEG ET NANOPARTICULES LIPIDIQUES

(30) Priority: 18.12.2020 US 202063127684 P
(43) Date of publication of application: 25.10.2023
(73) Proprietor: GENEVANT SCIENCES GMBH, 4051 Basel (CH)
(72) Inventor: HEYES, James, Cambridge, Massachusetts 02142 (US); HOLLAND, Richard, J., Cambridge, Massachusetts 02142 (US); LAM, Kieu, Mong, Cambridge, Massachusetts 02142 (US); MARTIN, Alan, D., Cambridge, Massachusetts 02142 (US); WOOD, Mark, Cambridge, Massachusetts 02142 (US); ZHANG, Wenxuan, Cambridge, Massachusetts 02142 (US)
(74) Representative: J A Kemp LLP
(86) International application number: PCT/US2021/064351
(87) International publication number: WO 2022/133344

(56) References cited:
- WO-A1-2016/118697
- WO-A1-2020/219941
- WO-A1-2020/219941
- US-B2- 7 803 397
- DATABASE Pubchem 27 March 2018 (2018-03-27), ANONYMOUS : "SUBSTANCE RECORD 10289-94-8", XP055953160, Database accession no. SID 355127355

## Description

### BACKGROUND

Lipid nanoparticles (LNPs) are effective drug delivery systems for biologically active compounds, such as therapeutic nucleic acids, proteins, and peptides, which are otherwise cell impermeable. Drugs based on nucleic acids, which include large nucleic acid molecules such as, *e.g., in vitro* transcribed messenger RNA (mRNA) as well as smaller polynucleotides that interact with a messenger RNA or a gene, have to be delivered to the proper cellular compartment in order to be effective. For example, double-stranded nucleic acids such as double-stranded RNA molecules (dsRNA), including, *e.g*., siRNAs, suffer from their physico-chemical properties that render them impermeable to cells. Upon delivery into the proper compartment, siRNAs block gene expression through a highly conserved regulatory mechanism known as RNA interference (RNAi). Typically, siRNAs are large in size with a molecular weight ranging from 12-17 kDa and are highly anionic due to their phosphate backbone with up to 50 negative charges. In addition, the two complementary RNA strands result in a rigid helix. These features contribute to the siRNA's poor "drug-like" properties. When administered intravenously, the siRNA is rapidly excreted from the body with a typical half-life in the range of only 10 minutes. Additionally, siRNAs are rapidly degraded by nucleases present in blood and other fluids or in tissues and have been shown to stimulate strong immune responses *in vitro* and *in vivo. See, e.g.,* Robbins et al., Oligonucleotides 19:89-102, 2009. mRNA molecules suffer from similar issues of impermeability, fragility, and immunogenicity. See International Patent Application Publication Number WO2016/118697.

WO2020/219941A1 discloses formulations containing lipid nanoparticles derived by PEG.

Lipid nanoparticle formulations have improved nucleic acid delivery *in vivo.* For example, such formulations can have significantly reduced siRNA doses necessary to achieve target knockdown *in vivo. see* Zimmermann et al., Nature 441:111-114, 2006. Typically, such lipid nanoparticle drug delivery systems are multi-component formulations comprising cationic (or ionizable) lipids, helper lipids, and lipids containing polyethylene glycol. It should be noted that the terms 'cationic' and 'ionizable' as they relate to lipids herein, unless otherwise described, are used interchangeably. The positively charged cationic lipids bind to the anionic nucleic acid, while the other components support a stable self-assembly of the lipid nanoparticles.

Efforts have been directed toward improving delivery efficacy of lipid nanoparticle formulations. Many such efforts have been aimed toward developing more appropriate cationic lipids. *See, e.g.,* Akinc et al., Nature Biotechnology 26:561-569, 2008; Love et al., Proc. Natl. Acad. Sci. USA 107:1864-1869, 2010; Baigude et al., Journal of Controlled Release 107:276-287, 2005; Semple et al., Nature Biotechnology 28:172-176, 2010. Further, the PEG lipid PEG2000-C-DMA has been used in LNP formulations that have entered human clinical trials in applications as diverse as oncology, vaccines, antivirals and metabolic diseases. These LNP formulations have been exploited to deliver therapeutic payloads including, but not limited to, oligonucleic acids such as plasmid DNA, siRNA, mRNA and self-replicating RNA. Despites these efforts, there remains a need for lipid nanoparticle containing formulations that provide higher potency following administration and that allow for the administration of lower doses of nucleic acids, such as mRNA and siRNA. For example, there is a need for additional PEG lipids having, e.g., unique or improved properties for use in lipid nanoparticle formulations.

### SUMMARY

Accordingly, provided here is a compound of formula (I): 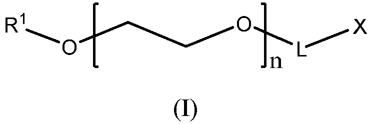 or a salt thereof, wherein:
R¹ is H, (C₁-C₆)alkyl, or (C₁-C₆)alkanoyl;
n is an integer in the range of from about 10 to about 150; and
L is absent and X is -C(=O)NR²R³; or
L is (C₁-C₆)alkyl and X is selected from the group consisting of -N(R⁴)C(=O)CH(R²)(R³), -OCH(R²)(R³), -C(=O)OCH₂CH(R²)(R³), -N(R⁴)C(=O)N(R²)(R³), and -SO₂N(R²)(R³);
R² is (C₁₀-C₂₀)alkyl;
R³ is (C₁₀-C₂₀)alkyl; and
R⁴ is H or (C₁-C₆)alkyl.

This compound can be used as a component of a lipid nanoparticle useful, *e.g.,* for delivering therapeutic agents such as siRNA and mRNA.

Provided herewith are lipid nanoparticles and pharmaceutical compositions comprising the lipid nanoparticles. The lipid nanoparticles and pharmaceutical compositions are particularly useful for delivering a nucleic acid to a patient (*e.g.,* a human) or to a cell.

The invention also provides a pharmaceutical composition comprising a lipid nanoparticle of the invention, and a pharmaceutically acceptable carrier.

The invention also provides methods of administering nucleic acids to a living cell, *in vitro.*

The invention also provides a lipid nanoparticle of the invention wherein the therapeutic agent is mRNA, for use in a method for treating a disease characterized by a deficiency of a functional protein (*e.g*., caused by a genetic defect), the method comprising: administering to a subject having the disease, the lipid nanoparticle of the invention, wherein the mRNA encodes the functional protein or a protein having the same biological activity as the functional protein.

The invention also provides a lipid nanoparticle of the invention wherein the therapeutic agent is siRNA, for use in a method for treating a disease characterized by overexpression of a polypeptide, comprising administering to a subject having the disease the lipid nanoparticle of the invention, wherein the siRNA targets expression of the overexpressed polypeptide.

Also described herein are processes and intermediates that are useful for making lipid nanoparticles of the invention.

### BRIEF DESCRIPTION OF THE FIGURES

Figure 1. Figure 1 depicts results of anti-PEG antibody analysis, with Day 0, Day 7 and Day 14 results depicted from left to right for each compound.
Figure 2. Figure 2 depicts results of anti-PEG antibody analysis, with Day 0, Day 7 and Day 14 results depicted from left to right for each compound.
Figure 3. Figure 3 depicts results of EPO expression analysis, with Day 0, Day 7 and Day 14 results depicted from left to right for each compound.
Figure 4. Figure 4 depicts results of lipid clearance analysis. PEG-C-DMA is middle trace at the 90 minute point, Compound 9 is lower trace at the 90 minute point, and Compound 31 is upper trace at the 90 minute point.
Figure 5. Figure 5 depicts results of OTC expression analysis.
Figure 6. Figure 6 depicts results of anti-OVA IgG analysis, with Day 0, 7, 14, 21, 28, and 35 results depicted from left to right for each compound.

### DETAILED DESCRIPTION

### Definitions

The following definitions are used, unless otherwise described: halo or halogen is fluoro, chloro, bromo, or iodo. Alkyl denotes both straight and branched groups; but reference to an individual radical such as propyl embraces only the straight chain radical, a branched chain isomer such as isopropyl being specifically referred to.

The term "alkyl", by itself or as part of another substituent, means, unless otherwise stated, a straight or branched chain hydrocarbon radical, having the number of carbon atoms designated (i.e., (C₁₋C₈)alkyl means one to eight carbons). Examples include (C₁-C₈)alkyl, (C₂-C₈)alkyl, C₁-C₆)alkyl, (C₂-C₆)alkyl and (C₃-C₆)alkyl. Examples of alkyl groups include methyl, ethyl, n-propyl, iso-propyl, n-butyl, t-butyl, iso-butyl, sec-butyl, n-pentyl, n-hexyl, n-heptyl, n-octyl, and and higher homologs and isomers.

As used herein a wavy line " 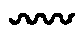 " that intersects a bond in a chemical structure indicates the point of attachment of the bond that the wavy bond intersects in the chemical structure to the remainder of a molecule.

The terms "treat", "treatment", or "treating" to the extent it relates to a disease or condition includes inhibiting the disease or condition, eliminating the disease or condition, and/or relieving one or more symptoms of the disease or condition. The terms "treat", "treatment", or "treating" also refer to both therapeutic treatment and/or prophylactic treatment or preventative measures, wherein the object is to prevent or slow down (lessen) an undesired physiological change or disorder, such as, for example, the development or spread of cancer. For example, beneficial or desired clinical results include, but are not limited to, alleviation of symptoms, diminishment of extent of disease or disorder, stabilized (i.e., not worsening) state of disease or disorder, delay or slowing of disease progression, amelioration or palliation of the disease state or disorder, and remission (whether partial or total), whether detectable or undetectable. "Treat", "treatment", or "treating," can also mean prolonging survival as compared to expected survival if not receiving treatment. Those in need of treatment include those already with the disease or disorder as well as those prone to have the disease or disorder or those in which the disease or disorder is to be prevented. In one embodiment "treat", "treatment", or "treating" does not include preventing or prevention,

The phrase "therapeutically effective amount" or "effective amount" includes but is not limited to an amount of a compound of the that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein.

The term "mammal" as used herein refers to humans, higher non-human primates, rodents, domestic, cows, horses, pigs, sheep, dogs and cats. In one embodiment, the mammal is a human. The term "patient" as used herein refers to any animal including mammals. In one embodiment, the patient is a mammalian patientIn one embodiment, the patient is a human patient.

The compounds disclosed herein can also exist as tautomeric isomers in certain cases. Although only one delocalized resonance structure may be depicted, all such forms are contemplated within the scope of the invention.

It is understood by one skilled in the art that this invention also includes any compound claimed that may be enriched at any or all atoms above naturally occurring isotopic ratios with one or more isotopes such as, but not limited to, deuterium (²H or D). As a non-limiting example, a -CH₃ group may be substituted with -CD₃.

The pharmaceutical compositions of the invention can comprise one or more excipients. When used in combination with the pharmaceutical compositions of the invention the term "excipients" refers generally to an additional ingredient that is combined with the compound of formula (I) or the pharmaceutically acceptable salt thereof to provide a corresponding composition. For example, when used in combination with the pharmaceutical compositions of the invention the term "excipients" includes, but is not limited to: carriers, binders, disintegrating agents, lubricants, sweetening agents, flavoring agents, coatings, preservatives, and dyes.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention can contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and l or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer can also be referred to as an enantiomer, and a mixture of such isomers is often called an enantiomeric mixture. A 50:50 mixture of enantiomers is referred to as a racemic mixture or a racemate, which can occur where there has been no stereoselection or stereospecificity in a chemical reaction or process. The terms "racemic mixture" and "racemate" refer to an equimolar mixture of two enantiomeric species, devoid of optical activity.

It will be appreciated by those skilled in the art that compounds of the invention having a chiral center may exist in and be isolated in optically active and racemic forms. Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase.

When a bond in a compound formula herein is drawn in a non-stereochemical manner (e.g. flat), the atom to which the bond is attached includes all stereochemical possibilities. When a bond in a compound formula herein is drawn in a defined stereochemical manner (e.g. bold, bold-wedge, dashed or dashed-wedge), it is to be understood that the atom to which the stereochemical bond is attached is enriched in the absolute stereoisomer depicted unless otherwise noted. In one embodiment, the compound may be at least 51% the absolute stereoisomer depicted. In another embodiment, the compound may be at least 60% the absolute stereoisomer depicted. In another embodiment, the compound may be at least 80% the absolute stereoisomer depicted. In another embodiment, the compound may be at least 90% the absolute stereoisomer depicted. In another embodiment, the compound may be at least 95 the absolute stereoisomer depicted. In another embodiment, the compound may be at least 99% the absolute stereoisomer depicted.

The term "residue" as it applies to the residue of a compound refers to a compound that has been modified in any manner which results in the creation of an open valence wherein the site of the open valence. The open valence can be created by the removal of 1 or more atoms from the compound (e.g., removal of a single atom such as hydrogen or removal of more than one atom such as a group of atoms including but not limited to an amine, hydroxyl, methyl, amide (e.g., -C(=O)NH₂) or acetyl group). The open valence can also be created by the chemical conversion of a first function group of the compound to a second functional group of the compound (e.g., reduction of a carbonyl group, replacement of a carbonyl group with an amine, ) followed by the removal of 1 or more atoms from the second functional group to create the open valence.

Specific values listed below for radicals, substituents, and ranges, are for illustration only; they do not exclude other defined values or other values within defined ranges for the radicals and substituents. It is to be understood that two or more values may be combined. It is also to be understood that the values listed herein below (or subsets thereof) can be excluded.

Specifically, (C₁-C₆)alkyl can be methyl, ethyl, propyl, isopropyl, butyl, iso-butyl, sec-butyl, pentyl, 3-pentyl, or hexyl; and (C₁-C₆)alkanoyl can be acetyl, propanoyl or butanoyl.

A specific compound of formula (I) is a compound wherein L is absent and X is -C(=O)NR²R³, for example, a compound of formula (Ia):

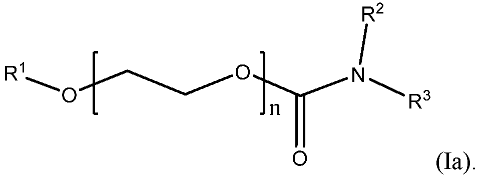

A specific compound of formula (I) is a compound wherein L is (C₁-C₆)alkyl and X is -N(R⁴)C(=O)CH(R²)(R³) , for example, a compound of formula (Ib):

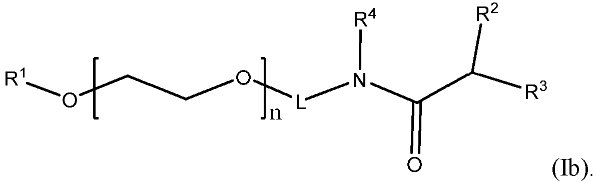

A specific compound of formula (I) is a compound wherein L is (C₁-C₆)alkyl and X is -OCH(R²)(R³) , for example, a compound of formula (Ic):

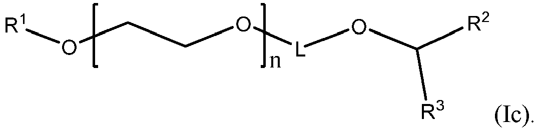

A specific compound of formula (I) is a compound wherein L is (C₁-C₆)alkyl and X is -C(=O)CH₂CH(R²)(R³) , for example, a compound of formula (Id):

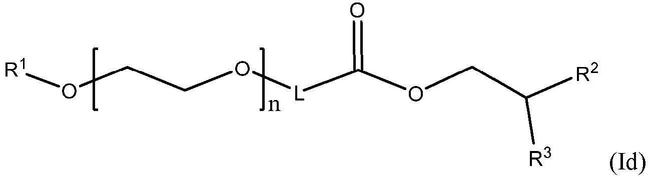

A specific compound of formula (I) is a compound wherein L is (C₁-C₆)alkyl and X is -N(R⁴)C(=O)N(R²)(R³) , for example, a compound of formula (Ie):

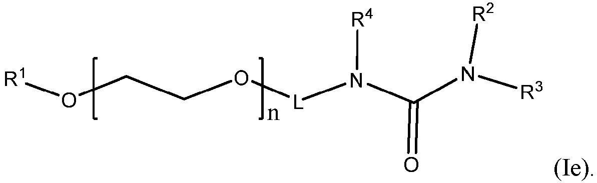

A specific compound of formula (I) is a compound wherein L is (C₁-C₆)alkyl and X is -SO₂N(R²)(R³) , for example, a compound of formula (If):

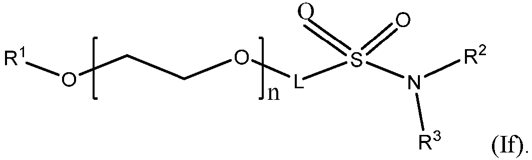

A specific value for R¹ is H.

A specific value for R¹ is (C₁-C₆)alkyl.

A specific value for R¹ is methyl.

A specific value for R¹ is (C₁-C₆)alkanoyl.

A specific value for R¹ is CH₃C(=O)-.

A specific value for n is an integer in the range of from about 20 to about 100.

A specific value for n is an integer in the range of from about 20 to about 70.

A specific value for n is an integer in the range of from about 20 to about 60.

A specific value for n is an integer in the range of from about 20 to about 50.

A specific value for n is an integer in the range of from about 30 to about 100.

A specific value for n is an integer in the range of from about 40 to about 100.

A specific value for n is an integer in the range of from about 40 to about 60.

A specific value for n is an integer in the range of from about 40 to about 50.

A specific value for n is an integer in the range of from about 44 to about 46.

In one specific embodiment, the group: 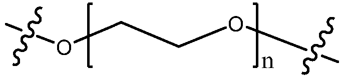 is PEG-2000.

A specific value for R² is C₁₁-alkyl, C₁₂-alkyl, C₁₃-alkyl, C₁₄-alkyl, C₁₅-alkyl, C₁₆-alkyl, C₁₇-alkyl, C₁₈-alkyl, C₁₉-alkyl, or C₂₀-alkyl.

A specific value for R² is C₁₂-alkyl, C₁₃-alkyl, C₁₄-alkyl, C₁₅-alkyl, C₁₆-alkyl, C₁₇-alkyl, or C₁₈-alkyl.

A specific value for R² is C₁₂-alkyl, C₁₄-alkyl, or C₁₆-alkyl.

A specific value for R² is C₁₄-alkyl.

A specific value for R³ is C₁₁-alkyl, C₁₂-alkyl, C₁₃-alkyl, C₁₄-alkyl, C₁₅-alkyl, C₁₆-alkyl, C₁₇-alkyl, C₁₈-alkyl, C₁₉-alkyl, or C₂₀-alkyl.

A specific value for R³ is C₁₂-alkyl, C₁₃-alkyl, C₁₄-alkyl, C₁₅-alkyl, C₁₆-alkyl, C₁₇-alkyl, or C₁₈-alkyl.

A specific value for R³ is C₁₂-alkyl, C₁₄-alkyl, or C₁₆-alkyl.

A specific value for R³ is C₁₄-alkyl.

A specific value for R⁴ is H.

A specific value for R⁴ is (C₁-C₆)alkyl.

A specific value for R⁴ is methyl.

The term "about" means ±5%, ±4%, ±3%, ±2%, or ±1%.

The term "interfering RNA" or "RNAi" or "interfering RNA sequence" refers to single-stranded RNA (*e.g*., mature miRNA) or double-stranded RNA (i.e., duplex RNA such as siRNA, aiRNA, or pre-miRNA) that is capable of reducing or inhibiting the expression of a target gene or sequence (*e.g*., by mediating the degradation or inhibiting the translation of mRNAs which are complementary to the interfering RNA sequence) when the interfering RNA is in the same cell as the target gene or sequence. Interfering RNA thus refers to the single-stranded RNA that is complementary to a target mRNA sequence or to the double-stranded RNA formed by two complementary strands or by a single, self-complementary strand. Interfering RNA may have substantial or complete identity to the target gene or sequence, or may comprise a region of mismatch (i.e., a mismatch motif). The sequence of the interfering RNA can correspond to the full-length target gene, or a subsequence thereof.

Interfering RNA includes "small-interfering RNA" or "siRNA," *e.g*., interfering RNA of about 15-60, 15-50, or 15-40 (duplex) nucleotides in length, more typically about 15-30, 15-25, or 19-25 (duplex) nucleotides in length, and is preferably about 20-24, 21-22, or 21-23 (duplex) nucleotides in length (*e.g.,* each complementary sequence of the double-stranded siRNA is 15-60, 15-50, 15-40, 15-30, 15-25, or 19-25 nucleotides in length, preferably about 20-24, 21-22, or 21-23 nucleotides in length, and the double-stranded siRNA is about 15-60, 15-50, 15-40, 15-30, 15-25, or 19-25 base pairs in length, preferably about 18-22, 19-20, or 19-21 base pairs in length). siRNA duplexes may comprise 3' overhangs of about 1 to about 4 nucleotides or about 2 to about 3 nucleotides and 5' phosphate termini. Examples of siRNA include, without limitation, a double-stranded polynucleotide molecule assembled from two separate stranded molecules, wherein one strand is the sense strand and the other is the complementary antisense strand; a double-stranded polynucleotide molecule assembled from a single stranded molecule, where the sense and antisense regions are linked by a nucleic acid-based or non-nucleic acid-based linker; a double-stranded polynucleotide molecule with a hairpin secondary structure having self-complementary sense and antisense regions; and a circular single-stranded polynucleotide molecule with two or more loop structures and a stem having self-complementary sense and antisense regions, where the circular polynucleotide can be processed in vivo or in vitro to generate an active double-stranded siRNA molecule.

Preferably, siRNA are chemically synthesized. siRNA can also be generated by cleavage of longer dsRNA (*e.g.,* dsRNA greater than about 25 nucleotides in length) with the *E. coli* RNase III or Dicer. These enzymes process the dsRNA into biologically active siRNA (see, *e.g.,* Yang et al., Proc. Natl. Acad. Sci. USA, 99:9942-9947 (2002); Calegari et al., Proc. Natl. Acad. Sci. USA, 99:14236 (2002); Byrom et al., Ambion TechNotes, 10(1):4-6 (2003); Kawasaki et al., Nucleic Acids Res., 31:981-987 (2003); Knight et al., Science, 293:2269-2271 (2001); and Robertson et al., J. Biol. Chem., 243:82 (1968)). Preferably, dsRNA are at least 50 nucleotides to about 100, 200, 300, 400, or 500 nucleotides in length. A dsRNA may be as long as 1000, 1500, 2000, 5000 nucleotides in length, or longer. The dsRNA can encode for an entire gene transcript or a partial gene transcript. In certain instances, siRNA may be encoded by a plasmid (*e.g*., transcribed as sequences that automatically fold into duplexes with hairpin loops).

As used herein, the term "mismatch motif" or "mismatch region" refers to a portion of an interfering RNA (*e.g*., siRNA, aiRNA, miRNA) sequence that does not have 100% complementarity to its target sequence. An interfering RNA may have at least one, two, three, four, five, six, or more mismatch regions. The mismatch regions may be contiguous or may be separated by 1, 2, 3, 4, 5, 6, 7, 8, 9, 10, 11, 12, or more nucleotides. The mismatch motifs or regions may comprise a single nucleotide or may comprise two, three, four, five, or more nucleotides.

An "effective amount" or "therapeutically effective amount" of an nucleic acid such as a nucleic acid (*e.g.,* an interfering RNA or mRNA) is an amount sufficient to produce the desired effect, *e.g.,* an inhibition of expression of a target sequence in comparison to the normal expression level detected in the absence of an interfering RNA; or mRNA-directed expression of an amount of a protein that causes a desirable biological effect in the organism within which the protein is expressed. Inhibition of expression of a target gene or target sequence is achieved when the value obtained with an interfering RNA relative to the control is about 90%, 85%, 80%, 75%, 70%, 65%, 60%, 55%, 50%, 45%, 40%, 35%, 30%, 25%, 20%, 15%, 10%, 5%, or 0%. In other embodiments, the expressed protein is an active form of a protein that is normally expressed in a cell type within the body, and the therapeutically effective amount of the mRNA is an amount that produces an amount of the encoded protein that is at least 50% (*e.g.,* at least 60%, or at least 70%, or at least 80%, or at least 90%) of the amount of the protein that is normally expressed in the cell type of a healthy individual. Suitable assays for measuring expression of a target gene or target sequence include, *e.g*., examination of protein or RNA levels using techniques known to those of skill in the art such as dot blots, northern blots, in situ hybridization, ELISA, immunoprecipitation, enzyme function, as well as phenotypic assays known to those of skill in the art.

By "decrease," "decreasing," "reduce," or "reducing" of an immune response by an interfering RNA is intended to mean a detectable decrease of an immune response to a given interfering RNA (*e.g.,* a modified interfering RNA). The amount of decrease of an immune response by a modified interfering RNA may be determined relative to the level of an immune response in the presence of an unmodified interfering RNA. A detectable decrease can be about 5%, 10%, 15%, 20%, 25%, 30%, 35%, 40%, 45%, 50%, 55%, 60%, 65%, 70%, 75%, 80%, 85%, 90%, 95%, 100%, or more lower than the immune response detected in the presence of the unmodified interfering RNA. A decrease in the immune response to interfering RNA is typically measured by a decrease in cytokine production (*e.g*., IFNγ, IFNα, TNFα, IL-6, or IL-12) by a responder cell in vitro or a decrease in cytokine production in the sera of a mammalian subject after administration of the interfering RNA.

By "decrease," "decreasing," "reduce," or "reducing" of an immune response by an mRNA is intended to mean a detectable decrease of an immune response to a given mRNA (*e.g*., a modified mRNA). The amount of decrease of an immune response by a modified mRNA may be determined relative to the level of an immune response in the presence of an unmodified mRNA. A detectable decrease can be about 5%, 10%, 15%, 20%, 25%, 30%, 35%, 40%, 45%, 50%, 55%, 60%, 65%, 70%, 75%, 80%, 85%, 90%, 95%, 100%, or more lower than the immune response detected in the presence of the unmodified mRNA. A decrease in the immune response to mRNA is typically measured by a decrease in cytokine production (*e.g*., IFNγ, IFNα, TNFα, IL-6, or IL-12) by a responder cell *in vitro* or a decrease in cytokine production in the sera of a mammalian subject after administration of the mRNA.

As used herein, the term "responder cell" refers to a cell, preferably a mammalian cell, which produces a detectable immune response when contacted with an immunostimulatory interfering RNA such as an unmodified siRNA. Exemplary responder cells include, *e.g.,* dendritic cells, macrophages, peripheral blood mononuclear cells (PBMCs), splenocytes, and the like. Detectable immune responses include, *e.g*., production of cytokines or growth factors such as TNF-α, IFN-α, IFN-β, IFN-γ, IL-1, IL-2, IL-3, IL-4, IL-5, IL-6, IL-10, IL-12, IL-13, TGF, and combinations thereof.

"Substantial identity" refers to a sequence that hybridizes to a reference sequence under stringent conditions, or to a sequence that has a specified percent identity over a specified region of a reference sequence.

The phrase "stringent hybridization conditions" refers to conditions under which a nucleic acid will hybridize to its target sequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology-Hybridization with Nucleic Probes, "Overview of principles of hybridization and the strategy of nucleic acid assays" (1993). Generally, stringent conditions are selected to be about 5-10° C. lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization.

Exemplary stringent hybridization conditions can be as follows: 50% formamide, 5×SSC, and 1% SDS, incubating at 42° C., or, 5×SSC, 1% SDS, incubating at 65° C., with wash in 0.2×SSC, and 0.1% SDS at 65° C. For PCR, a temperature of about 36° C. is typical for low stringency amplification, although annealing temperatures may vary between about 32° C. and 48° C. depending on primer length. For high stringency PCR amplification, a temperature of about 62° C. is typical, although high stringency annealing temperatures can range from about 50° C. to about 65° C., depending on the primer length and specificity. Typical cycle conditions for both high and low stringency amplifications include a denaturation phase of 90° C.-95° C. for 30 sec.-2 min., an annealing phase lasting 30 sec.-2 min., and an extension phase of about 72° C. for 1-2 min. Protocols and guidelines for low and high stringency amplification reactions are provided, *e.g.,* in Innis et al., PCR Protocols, A Guide to Methods and Applications, Academic Press, Inc. N.Y. (1990).

Nucleic acids that do not hybridize to each other under stringent conditions are still substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37° C., and a wash in 1×SSC at 45° C. A positive hybridization is at least twice background. Those of ordinary skill will readily recognize that alternative hybridization and wash conditions can be utilized to provide conditions of similar stringency. Additional guidelines for determining hybridization parameters are provided in numerous references, *e.g.,* Current Protocols in Molecular Biology, Ausubel et al., eds.

The terms "substantially identical" or "substantial identity," in the context of two or more nucleic acids, refer to two or more sequences or subsequences that are the same or have a specified percentage of nucleotides that are the same (i.e., at least about 60%, preferably at least about 65%, 70%, 75%, 80%, 85%, 90%, or 95% identity over a specified region), when compared and aligned for maximum correspondence over a comparison window, or designated region as measured using one of the following sequence comparison algorithms or by manual alignment and visual inspection. This definition, when the context indicates, also refers analogously to the complement of a sequence. Preferably, the substantial identity exists over a region that is at least about 5, 10, 15, 20, 25, 30, 35, 40, 45, 50, 55, or 60 nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of a number of contiguous positions selected from the group consisting of from about 5 to about 60, usually about 10 to about 45, more usually about 15 to about 30, in which a sequence may be compared to a reference sequence of the same number of contiguous positions after the two sequences are optimally aligned. Methods of alignment of sequences for comparison are well known in the art. Optimal alignment of sequences for comparison can be conducted, *e.g.,* by the local homology algorithm of Smith and Waterman, Adv. Appl. Math., 2:482 (1981), by the homology alignment algorithm of Needleman and Wunsch, J. Mol. Biol., 48:443 (1970), by the search for similarity method of Pearson and Lipman, Proc. Natl. Acad. Sci. USA, 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, *e.g.,* Current Protocols in Molecular Biology, Ausubel et al., eds. (1995 supplement)).

A preferred example of algorithms that are suitable for determining percent sequence identity and sequence similarity are the BLAST and BLAST 2.0 algorithms, which are described in Altschul et al., Nuc. Acids Res., 25:3389-3402 (1977) and Altschul et al., J. Mol. Biol., 215:403-410 (1990), respectively. BLAST and BLAST 2.0 are used, with the parameters described herein, to determine percent sequence identity for the nucleic acids. Software for performing BLAST analyses is publicly available through the National Center for Biotechnology Information (http://www.ncbi.nlm.nih.gov/).

The BLAST algorithm also performs a statistical analysis of the similarity between two sequences (see, *e.g.,* Karlin and Altschul, Proc. Natl. Acad. Sci. USA, 90:5873-5787 (1993)). One measure of similarity provided by the BLAST algorithm is the smallest sum probability (P(N)), which provides an indication of the probability by which a match between two nucleotide sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The term "nucleic acid" as used herein refers to a polymer containing at least two deoxyribonucleotides or ribonucleotides in either single- or double-stranded form and includes DNA and RNA. DNA may be in the form of, e.g., antisense molecules, plasmid DNA, precondensed DNA, a PCR product, vectors (P1, PAC, BAC, YAC, artificial chromosomes), expression cassettes, chimeric sequences, chromosomal DNA, or derivatives and combinations of these groups. RNA may be in the form of siRNA, asymmetrical interfering RNA (aiRNA), microRNA (miRNA), mRNA, tRNA, rRNA, tRNA, viral RNA (vRNA), self-amplifying RNA, and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (*e.g*., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)). "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides.

The term "gene" refers to a nucleic acid (*e.g.,* DNA or RNA) sequence that comprises partial length or entire length coding sequences necessary for the production of a polypeptide or precursor polypeptide.

"Gene product," as used herein, refers to a product of a gene such as an RNA transcript or a polypeptide.

The term "lipid" refers to a group of organic compounds that include, but are not limited to, esters of fatty acids and are characterized by being insoluble in water, but soluble in many organic solvents. They are usually divided into at least three classes: (1) "simple lipids," which include fats and oils as well as waxes; (2) "compound lipids," which include phospholipids and glycolipids; and (3) "derived lipids" such as steroids.

The term "alkyl", by itself or as part of another substituent, means, unless otherwise stated, a straight or branched chain hydrocarbon radical, which can be saturated or unsaturated, having the number of carbon atoms designated (*i.e.,* C₁₋₈ means one to eight carbons). Examples of alkyl groups include methyl, ethyl, n-propyl, iso-propyl, n-butyl, t-butyl, iso-butyl, sec-butyl, n-pentyl, n-hexyl, n-heptyl, n-octyl, and the like. The term "alkenyl" refers to an unsaturated alkyl radical having one or more double bonds. Similarly, the term "alkynyl" refers to an unsaturated alkyl radical having one or more triple bonds. Examples of such unsaturated alkyl groups include vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers.

As used herein, the term lipid nanoparticle "LNP" refers to a lipid-nucleic acid particle or a nucleic acid-lipid particle (*e.g.,* a stable nucleic acid-lipid particle). A LNP represents a particle made from lipids (*e.g.,* a cationic lipid, a non-cationic lipid, and a conjugated lipid that prevents aggregation of the particle), and a nucleic acid, wherein the nucleic acid (*e.g*., siRNA, aiRNA, miRNA, ssDNA, dsDNA, ssRNA, short hairpin RNA (shRNA), dsRNA, mRNA, self-amplifying RNA, or a plasmid, including plasmids from which an interfering RNA or mRNA is transcribed) is encapsulated within the lipid. In one embodiment, the nucleic acid is at least 50% encapsulated in the lipid; in one embodiment, the nucleic acid is at least 75% encapsulated in the lipid; in one embodiment, the nucleic acid is at least 90% encapsulated in the lipid; and in one embodiment, the nucleic acid is completely encapsulated in the lipid. LNPs typically contain a cationic lipid, a non-cationic lipid, and a lipid conjugate (*e.g.,* a PEG-lipid conjugate). LNP are extremely useful for systemic applications, as they can exhibit extended circulation lifetimes following intravenous (i.v.) injection, they can accumulate at distal sites (*e.g.,* sites physically separated from the administration site), and they can mediate expression of the transfected gene or silencing of target gene expression at these distal sites.

The lipid particles of the invention (*e.g.,* LNPs) typically have a mean diameter of from about 40 nm to about 150 nm, from about 40 nm to about 80 nm, from about 40 nm to about 60 nm, from about 50 nm to about 60 nm from about from about 50 nm to about 150 nm, from about 60 nm to about 130 nm, from about 70 nm to about 110 nm, or from about 70 to about 90 nm, and are substantially non-toxic. In addition, nucleic acids, when present in the lipid particles of the invention, are resistant in aqueous solution to degradation with a nuclease. Nucleic acid-lipid particles and their method of preparation are disclosed in, *e.g.,* U.S. Patent Publication Nos. 20040142025 and 20070042031.

As used herein, "lipid encapsulated" can refer to a lipid particle that provides a nucleic acid (*e.g.,* an interfering RNA or mRNA), with full encapsulation, partial encapsulation, or both. In one embodiment, the nucleic acid is fully encapsulated in the lipid particle (*e.g.,* to form an LNP, or other nucleic acid-lipid particle).

The term "cationic lipid" refers to any of a number of lipid species that carry a net positive charge at a selected pH, such as physiological pH (*e.g.,* pH of about 7.0). It has been surprisingly found that cationic lipids comprising alkyl chains with multiple sites of unsaturation, *e.g.,* at least two or three sites of unsaturation, are particularly useful for forming lipid particles with increased membrane fluidity. A number of cationic lipids and related analogs, which are also useful in the present invention, have been described in U.S. Patent Publication Nos. 20060083780 and 20060240554; U.S. Pat. Nos. 5,208,036; 5,264,618; 5,279,833; 5,283,185; 5,753,613; and 5,785,992; and PCT Publication No. WO 96/10390. Non-limiting examples of cationic lipids are described in detail herein. In some cases, the cationic lipids comprise a protonatable tertiary amine (*e.g.,* pH titratable) head group, C18 alkyl chains, ether linkages between the head group and alkyl chains, and 0 to 3 double bonds. Such lipids include, *e.g.,* DSDMA, DLinDMA, DLenDMA, and DODMA.

In the lipid nanoparticles described herein, the cationic lipid may be, *e.g.,* one or more of the following: 1,2-dilinoleyloxy-N,N-dimethylaminopropane (DLinDMA), 1,2-dilinolenyloxy-N,N-dimethylaminopropane (DLenDMA), 2,2-dilinoleyl-4-(2-dimethylaminoethyl)-[1,3]-dioxolane (DLin-K-C2-DMA; "XTC2"), 2,2-dilinoleyl-4-(3-dimethylaminopropyl)-[1,3]-dioxolane (DLin-K-C3-DMA), 2,2-dilinoleyl-4-(4-dimethylaminobutyl)-[1,3]-dioxolane (DLin-K-C4-DMA), 2,2-dilinoleyl-5-dimethylaminomethyl-[1,3]-dioxane (DLin-K6-DMA), 2,2-dilinoleyl-4-N-methylpepiazino-[1,3]-dioxolane (DLin-K-MPZ), 2,2-dilinoleyl-4-dimethylaminomethyl-[1,3]-dioxolane (DLin-K-DMA), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanedio (DOAP), 1,2-dilinoleyloxo-3-(2-N,N-dimethylamino)ethoxypropane (DLin-EG-DMA), N,N-dioleyl-N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3-(N-(N',N'-dimethylaminoethane)-carbamoyl)cholesterol (DC-Chol), N-(1,2-dimyristyloxyprop-3-yl)-N,N-dimethyl-N-hydroxyethyl ammonium bromide (DMRIE), 2,3-dioleyloxy-N-[2(spermine-carboxamido)ethyl]-N,N-dimethyl-1-propanaminiumtrifluoroacetate (DOSPA), dioctadecylamidoglycyl spermine (DOGS), 3-dimethylamino-2-(cholest-5-en-3-beta-oxybutan-4-oxy)-1-(cis,cis-9,12-octadecadienoxy)propane (CLinDMA), 2-[5'-(cholest-5-en-3-beta-oxy)-3'-oxapentoxy)-3-dimethyl-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), or mixtures thereof. In certain embodiments, the cationic lipid is DLinDMA, DLin-K-C2-DMA ("XTC2"), or mixtures thereof.

In certain embodiments, the cationic lipid is selected from one of the following lipids: 3-(((6Z,9Z,28Z,31Z)-heptatriaconta-6,9,28,31-tetraen-19-yl)oxy)-N,N-dimethylpropan-1-amine; (6Z,9Z,28Z,31Z)-heptatriaconta-6,9,28,31-tetraen-19-yl 4-(dimethylamino)butanoate; (6Z,16Z)-12-((Z)-dec-4-en-1-yl)docosa-6,16-dien-11-yl 5-(dimethylamino)pentanoate; (6Z,16Z)-12-((Z)-dec-4-en-1-yl)docosa-6,16-dien-11-yl 6-(dimethylamino)hexanoate; N,N-dimethyl-4-(tris(((Z)-dec-4-en-1-yl)oxy)silyl)butan-1-amine; N,N-dimethyl-5-(tris(((Z)-dec-4-en-1-yl)oxy)silyl)pentan-1-amine; N,N-dimethyl-6-(tris(((Z)-dec-4-en-1-yl)oxy)silyl)hexan-1-amine; 2-(methyl(4-(tris(((Z)-dec-4-en-1-yl)oxy)silyl)butyl)amino)ethan-1-ol; (6Z,16Z)-12-((6-(dimethylamino)hexanoyl)oxy)docosa-6,16-dien-11-yl (Z)-undec-5-enoate; N1,N3-bis(4-(bis(((Z)-dec-4-en-1-yl)oxy)(methyl)silyl)butyl)-N1,N3-dimethylpropane-1,3-diamine; N1,N3-dimethyl-N1,N3-bis(4-(tris(((Z)-hept-3-en-1-yl)oxy)silyl)butyl)propane-1,3-diamine; (1r,4r)-N1,N4-bis(4-(bis(((Z)-dec-4-en-1-yl)oxy)(methyl)silyl)butyl)-N1,N4-dimethylcyclohexane-1,4-diamine; 2,8-bis(4-(bis(((Z)-dec-4-en-1-yl)oxy)(methyl)silyl)butyl)-2,8-diazaspiro[4.5]decane; or bis(2-butyloctyl) 10-(N-(3-(dimethylamino)propyl) nonanamido)- nonadecanedioate; di(tridecan-7-yl) 10-(N-(3-(dimethylamino)propyl)octanamido)-nonadecanedioate; di(tridecan-7-yl) 10-(N-decyl-4-(dimethylamino)butanamido)nonadecanedioate; ((4-hydroxybutyl)azanediyl)bis(nonane-9,1-diyl) bis(2-butyloctanoate) or heptadecan-9-yl 8-((2-hydroxyethyl)(8-(nonyloxy)-8-oxooctyl)amino)octanoate; 3-((4,4-bis(octyloxy)butanoyl)oxy)-2-((((3-(diethylamino)propoxy)carbonyl)oxy)methyl)propyl (9Z,12Z)-octadeca-9,12-dienoate or 3,6-bis(4-(bis(2-hydroxydodecyl)amino)butyl)piperazine-2,5-dione; 3,6-bis(4-(bis((9Z,12Z)-2-hydroxyoctadeca-9,12-dien-1-yl)amino)butyl)piperazine-2,5-dione; 1,1'-((2-(1-(2-((2-(bis(2-hydroxydodecyl)amino)ethyl)(2-hydroxydodecyl)amino)ethyl)piperidin-4-yl)ethyl)azanediyl)bis(dodecan-2-ol); tetratridecyl 3,3',3",3"'-((azanediylbis(propane-3,1-diyl))bis(azanetriyl))tetrapropionate or nonyl 8-((8,8-bis(octyloxy)octyl)(2-hydroxyethyl)amino)octanoate or di((Z)-non-2-en-1-yl) 8,8'-((((2-(dimethylamino)ethyl)thio)carbonyl)azanediyl)dioctanoate.

The synthesis of cationic lipids such as DLin-K-C2-DMA ("XTC2"), DLin-K-C3-DMA, DLin-K-C4-DMA, DLin-K6-DMA, and DLin-K-MPZ, as well as additional cationic lipids, is described in U.S. Provisional Application No. 61/104,212, filed Oct. 9, 2008. The synthesis of cationic lipids such as DLin-K-DMA, DLin-C-DAP, DLin-DAC, DLin-MA, DLinDAP, DLin-S-DMA, DLin-2-DMAP, DLin-TMA.Cl, DLin-TAP.Cl, DLin-MPZ, DLinAP, DOAP, and DLin-EG-DMA, as well as additional cationic lipids, is described in PCT Application No. PCT/US08/88676, filed Dec. 31, 2008. The synthesis of cationic lipids such as CLinDMA, as well as additional cationic lipids, is described in U.S. Patent Publication No. 20060240554.

Any of a variety of cationic lipids may be used in the lipid particles of the invention (*e.g.,* LNP), either alone or in combination with one or more other cationic lipid species or non-cationic lipid species.

Cationic lipids which are useful in the present invention can be any of a number of lipid species which carry a net positive charge at physiological pH. Such lipids include, but are not limited to, N,N-dioleyl-N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3-(N-(N',N'-dimethylaminoethane)-carbamoyl)cholesterol (DC-Chol), N-(1,2-dimyristyloxyprop-3-yl)-N,N-dimethyl-N-hydroxyethyl ammonium bromide (DMRIE), 2,3-dioleyloxy-N-[2(spermine-carboxamido)ethyl]-N,N-dimethyl-1-propanaminiumtrifluoroacetate (DOSPA), dioctadecylamidoglycyl spermine (DOGS), 3-dimethylamino-2-(cholest-5-en-3-beta-oxybutan-4-oxy)-1-(cis,cis-9,12-octadecadienoxy)propane (CLinDMA), 2-[5'-(cholest-5-en-3.beta.-oxy)-3'-oxapentoxy)-3-dimethyl-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-Dilinoleylcarbamyl-3-dimethylaminopropane (DLincarbDAP), 1,2-Dilinoleoylcarbamyl-3-dimethylaminopropane (DLinCDAP), and mixtures thereof. A number of these lipids and related analogs have been described in U.S. Patent Publication Nos. 20060083780 and 20060240554; U.S. Pat. Nos. 5,208,036; 5,264,618; 5,279,833; 5,283,185; 5,753,613; and 5,785,992; and PCT Publication No. WO 96/10390. Additionally, a number of commercial preparations of cationic lipids are available and can be used in the present invention. These include, *e.g.,* LIPOFECTIN^{®} (commercially available cationic liposomes comprising DOTMA and DOPE, from GIBCO/BRL, Grand Island, N.Y., USA); LIPOFECTAMINE^{®} (commercially available cationic liposomes comprising DOSPA and DOPE, from GIBCO/BRL); and TRANSFECTAM^{®} (commercially available cationic liposomes comprising DOGS from Promega Corp., Madison, Wis., USA).

Additionally, cationic lipids of Formula I having the following structures are useful in the present invention. 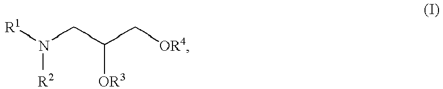 wherein R¹ and R² are independently selected and are H or C₁-C₃ alkyls, R³ and R⁴ are independently selected and are alkyl groups having from about 10 to about 20 carbon atoms, and at least one of R³ and R⁴ comprises at least two sites of unsaturation. In certain instances, R³ and R⁴ are both the same, i.e., R³ and R⁴ are both linoleyl (C₁₈), etc. In certain other instances, R³ and R⁴ are different, i.e., R³ is tetradectrienyl (C₁₄) and R⁴ is linoleyl (C₁₈). In one embodiment, the cationic lipid of Formula I is symmetrical, i.e., R³ and R⁴ are both the same. In another embodiment, both R³ and R⁴ comprise at least two sites of unsaturation. In some embodiments, R³ and R⁴ are independently selected from the group consisting of dodecadienyl, tetradecadienyl, hexadecadienyl, linoleyl, and icosadienyl. In one embodiment, R³ and R⁴ are both linoleyl. In some embodiments, R³ and R⁴ comprise at least three sites of unsaturation and are independently selected from, *e.g.,* dodecatrienyl, tetradectrienyl, hexadecatrienyl, linolenyl, and icosatrienyl. In particular embodiments, the cationic lipid of Formula I is 1,2-dilinoleyloxy-N,N-dimethylaminopropane (DLinDMA) or 1,2-dilinolenyloxy-N,N-dimethylaminopropane (DLenDMA).

Furthermore, cationic lipids of Formula II having the following structures are useful in the present invention. 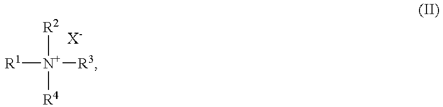 wherein R¹ and R² are independently selected and are H or C₁-C₃ alkyls, R³ and R⁴ are independently selected and are alkyl groups having from about 10 to about 20 carbon atoms, and at least one of R³ and R⁴ comprises at least two sites of unsaturation. In certain instances, R³ and R⁴ are both the same, i.e., R³ and R⁴ are both linoleyl (C₁₈), etc. In certain other instances, R³ and R⁴ are different, i.e., R³ is tetradectrienyl (C₁₄) and R⁴ is linoleyl (C₁₈). In one embodiment, the cationic lipids of the present invention are symmetrical, i.e., R³ and R⁴ are both the same. In another embodiment, both R³ and R⁴ comprise at least two sites of unsaturation. In some embodiments, R³ and R⁴ are independently selected from the group consisting of dodecadienyl, tetradecadienyl, hexadecadienyl, linoleyl, and icosadienyl. In one embodiment, R³ and R⁴ are both linoleyl. In some embodiments, R³ and R⁴ comprise at least three sites of unsaturation and are independently selected from, *e.g.,* dodecatrienyl, tetradectrienyl, hexadecatrienyl, linolenyl, and icosatrienyl.

Moreover, cationic lipids of Formula III having the following structures (or salts thereof) are useful in the present invention. 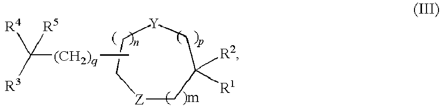 wherein R¹ and R² are either the same or different and independently optionally substituted C₁₂-C₂₄ alkyl, optionally substituted C₁₂-C₂₄ alkenyl, optionally substituted C₁₂-C₂₄ alkynyl, or optionally substituted C₁₂-C₂₄ acyl; R³ and R⁴ are either the same or different and independently optionally substituted C₁-C₆ alkyl, optionally substituted C₁-C₆ alkenyl, or optionally substituted C₁-C₆ alkynyl or R³ and R⁴ may join to form an optionally substituted heterocyclic ring of 4 to 6 carbon atoms and 1 or 2 heteroatoms chosen from nitrogen and oxygen; R⁵ is either absent or hydrogen or C₁-C₆ alkyl to provide a quaternary amine; m, n, and p are either the same or different and independently either 0 or 1 with the proviso that m, n, and p are not simultaneously 0; q is 0, 1, 2, 3, or 4; and Y and Z are either the same or different and independently O, S, or NH.

In some embodiments, the cationic lipid of Formula III is 2,2-dilinoleyl-4-(2-dimethylaminoethyl)-[1,3]-dioxolane (DLin-K-C2-DMA; "XTC2"), 2,2-dilinoleyl-4-(3-dimethylaminopropyl)-[1,3]-dioxolane (DLin-K-C3-DMA), 2,2-dilinoleyl-4-(4-dimethylaminobutyl)-[1,3]-dioxolane (DLin-K-C4-DMA), 2,2-dilinoleyl-5-dimethylaminomethyl-[1,3]-dioxane (DLin-K6-DMA), 2,2-dilinoleyl-4-N-methylpepiazino-[1,3]-dioxolane (DLin-K-MPZ), 2,2-dilinoleyl-4-dimethylaminomethyl-[1,3]-dioxolane (DLin-K-DMA), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanedio (DOAP), 1,2-dilinoleyloxo-3-(2-N,N-dimethylamino)ethoxypropane (DLin-EG-DMA), or mixtures thereof. In some embodiments, the cationic lipid of Formula III is DLin-K-C2-DMA (XTC2).

The cationic lipid typically comprises from about 50 mol % to about 90 mol %, from about 50 mol % to about 85 mol %, from about 50 mol % to about 80 mol %, from about 50 mol % to about 75 mol %, from about 50 mol % to about 70 mol %, from about 50 mol % to about 65 mol %, or from about 55 mol % to about 65 mol % of the total lipid present in the particle.

It will be readily apparent to one of skill in the art that depending on the intended use of the particles, the proportions of the components can be varied and the delivery efficiency of a particular formulation can be measured using, *e.g.,* an endosomal release parameter (ERP) assay.

The non-cationic lipids used in the lipid particles of the invention (*e.g.,* LNP) can be any of a variety of neutral uncharged, zwitterionic, or anionic lipids capable of producing a stable complex.

Non-limiting examples of non-cationic lipids include phospholipids such as lecithin, phosphatidylethanolamine, lysolecithin, lysophosphatidylethanolamine, phosphatidylserine, phosphatidylinositol, sphingomyelin, egg sphingomyelin (ESM), cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoyl-phosphatidylcholine (POPC), palmitoyloleoyl-phosphatidylethanolamine (POPE), palmitoyloleyol-phosphatidylglycerol (POPG), dioleoylphosphatidylethanolamine 4-(N-maleimidomethyl)-cyclohexane-1-carboxylate (DOPE-mal), dipalmitoyl-phosphatidylethanolamine (DPPE), dimyristoyl-phosphatidylethanolamine (DMPE), distearoyl-phosphatidylethanolamine (DSPE), monomethyl-phosphatidylethanolamine, dimethyl-phosphatidylethanolamine, dielaidoyl-phosphatidylethanolamine (DEPE), stearoyloleoyl-phosphatidylethanolamine (SOPE), lysophosphatidylcholine, dilinoleoylphosphatidylcholine, and mixtures thereof. Other diacylphosphatidylcholine and diacylphosphatidylethanolamine phospholipids can also be used. The acyl groups in these lipids are typically acyl groups derived from fatty acids having C₁₀-C₂₄ carbon chains, e.g., lauroyl, myristoyl, palmitoyl, stearoyl, or oleoyl.

Additional examples of non-cationic lipids include sterols such as cholesterol and derivatives thereof such as cholestanol, cholestanone, cholestenone, coprostanol, cholesteryl-2'-hydroxyethyl ether, cholesteryl-4'-hydroxybutyl ether, and mixtures thereof.

In some embodiments, the non-cationic lipid present in the lipid particles (*e.g.,* LNP) comprises or consists of cholesterol or a derivative thereof, *e.g.,* a phospholipid-free lipid particle formulation. In other embodiments, the non-cationic lipid present in the lipid particles (*e.g.,* LNP) comprises or consists of one or more phospholipids, *e.g.,* a cholesterol-free lipid particle formulation. In further embodiments, the non-cationic lipid present in the lipid particles (*e.g.,* LNP) comprises or consists of a mixture of one or more phospholipids and cholesterol or a derivative thereof.

Other examples of non-cationic lipids suitable for use in the present invention include nonphosphorous containing lipids such as, *e.g.,* stearylamine, dodecylamine, hexadecylamine, acetyl palmitate, glycerolricinoleate, hexadecyl stereate, isopropyl myristate, amphoteric acrylic polymers, triethanolamine-lauryl sulfate, alkyl-aryl sulfate polyethyloxylated fatty acid amides, dioctadecyldimethyl ammonium bromide, ceramide, sphingomyelin, and the like.

The term "hydrophobic lipid" refers to compounds having apolar groups that include, but are not limited to, long-chain saturated and unsaturated aliphatic hydrocarbon groups and such groups optionally substituted by one or more aromatic, cycloaliphatic, or heterocyclic group(s). Suitable examples include, but are not limited to, diacylglycerol, dialkylglycerol, N-N-dialkylamino, 1,2-diacyloxy-3-aminopropane, and 1,2-dialkyl-3-aminopropane.

The term "fusogenic" refers to the ability of a lipid particle, such as a LNP, to fuse with the membranes of a cell. The membranes can be either the plasma membrane or membranes surrounding organelles, *e.g.,* endosome, nucleus, etc.

As used herein, the term "aqueous solution" refers to a composition comprising in whole, or in part, water.

As used herein, the term "organic lipid solution" refers to a composition comprising in whole, or in part, an organic solvent having a lipid.

"Distal site," as used herein, refers to a physically separated site, which is not limited to an adjacent capillary bed, but includes sites broadly distributed throughout an organism.

"Serum-stable" in relation to lipid nanoparticles such as LNP means that the particle is not significantly degraded after exposure to a serum or nuclease assay that would significantly degrade free DNA or RNA. Suitable assays include, for example, a standard serum assay, a DNAse assay, or an RNAse assay.

"Systemic delivery," as used herein, refers to delivery of lipid particles that leads to a broad biodistribution of an nucleic acid, such as an interfering RNA or mRNA, within an organism. Some techniques of administration can lead to the systemic delivery of certain agents, but not others. Systemic delivery means that a useful, preferably therapeutic, amount of an agent is exposed to most parts of the body. To obtain broad biodistribution generally requires a blood lifetime such that the agent is not rapidly degraded or cleared (such as by first pass organs (liver, lung, etc.) or by rapid, nonspecific cell binding) before reaching a disease site distal to the site of administration. Systemic delivery of lipid particles can be by any means known in the art including, for example, intravenous, subcutaneous, and intraperitoneal. In a preferred embodiment, systemic delivery of lipid particles is by intravenous delivery.

"Local delivery," as used herein, refers to delivery of an nucleic acid, such as an interfering RNA or mRNA, directly to a target site within an organism. For example, an agent can be locally delivered by direct injection into a disease site such as a tumor or other target site such as a site of inflammation or a target organ such as the liver, heart, pancreas, kidney, and the like.

The term "mammal" refers to any mammalian species such as a human, mouse, rat, dog, cat, hamster, guinea pig, rabbit, livestock, and the like.

The term "cancer" refers to any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Examples of different types of cancer include, but are not limited to, lung cancer, colon cancer, rectal cancer, anal cancer, bile duct cancer, small intestine cancer, stomach (gastric) cancer, esophageal cancer; gallbladder cancer, liver cancer, pancreatic cancer, appendix cancer, breast cancer, ovarian cancer; cervical cancer, prostate cancer, renal cancer (*e.g.,* renal cell carcinoma), cancer of the central nervous system, glioblastoma, skin cancer, lymphomas, choriocarcinomas, head and neck cancers, osteogenic sarcomas, and blood cancers. Non-limiting examples of specific types of liver cancer include hepatocellular carcinoma (HCC), secondary liver cancer (*e.g.,* caused by metastasis of some other non-liver cancer cell type), and hepatoblastoma. As used herein, a "tumor" comprises one or more cancerous cells.

Certain embodiments provide a pharmaceutical composition comprising a lipid nanoparticle as described herein, and a pharmaceutically acceptable carrier.

In certain embodiments, the pharmaceutical composition is formulated for intravenous administration.

Also described for reference purposes is a method for delivering a nucleic acid to a cell comprising contacting the cell with a lipid nanoparticle as described herein.

Certain embodiments provide a lipid nanoparticle as described herein for use in a method for treating a disease characterized a deficiency of a functional protein (*e.g.,* caused by a genetic defect), the method comprising: administering to a subject having the disease, the lipid nanoparticle as described herein, wherein the lipid nanoparticle comprises mRNA that encodes the functional protein or a protein having the same biological activity as the functional protein.

Certain embodiments provide a lipid nanoparticle as described herein for use in a method for treating a disease characterized by overexpression of a polypeptide, comprising administering to a subject having the disease the lipid nanoparticle as described herein, wherein the lipid nanoparticle comprises siRNA that targets expression of the overexpressed polypeptide.

Certain embodiments provide a lipid nanoparticle as described herein for use in the therapeutic or prophylactic treatment of a disease characterized by a deficiency of a functional protein (*e.g.,* caused by a genetic defect).

Certain embodiments provide a lipid nanoparticle as described herein for use in the therapeutic or prophylactic treatment of a disease characterized by overexpression of a polypeptide.

In certain embodiments, the nucleic acid is fully encapsulated within the lipid portion of the lipid particle such that the nucleic acid in the lipid particle is resistant in aqueous solution to enzymatic degradation, *e.g.,* by a nuclease or protease. In certain other embodiments, the lipid particles are substantially non-toxic to mammals such as humans.

In certain instances, the nucleic acid comprises an interfering RNA molecule such as, *e.g.,* an siRNA, aiRNA, miRNA, or mixtures thereof. In certain other instances, the nucleic acid comprises single-stranded or double-stranded DNA, RNA, or a DNA/RNA hybrid such as, *e.g.,* an antisense oligonucleotide, a ribozyme, a plasmid, an immunostimulatory oligonucleotide, or mixtures thereof. In certain other instances, the nucleic acid comprises one or more mRNA molecules (*e.g.,* a cocktail).

In one embodiment, the nucleic acid comprises an siRNA. In one embodiment, the siRNA molecule comprises a double-stranded region of about 15 to about 60 nucleotides in length (*e.g.,* about 15-60, 15-50, 15-40, 15-30, 15-25, or 19-25 nucleotides in length, or 15, 16, 17, 18, 19, 20, 21, 22, 23, 24, or 25 nucleotides in length). The siRNA molecules of the invention are capable of silencing the expression of a target sequence in vitro and/or in vivo.

In some embodiments, the siRNA molecule comprises at least one modified nucleotide. In certain preferred embodiments, the siRNA molecule comprises one, two, three, four, five, six, seven, eight, nine, ten, or more modified nucleotides in the double-stranded region. In certain instances, the siRNA comprises from about 1% to about 100% (*e.g.,* about 1%, 5%, 10%, 15%, 20%, 25%, 30%, 35%, 40%, 45%, 50%, 55%, 60%, 65%, 70%, 75%, 80%, 85%, 90%, 95%, or 100%) modified nucleotides in the double-stranded region. In preferred embodiments, less than about 25% (*e.g.,* less than about 25%, 20%, 15%, 10%, or 5%) or from about 1% to about 25% (*e.g.,* from about 1%-25%, 5%-25%, 10%-25%, 15%-25%, 20%-25%, or 10%-20%) of the nucleotides in the double-stranded region comprise modified nucleotides.

In other embodiments, the siRNA molecule comprises modified nucleotides including, but not limited to, 2'-O-methyl (2'OMe) nucleotides, 2'-deoxy-2'-fluoro (2'F) nucleotides, 2'-deoxy nucleotides, 2'-O-(2-methoxyethyl) (MOE) nucleotides, locked nucleic acid (LNA) nucleotides, and mixtures thereof. In preferred embodiments, the siRNA comprises 2'OMe nucleotides (*e.g.,* 2'OMe purine and/or pyrimidine nucleotides) such as, for example, 2'OMe-guanosine nucleotides, 2'OMe-uridine nucleotides, 2'OMe-adenosine nucleotides, 2'OMe-cytosine nucleotides, and mixtures thereof. In certain instances, the siRNA does not comprise 2'OMe-cytosine nucleotides. In other embodiments, the siRNA comprises a hairpin loop structure.

The siRNA may comprise modified nucleotides in one strand (i.e., sense or antisense) or both strands of the double-stranded region of the siRNA molecule. Preferably, uridine and/or guanosine nucleotides are modified at selective positions in the double-stranded region of the siRNA duplex. With regard to uridine nucleotide modifications, at least one, two, three, four, five, six, or more of the uridine nucleotides in the sense and/or antisense strand can be a modified uridine nucleotide such as a 2'OMe-uridine nucleotide. In some embodiments, every uridine nucleotide in the sense and/or antisense strand is a 2'OMe-uridine nucleotide. With regard to guanosine nucleotide modifications, at least one, two, three, four, five, six, or more of the guanosine nucleotides in the sense and/or antisense strand can be a modified guanosine nucleotide such as a 2'OMe-guanosine nucleotide. In some embodiments, every guanosine nucleotide in the sense and/or antisense strand is a 2'OMe-guanosine nucleotide.

In certain embodiments, at least one, two, three, four, five, six, seven, or more 5'-GU-3' motifs in an siRNA sequence may be modified, *e.g.,* by introducing mismatches to eliminate the 5'-GU-3' motifs and/or by introducing modified nucleotides such as 2'OMe nucleotides. The 5'-GU-3' motif can be in the sense strand, the antisense strand, or both strands of the siRNA sequence. The 5'-GU-3' motifs may be adjacent to each other or, alternatively, they may be separated by 1, 2, 3, 4, 5, 6, 7, 8, 9, 10, 11, 12, or more nucleotides.

In some preferred embodiments, a modified siRNA molecule is less immunostimulatory than a corresponding unmodified siRNA sequence. In such embodiments, the modified siRNA molecule with reduced immunostimulatory properties advantageously retains RNAi activity against the target sequence. In another embodiment, the immunostimulatory properties of the modified siRNA molecule and its ability to silence target gene expression can be balanced or optimized by the introduction of minimal and selective 2'OMe modifications within the siRNA sequence such as, e.g., within the double-stranded region of the siRNA duplex. In certain instances, the modified siRNA is at least about 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, or 100% less immunostimulatory than the corresponding unmodified siRNA. It will be readily apparent to those of skill in the art that the immunostimulatory properties of the modified siRNA molecule and the corresponding unmodified siRNA molecule can be determined by, for example, measuring INF-α and/or IL-6 levels from about two to about twelve hours after systemic administration in a mammal or transfection of a mammalian responder cell using an appropriate lipid-based delivery system (such as the LNP delivery system disclosed herein).

In certain embodiments, a modified siRNA molecule has an IC₅₀ (i.e., half-maximal inhibitory concentration) less than or equal to ten-fold that of the corresponding unmodified siRNA (i.e., the modified siRNA has an IC₅₀ that is less than or equal to ten-times the IC₅₀ of the corresponding unmodified siRNA). In other embodiments, the modified siRNA has an IC₅₀ less than or equal to three-fold that of the corresponding unmodified siRNA sequence. In yet other embodiments, the modified siRNA has an IC₅₀ less than or equal to two-fold that of the corresponding unmodified siRNA. It will be readily apparent to those of skill in the art that a dose-response curve can be generated and the IC₅₀ values for the modified siRNA and the corresponding unmodified siRNA can be readily determined using methods known to those of skill in the art.

In yet another embodiment, a modified siRNA molecule is capable of silencing at least about 5%, 10%, 15%, 20%, 25%, 30%, 35%, 40%, 45%, 50%, 55%, 60%, 65%, 70%, 75%, 80%, 85%, 90%, 95%, or 100% of the expression of the target sequence relative to the corresponding unmodified siRNA sequence.

In some embodiments, the siRNA molecule does not comprise phosphate backbone modifications, *e.g.,* in the sense and/or antisense strand of the double-stranded region. In other embodiments, the siRNA comprises one, two, three, four, or more phosphate backbone modifications, *e.g.,* in the sense and/or antisense strand of the double-stranded region. In preferred embodiments, the siRNA does not comprise phosphate backbone modifications.

In further embodiments, the siRNA does not comprise 2'-deoxy nucleotides, *e.g.,* in the sense and/or antisense strand of the double-stranded region. In yet further embodiments, the siRNA comprises one, two, three, four, or more 2'-deoxy nucleotides, *e.g.,* in the sense and/or antisense strand of the double-stranded region. In preferred embodiments, the siRNA does not comprise 2'-deoxy nucleotides.

In certain instances, the nucleotide at the 3'-end of the double-stranded region in the sense and/or antisense strand is not a modified nucleotide. In certain other instances, the nucleotides near the 3'-end (*e.g.,* within one, two, three, or four nucleotides of the 3'-end) of the double-stranded region in the sense and/or antisense strand are not modified nucleotides.

The siRNA molecules described herein may have 3' overhangs of one, two, three, four, or more nucleotides on one or both sides of the double-stranded region, or may lack overhangs (i.e., have blunt ends) on one or both sides of the double-stranded region. Preferably, the siRNA has 3' overhangs of two nucleotides on each side of the double-stranded region. In certain instances, the 3' overhang on the antisense strand has complementarity to the target sequence and the 3' overhang on the sense strand has complementarity to a complementary strand of the target sequence. Alternatively, the 3' overhangs do not have complementarity to the target sequence or the complementary strand thereof. In some embodiments, the 3' overhangs comprise one, two, three, four, or more nucleotides such as 2'-deoxy (2'H) nucleotides. In certain preferred embodiments, the 3' overhangs comprise deoxythymidine (dT) and/or uridine nucleotides. In other embodiments, one or more of the nucleotides in the 3' overhangs on one or both sides of the double-stranded region comprise modified nucleotides. Non-limiting examples of modified nucleotides are described above and include 2'OMe nucleotides, 2'-deoxy-2'F nucleotides, 2'-deoxy nucleotides, 2'-O-2-MOE nucleotides, LNA nucleotides, and mixtures thereof. In preferred embodiments, one, two, three, four, or more nucleotides in the 3' overhangs present on the sense and/or antisense strand of the siRNA comprise 2'OMe nucleotides (*e.g.,* 2'OMe purine and/or pyrimidine nucleotides) such as, for example, 2'OMe-guanosine nucleotides, 2'OMe-uridine nucleotides, 2'OMe-adenosine nucleotides, 2'OMe-cytosine nucleotides, and mixtures thereof.

The siRNA may comprise at least one or a cocktail (*e.g.,* at least two, three, four, five, six, seven, eight, nine, ten, or more) of unmodified and/or modified siRNA sequences that silence target gene expression. The cocktail of siRNA may comprise sequences which are directed to the same region or domain (*e.g.,* a "hot spot") and/or to different regions or domains of one or more target genes. In certain instances, one or more (*e.g.,* at least two, three, four, five, six, seven, eight, nine, ten, or more) modified siRNA that silence target gene expression are present in a cocktail. In certain other instances, one or more (*e.g.,* at least two, three, four, five, six, seven, eight, nine, ten, or more) unmodified siRNA sequences that silence target gene expression are present in a cocktail.

In some embodiments, the antisense strand of the siRNA molecule comprises or consists of a sequence that is at least about 80%, 85%, 90%, 95%, 96%, 97%, 98%, or 99% complementary to the target sequence or a portion thereof. In other embodiments, the antisense strand of the siRNA molecule comprises or consists of a sequence that is 100% complementary to the target sequence or a portion thereof. In further embodiments, the antisense strand of the siRNA molecule comprises or consists of a sequence that specifically hybridizes to the target sequence or a portion thereof.

In further embodiments, the sense strand of the siRNA molecule comprises or consists of a sequence that is at least about 80%, 85%, 90%, 95%, 96%, 97%, 98%, or 99% identical to the target sequence or a portion thereof. In additional embodiments, the sense strand of the siRNA molecule comprises or consists of a sequence that is 100% identical to the target sequence or a portion thereof.

Examples of cholesterol derivatives include, but are not limited to, cholestanol, cholestanone, cholestenone, coprostanol, cholesteryl-2'-hydroxyethyl ether, cholesteryl-4'-hydroxybutyl ether, and mixtures thereof. The synthesis of cholesteryl-2'-hydroxyethyl ether is described herein.

As used herein, DSPC means distearoylphosphatidylcholine.

In the lipid particles of the invention, the nucleic acid may be fully encapsulated within the lipid portion of the particle, thereby protecting the nucleic acid from enzymatic degradation. In preferred embodiments, a LNP comprising a nucleic acid, such as an interfering RNA (*e.g.,* siRNA) or mRNA, is fully encapsulated within the lipid portion of the particle, thereby protecting the nucleic acid from nuclease degradation. In certain instances, the nucleic acid in the LNP is not substantially degraded after exposure of the particle to a nuclease at 37° C. for at least about 20, 30, 45, or 60 minutes. In certain other instances, the nucleic acid in the LNP is not substantially degraded after incubation of the particle in serum at 37° C. for at least about 30, 45, or 60 minutes or at least about 2, 3, 4, 5, 6, 7, 8, 9, 10, 12, 14, 16, 18, 20, 22, 24, 26, 28, 30, 32, 34, or 36 hours. In other embodiments, the nucleic acid (*e.g.,* nucleic acid, such as siRNA or mRNA) is complexed with the lipid portion of the particle. One of the benefits of the formulations of the present invention is that the lipid particle compositions are substantially non-toxic to mammals such as humans.

The term "fully encapsulated" indicates that the nucleic acid in the lipid particle is not significantly degraded after exposure to serum or a nuclease or protease assay that would significantly degrade free DNA, RNA, or protein. In a fully encapsulated system, preferably less than about 25% of the nucleic acid in the particle is degraded in a treatment that would normally degrade 100% of free nucleic acid, more preferably less than about 10%, and most preferably less than about 5% of the nucleic acid in the particle is degraded. In the context of nucleic acid therapeutic agents, full encapsulation may be determined by an Oligreen^{®} assay. Oligreen^{®} is an ultra-sensitive fluorescent nucleic acid stain for quantitating oligonucleotides and single-stranded DNA or RNA in solution (available from Invitrogen Corporation; Carlsbad, Calif.). "Fully encapsulated" also indicates that the lipid particles are serum-stable, that is, that they do not rapidly decompose into their component parts upon in vivo administration.

In another aspect, the present invention provides a lipid particle (*e.g.,* LNP) composition comprising a plurality of lipid particles. In preferred embodiments, the nucleic acid (*e.g.,* nucleic acid) is fully encapsulated within the lipid portion of the lipid particles (*e.g.,* LNP), such that from about 30% to about 100%, from about 40% to about 100%, from about 50% to about 100%, from about 60% to about 100%, from about 70% to about 100%, from about 80% to about 100%, from about 90% to about 100%, from about 30% to about 95%, from about 40% to about 95%, from about 50% to about 95%, from about 60% to about 95%, %, from about 70% to about 95%, from about 80% to about 95%, from about 85% to about 95%, from about 90% to about 95%, from about 30% to about 90%, from about 40% to about 90%, from about 50% to about 90%, from about 60% to about 90%, from about 70% to about 90%, from about 80% to about 90%, or at least about 30%, 35%, 40%, 45%, 50%, 55%, 60%, 65%, 70%, 75%, 80%, 85%, 90%, 91%, 92%, 93%, 94%, 95%, 96%, 97%, 98%, or 99% (or any fraction thereof or range therein) of the lipid particles (*e.g.,* LNP) have the nucleic acid encapsulated therein.

Typically, the lipid particles (*e.g.,* LNP) of the invention have a lipid:active agent (*e.g.,* lipid:nucleic acid) ratio (mass/mass ratio) of from about 1 to about 100. In some instances, the lipid:active agent (*e.g.,* lipid:nucleic acid) ratio (mass/mass ratio) ranges from about 1 to about 50, from about 2 to about 25, from about 3 to about 20, from about 4 to about 15, or from about 5 to about 10.

Typically, the lipid particles (e.g., LNP) of the invention have a mean diameter of from about 40 nm to about 150 nm. In some embodiments, the lipid particles of the invention have a mean diameter of from about 40 nm to about 130 nm, from about 40 nm to about 120 nm, from about 40 nm to about 100 nm, from about 50 nm to about 120 nm, from about 50 nm to about 100 nm, from about 40 nm to about 80 nm, from about 40 nm to about 60 nm, from about 50 nm to about 60 nm, from about 60 nm to about 120 nm, from about 60 nm to about 110 nm, from about 60 nm to about 100 nm, from about 60 nm to about 90 nm, from about 60 nm to about 80 nm, from about 70 nm to about 120 nm, from about 70 nm to about 110 nm, from about 70 nm to about 100 nm, from about 70 nm to about 90 nm, from about 70 nm to about 80 nm, or less than about 150 nm, 120 nm, 110 nm, 100 nm, 90 nm, or 80 nm (or any fraction thereof or range therein).The present invention also provides a pharmaceutical composition comprising a lipid particle (*e.g.,* LNP) described herein and a pharmaceutically acceptable carrier.

In a preferred embodiment, the mode of administration includes, but is not limited to, oral, intranasal, intravenous, intraperitoneal, intramuscular, intra-articular, intralesional, intratracheal, subcutaneous, and intradermal. Preferably, the mammalian subject is a human.

In one embodiment, at least about 5%, 10%, 15%, 20%, or 25% of the total injected dose of the lipid particles (*e.g.,* LNP) is present in plasma about 8, 12, 24, 36, or 48 hours after injection. In other embodiments, more than about 20%, 30%, 40% and as much as about 60%, 70% or 80% of the total injected dose of the lipid particles (*e.g.,* LNP) is present in plasma about 8, 12, 24, 36, or 48 hours after injection. In certain instances, more than about 10% of a plurality of the particles is present in the plasma of a mammal about 1 hour after administration. In certain other instances, the presence of the lipid particles (*e.g.,* LNP) is detectable at least about 1 hour after administration of the particle. In certain embodiments, the presence of an nucleic acid, such as an interfering RNA (*e.g.,* siRNA) or mRNA is detectable in cells of the at about 8, 12, 24, 36, 48, 60, 72 or 96 hours after administration (*e.g.,* lung, liver, tumor, or at a site of inflammation). In other embodiments, downregulation of expression of a target sequence by an nucleic acid, such as an interfering RNA (*e.g.,* siRNA) is detectable at about 8, 12, 24, 36, 48, 60, 72 or 96 hours after administration. In yet other embodiments, downregulation of expression of a target sequence by an nucleic acid such as an interfering RNA (*e.g.,* siRNA) occurs preferentially in tumor cells or in cells at a site of inflammation. In further embodiments, the presence or effect of an nucleic acid such as an interfering RNA (*e.g.,* siRNA) in cells at a site proximal or distal to the site of administration or in cells of the lung, liver, or a tumor is detectable at about 12, 24, 48, 72, or 96 hours, or at about 6, 8, 10, 12, 14, 16, 18, 19, 20, 22, 24, 26, or 28 days after administration. In other embodiments, upregulation of expression of a target sequence by an nucleic acid, such as an mRNA or self-amplifying RNA is detectable at about 8, 12, 24, 36, 48, 60, 72 or 96 hours after administration. In yet other embodiments, upregulation of expression of a target sequence by an nucleic acid such as an mRNA or self-amplifying RNA occurs preferentially in tumor cells or in cells at a site of inflammation. In further embodiments, the presence or effect of an nucleic acid such as an mRNA or self-amplifying RNA in cells at a site proximal or distal to the site of administration or in cells of the lung, liver, or a tumor is detectable at about 12, 24, 48, 72, or 96 hours, or at about 6, 8, 10, 12, 14, 16, 18, 19, 20, 22, 24, 26, or 28 days after administration. In additional embodiments, the lipid particles (*e.g.,* LNP) of the invention are administered parenterally or intraperitoneally. In embodiments, the lipid particles (*e.g.,* LNP) of the invention are administered intramuscularly.

In some embodiments, the lipid particles (*e.g.,* LNP) of the invention are useful in methods for the therapeutic delivery of one or more nucleic acids comprising an interfering RNA sequence (*e.g.,* siRNA). In particular, one object of this invention to provide in vitro and in vivo methods for treatment of a disease or disorder in a mammal (*e.g.,* a rodent such as a mouse or a primate such as a human, chimpanzee, or monkey) by downregulating or silencing the transcription and/or translation of one or more target nucleic acid sequences or genes of interest. As a non-limiting example, the methods are useful for in vivo delivery of interfering RNA (*e.g.,* siRNA) to the liver and/or tumor of a mammalian subject. In certain embodiments, the disease or disorder is associated with expression and/or overexpression of a gene and expression or overexpression of the gene is reduced by the interfering RNA (*e.g.,* siRNA). In certain other embodiments, a therapeutically effective amount of the lipid particle (*e.g.,* LNP) may be administered to the mammal. In some instances, an interfering RNA (*e.g.,* siRNA) is formulated into a LNP, and the particles are administered to patients requiring such treatment. In other instances, cells are removed from a patient, the interfering RNA (*e.g.,* siRNA) is delivered in vitro (*e.g.,* using a LNP described herein), and the cells are reinjected into the patient.

In an additional aspect, the present invention provides lipid particles (*e.g.,* LNP) comprising asymmetrical interfering RNA (aiRNA) molecules that silence the expression of a target gene and methods of using such particles to silence target gene expression.

In one embodiment, the aiRNA molecule comprises a double-stranded (duplex) region of about 10 to about 25 (base paired) nucleotides in length, wherein the aiRNA molecule comprises an antisense strand comprising 5' and 3' overhangs, and wherein the aiRNA molecule is capable of silencing target gene expression.

In certain instances, the aiRNA molecule comprises a double-stranded (duplex) region of about 12-20, 12-19, 12-18, 13-17, or 14-17 (base paired) nucleotides in length, more typically 12, 13, 14, 15, 16, 17, 18, 19, or 20 (base paired) nucleotides in length. In certain other instances, the 5' and 3' overhangs on the antisense strand comprise sequences that are complementary to the target RNA sequence, and may optionally further comprise nontargeting sequences. In some embodiments, each of the 5' and 3' overhangs on the antisense strand comprises or consists of one, two, three, four, five, six, seven, or more nucleotides.

In other embodiments, the aiRNA molecule comprises modified nucleotides selected from the group consisting of 2'0OMe nucleotides, 2'F nucleotides, 2'-deoxy nucleotides, 2'-O-MOE nucleotides, LNA nucleotides, and mixtures thereof. In a preferred embodiment, the aiRNA molecule comprises 2'OMe nucleotides. As a non-limiting example, the 2'OMe nucleotides may be selected from the group consisting of 2'OMe-guanosine nucleotides, 2'OMe-uridine nucleotides, and mixtures thereof.

In a related aspect, the present invention provides lipid particles (*e.g.,* LNP) comprising microRNA (miRNA) molecules that silence the expression of a target gene, and which are useful in methods of using such compositions to silence target gene expression.

In one embodiment, the miRNA molecule comprises about 15 to about 60 nucleotides in length, wherein the miRNA molecule is capable of silencing target gene expression.

In certain instances, the miRNA molecule comprises about 15-50, 15-40, or 15-30 nucleotides in length, more typically about 15-25 or 19-25 nucleotides in length, and are preferably about 20-24, 21-22, or 21-23 nucleotides in length. In a preferred embodiment, the miRNA molecule is a mature miRNA molecule targeting an RNA sequence of interest.

In some embodiments, the miRNA molecule comprises modified nucleotides selected from the group consisting of 2'0OMe nucleotides, 2'F nucleotides, 2'-deoxy nucleotides, 2'-O-MOE nucleotides, LNA nucleotides, and mixtures thereof. In a preferred embodiment, the miRNA molecule comprises 2'OMe nucleotides. As a non-limiting example, the 2'OMe nucleotides may be selected from the group consisting of 2'OMe-guanosine nucleotides, 2'OMe-uridine nucleotides, and mixtures thereof.

In some embodiments, the lipid particles (*e.g.,* LNP) of the invention are useful in methods for the therapeutic delivery of one or more mRNA molecules. In particular, it is one object of this invention to provide in vitro and in vivo methods for treatment of a disease or disorder in a mammal (*e.g.,* a rodent such as a mouse or a primate such as a human, chimpanzee, or monkey) through the expression of one or more target proteins. As a non-limiting example, the methods are useful for in vivo delivery of one or more mRNA molecules to a mammalian subject. In certain other embodiments, a therapeutically effective amount of the lipid particle (*e.g.,* LNP) may be administered to the mammal. In some instances, one or more mRNA molecules are formulated into a LNP, and the particles are administered to patients requiring such treatment. In other instances, cells are removed from a patient, one or more mRNA molecules are delivered in vitro (*e.g.,* using a LNP described herein), and the cells are reinjected into the patient.

In other embodiments, the siRNA molecule comprises modified nucleotides selected from the group consisting of 2'0OMe nucleotides, 2'F nucleotides, 2'-deoxy nucleotides, 2'-O-MOE nucleotides, LNA nucleotides, and mixtures thereof. In a related aspect, the present invention provides lipid particles (*e.g.,* LNP) comprising microRNA (miRNA) molecules that silence the expression of a target gene and methods of using such compositions to silence target gene expression.

As such, the lipid particles of the invention (e.g., LNP) are advantageous and suitable for use in the administration of active agents or therapeutic agents, such as nucleic acid (e.g., interfering RNA such as siRNA, aiRNA, and/or miRNA; or mRNA; guide RNA; self-amplifying RNA; circular RNA; DNA, including plasmid DNA and closed ended DNA; to a subject (e.g., a mammal such as a human) because they are stable in circulation, of a size required for pharmacodynamic behavior resulting in access to extravascular sites, and are capable of reaching target cell populations.

In the context of this invention, the terms "polynucleotide" and "oligonucleotide" refer to a polymer or oligomer of nucleotide or nucleoside monomers consisting of naturally-occurring bases, sugars and intersugar (backbone) linkages. The terms "polynucleotide" and "oligonucleotide" also include polymers or oligomers comprising non-naturally occurring monomers, or portions thereof, which function similarly. Such modified or substituted oligonucleotides are often preferred over native forms because of properties such as, for example, enhanced cellular uptake, reduced immunogenicity, and increased stability in the presence of nucleases.

Oligonucleotides are generally classified as deoxyribooligonucleotides or ribooligonucleotides. A deoxyribooligonucleotide consists of a 5-carbon sugar called deoxyribose joined covalently to phosphate at the 5' and 3' carbons of this sugar to form an alternating, unbranched polymer. A ribooligonucleotide consists of a similar repeating structure where the 5-carbon sugar is ribose.

The nucleic acid that is present in a lipid-nucleic acid particle according to this invention includes any form of nucleic acid that is known. The nucleic acids used herein can be single-stranded DNA or RNA, or double-stranded DNA or RNA, or DNA-RNA hybrids. Examples of double-stranded DNA are described herein and include, *e.g.,* structural genes, genes including control and termination regions, and self-replicating systems such as viral or plasmid DNA. Examples of double-stranded RNA are described herein and include, *e.g.,* siRNA and other RNAi agents such as aiRNA and pre-miRNA. Single-stranded nucleic acids include, *e.g.,* antisense oligonucleotides, ribozymes, mature miRNA, and triplex-forming oligonucleotides.

Nucleic acids may be of various lengths, generally dependent upon the particular form of nucleic acid. For example, in particular embodiments, plasmids or genes may be from about 1,000 to about 100,000 nucleotide residues in length. In particular embodiments, oligonucleotides may range from about 10 to about 100 nucleotides in length. In various related embodiments, oligonucleotides, both single-stranded, double-stranded, and triple-stranded, may range in length from about 10 to about 60 nucleotides, from about 15 to about 60 nucleotides, from about 20 to about 50 nucleotides, from about 15 to about 30 nucleotides, or from about 20 to about 30 nucleotides in length.

In particular embodiments, an oligonucleotide (or a strand thereof) of the invention specifically hybridizes to or is complementary to a target polynucleotide sequence. The terms "specifically hybridizable" and "complementary" as used herein indicate a sufficient degree of complementarity such that stable and specific binding occurs between the DNA or RNA target and the oligonucleotide. It is understood that an oligonucleotide need not be 100% complementary to its target nucleic acid sequence to be specifically hybridizable. In preferred embodiments, an oligonucleotide is specifically hybridizable when binding of the oligonucleotide to the target sequence interferes with the normal function of the target sequence to cause a loss of utility or expression therefrom, and there is a sufficient degree of complementarity to avoid non-specific binding of the oligonucleotide to non-target sequences under conditions in which specific binding is desired, i.e., under physiological conditions in the case of in vivo assays or therapeutic treatment, or, in the case of in vitro assays, under conditions in which the assays are conducted. Thus, the oligonucleotide may include 1, 2, 3, or more base substitutions as compared to the region of a gene or mRNA sequence that it is targeting or to which it specifically hybridizes.

### siRNA

The siRNA component of the lipid nanoparticles of the present invention is capable of silencing the expression of a target gene of interest. Each strand of the siRNA duplex is typically about 15 to about 60 nucleotides in length, preferably about 15 to about 30 nucleotides in length. In certain embodiments, the siRNA comprises at least one modified nucleotide. The modified siRNA is generally less immunostimulatory than a corresponding unmodified siRNA sequence and retains RNAi activity against the target gene of interest. In some embodiments, the modified siRNA contains at least one 2'OMe purine or pyrimidine nucleotide such as a 2'OMe-guanosine, 2'OMe-uridine, 2'OMe-adenosine, and/or 2'OMe-cytosine nucleotide. In preferred embodiments, one or more of the uridine and/or guanosine nucleotides are modified. The modified nucleotides can be present in one strand (i.e., sense or antisense) or both strands of the siRNA. The siRNA sequences may have overhangs (*e.g.,* 3' or 5' overhangs as described in Elbashir et al., Genes Dev., 15:188 (2001) or Nykänen et al., Cell, 107:309 (2001)), or may lack overhangs (i.e., have blunt ends).

The modified siRNA generally comprises from about 1% to about 100% (*e.g.,* about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, or 100%) modified nucleotides in the double-stranded region of the siRNA duplex. In certain embodiments, one, two, three, four, five, six, seven, eight, nine, ten, or more of the nucleotides in the double-stranded region of the siRNA comprise modified nucleotides.

In some embodiments, less than about 25% (*e.g.,* less than about 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, or 1%) of the nucleotides in the double-stranded region of the siRNA comprise modified nucleotides.

In other embodiments, from about 1% to about 25% (*e.g.,* from about 1%-25%, 2%-25%, 3%-25%, 4%-25%, 5%-25%, 6%-25%, 7%-25%, 8%-25%, 9%-25%, 10%-25%, 11%-25%, 12%-25%, 13%-25%, 14%-25%, 15%-25%, 16%-25%, 17%-25%, 18%-25%, 19%-25%, 20%-25%, 21%-25%, 22%-25%, 23%-25%, 24%-25%, etc.) or from about 1% to about 20% (*e.g.,* from about 1%-20%, 2%-20%, 3%-20%, 4%-20%, 5%-20%, 6%-20%, 7%-20%, 8%-20%, 9%-20%, 10%-20%, 11%-20%, 12%-20%, 13%-20%, 14%-20%, 15%-20%, 16%-20%, 17%-20%, 18%-20%, 19%-20%, 1%-19%, 2%-19%, 3%-19%, 4%-19%, 5%-19%, 6%-19%, 7%-19%, 8%-19%, 9%-19%, 10%-19%, 11%-19%, 12%-19%, 13%-19%, 14%-19%, 15%-19%, 16%-19%, 17%-19%, 18%-19%, 1%-18%, 2%-18%, 3%-18%, 4%-18%, 5%-18%, 6%-18%, 7%-18%, 8%-18%, 9%-18%, 10%-18%, 11%-18%, 12%-18%, 13%-18%, 14%-18%, 15%-18%, 16%-18%, 17%-18%, 1%-17%, 2%-17%, 3%-17%, 4%-17%, 5%-17%, 6%-17%, 7%-17%, 8%-17%, 9%-17%, 10%-17%, 11%-17%, 12%-17%, 13%-17%, 14%-17%, 15%-17%, 16%-17%, 1%-16%, 2%-16%, 3%-16%, 4%-16%, 5%-16%, 6%-16%, 7%-16%, 8%-16%, 9%-16%, 10%-16%, 11%-16%, 12%-16%, 13%-16%, 14%-16%, 15%-16%, 1%-15%, 2%-15%, 3%-15%, 4%-15%, 5%-15%, 6%-15%, 7%-15%, 8%-15%, 9%-15%, 10%-15%, 11%-15%, 12%-15%, 13%-15%, 14%-15%, etc.) of the nucleotides in the double-stranded region of the siRNA comprise modified nucleotides.

In further embodiments, *e.g.,* when one or both strands of the siRNA are selectively modified at uridine and/or guanosine nucleotides, the resulting modified siRNA can comprise less than about 30% modified nucleotides (*e.g.,* less than about 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, or 1% modified nucleotides) or from about 1% to about 30% modified nucleotides (*e.g.,* from about 1%-30%, 2%-30%, 3%-30%, 4%-30%, 5%-30%, 6%-30%, 7%-30%, 8%-30%, 9%-30%, 10%-30%, 11%-30%, 12%-30%, 13%-30%, 14%-30%, 15%-30%, 16%-30%, 17%-30%, 18%-30%, 19%-30%, 20%-30%, 21%-30%, 22%-30%, 23%-30%, 24%-30%, 25%-30%, 26%-30%, 27%-30%, 28%-30%, or 29%-30% modified nucleotides).

### Selection of siRNA Sequences

Suitable siRNA sequences can be identified using any means known in the art. Typically, the methods described in Elbashir et al., Nature, 411:494-498 (2001) and Elbashir et al., EMBO J., 20:6877-6888 (2001) are combined with rational design rules set forth in Reynolds et al., Nature Biotech., 22(3):326-330 (2004).

Generally, the nucleotide sequence 3' of the AUG start codon of a transcript from the target gene of interest is scanned for dinucleotide sequences (*e.g.,* AA, NA, CC, GG, or UU, wherein N=C, G, or U) (see, *e.g.,* Elbashir et al., EMBO J., 20:6877-6888 (2001)). The nucleotides immediately 3' to the dinucleotide sequences are identified as potential siRNA sequences (i.e., a target sequence or a sense strand sequence). Typically, the 19, 21, 23, 25, 27, 29, 31, 33, 35, or more nucleotides immediately 3' to the dinucleotide sequences are identified as potential siRNA sequences. In some embodiments, the dinucleotide sequence is an AA or NA sequence and the 19 nucleotides immediately 3' to the AA or NA dinucleotide are identified as potential siRNA sequences. siRNA sequences are usually spaced at different positions along the length of the target gene. To further enhance silencing efficiency of the siRNA sequences, potential siRNA sequences may be analyzed to identify sites that do not contain regions of homology to other coding sequences, *e.g.,* in the target cell or organism. For example, a suitable siRNA sequence of about 21 base pairs typically will not have more than 16-17 contiguous base pairs of homology to coding sequences in the target cell or organism. If the siRNA sequences are to be expressed from an RNA Pol III promoter, siRNA sequences lacking more than 4 contiguous A's or T's are selected.

Once a potential siRNA sequence has been identified, a complementary sequence (i.e., an antisense strand sequence) can be designed. A potential siRNA sequence can also be analyzed using a variety of criteria known in the art. For example, to enhance their silencing efficiency, the siRNA sequences may be analyzed by a rational design algorithm to identify sequences that have one or more of the following features: (1) G/C content of about 25% to about 60% G/C; (2) at least 3 A/Us at positions 15-19 of the sense strand; (3) no internal repeats; (4) an A at position 19 of the sense strand; (5) an A at position 3 of the sense strand; (6) a U at position 10 of the sense strand; (7) no G/C at position 19 of the sense strand; and (8) no G at position 13 of the sense strand. siRNA design tools that incorporate algorithms that assign suitable values of each of these features and are useful for selection of siRNA can be found at, *e.g.,* http://boz094.ust.hk/RNAi/siRNA. One of skill in the art will appreciate that sequences with one or more of the foregoing characteristics may be selected for further analysis and testing as potential siRNA sequences.

Additionally, potential siRNA sequences with one or more of the following criteria can often be eliminated as siRNA: (1) sequences comprising a stretch of 4 or more of the same base in a row; (2) sequences comprising homopolymers of Gs (i.e., to reduce possible non-specific effects due to structural characteristics of these polymers; (3) sequences comprising triple base motifs (*e.g.,* GGG, CCC, AAA, or TTT); (4) sequences comprising stretches of 7 or more G/Cs in a row; and (5) sequences comprising direct repeats of 4 or more bases within the candidates resulting in internal fold-back structures. However, one of skill in the art will appreciate that sequences with one or more of the foregoing characteristics may still be selected for further analysis and testing as potential siRNA sequences.

In some embodiments, potential siRNA sequences may be further analyzed based on siRNA duplex asymmetry as described in, *e.g.,* Khvorova et al., Cell, 115:209-216 (2003); and Schwarz et al., Cell, 115:199-208 (2003). In other embodiments, potential siRNA sequences may be further analyzed based on secondary structure at the target site as described in, *e.g.,* Luo et al., Biophys. Res. Commun., 318:303-310 (2004). For example, secondary structure at the target site can be modeled using the Mfold algorithm (available at http://www.bioinfo.rpi.edu/applications/mfold/rna/form1.cgi) to select siRNA sequences which favor accessibility at the target site where less secondary structure in the form of base-pairing and stem-loops is present.

Once a potential siRNA sequence has been identified, the sequence can be analyzed for the presence of any immunostimulatory properties, *e.g.,* using an in vitro cytokine assay or an in vivo animal model. Motifs in the sense and/or antisense strand of the siRNA sequence such as GU-rich motifs (*e.g.,* 5'-GU-3',5'-UGU-3',5'-GUGU-3',5'-UGUGU-3', etc.) can also provide an indication of whether the sequence may be immunostimulatory. Once an siRNA molecule is found to be immunostimulatory, it can then be modified to decrease its immunostimulatory properties as described herein. As a non-limiting example, an siRNA sequence can be contacted with a mammalian responder cell under conditions such that the cell produces a detectable immune response to determine whether the siRNA is an immunostimulatory or a non-immunostimulatory siRNA. The mammalian responder cell may be from a naive mammal (i.e., a mammal that has not previously been in contact with the gene product of the siRNA sequence). The mammalian responder cell may be, *e.g.,* a peripheral blood mononuclear cell (PBMC), a macrophage, and the like. The detectable immune response may comprise production of a cytokine or growth factor such as, *e.g.,* TNF-α, IFN-α, IFN-β, IFN-γ, IL-6, IL-12, or a combination thereof. An siRNA molecule identified as being immunostimulatory can then be modified to decrease its immunostimulatory properties by replacing at least one of the nucleotides on the sense and/or antisense strand with modified nucleotides. For example, less than about 30% (*e.g.,* less than about 30%, 25%, 20%, 15%, 10%, or 5%) of the nucleotides in the double-stranded region of the siRNA duplex can be replaced with modified nucleotides such as 2'OMe nucleotides. The modified siRNA can then be contacted with a mammalian responder cell as described above to confirm that its immunostimulatory properties have been reduced or abrogated.

Suitable in vitro assays for detecting an immune response include, but are not limited to, the double monoclonal antibody sandwich immunoassay technique of David et al. (U.S. Pat. No. 4,376,110); monoclonal-polyclonal antibody sandwich assays (Wide et al., in Kirkham and Hunter, eds., Radioimmunoassay Methods, E. and S. Livingstone, Edinburgh (1970)); the "Western blot" method of Gordon et al. (U.S. Pat. No. 4,452,901); immunoprecipitation of labeled ligand (Brown et al., J. Biol. Chem., 255:4980-4983 (1980)); enzyme-linked immunosorbent assays (ELISA) as described, for example, by Raines et al., J. Biol. Chem., 257:5154-5160 (1982); immunocytochemical techniques, including the use of fluorochromes (Brooks et al., Clin. Exp. Immunol., 39:477 (1980)); and neutralization of activity (Bowen-Pope et al., Proc. Natl. Acad. Sci. USA, 81:2396-2400 (1984)). In addition to the immunoassays described above, a number of other immunoassays are available, including those described in U.S. Pat. Nos. 3,817,827; 3,850,752; 3,901,654; 3,935,074; 3,984,533; 3,996,345; 4,034,074; and 4,098,876.

A non-limiting example of an in vivo model for detecting an immune response includes an in vivo mouse cytokine induction assay as described in, *e.g.,* Judge et al., Mol. Ther., 13:494-505 (2006). In certain embodiments, the assay that can be performed as follows: (1) siRNA can be administered by standard intravenous injection in the lateral tail vein; (2) blood can be collected by cardiac puncture about 6 hours after administration and processed as plasma for cytokine analysis; and (3) cytokines can be quantified using sandwich ELISA kits according to the manufacturer's instructions (*e.g.,* mouse and human IFN-α (PBL Biomedical; Piscataway, N.J.); human IL-6 and TNF-α (eBioscience; San Diego, Calif.); and mouse IL-6, TNF-α, and IFN-γ (BD Biosciences; San Diego, Calif.)).

Monoclonal antibodies that specifically bind cytokines and growth factors are commercially available from multiple sources and can be generated using methods known in the art (see, *e.g.,* Kohler et al., Nature, 256: 495-497 (1975) and Harlow and Lane, ANTIBODIES, A LABORATORY MANUAL, Cold Spring Harbor Publication, New York (1999)). Generation of monoclonal antibodies has been previously described and can be accomplished by any means known in the art (Buhring et al., in Hybridoma, Vol. 10, No. 1, pp. 77-78 (1991)). In some methods, the monoclonal antibody is labeled (*e.g.,* with any composition detectable by spectroscopic, photochemical, biochemical, electrical, optical, or chemical means) to facilitate detection.

### Generating siRNA Molecules

siRNA can be provided in several forms including, *e.g.,* as one or more isolated small-interfering RNA (siRNA) duplexes, as longer double-stranded RNA (dsRNA), or as siRNA or dsRNA transcribed from a transcriptional cassette in a DNA plasmid. The siRNA sequences may have overhangs (*e.g.,* 3' or 5' overhangs as described in Elbashir et al., Genes Dev., 15:188 (2001) or Nykänen et al., Cell, 107:309 (2001), or may lack overhangs (i.e., to have blunt ends).

An RNA population can be used to provide long precursor RNAs, or long precursor RNAs that have substantial or complete identity to a selected target sequence can be used to make the siRNA. The RNAs can be isolated from cells or tissue, synthesized, and/or cloned according to methods well known to those of skill in the art. The RNA can be a mixed population (obtained from cells or tissue, transcribed from cDNA, subtracted, selected, etc.), or can represent a single target sequence. RNA can be naturally occurring (*e.g.,* isolated from tissue or cell samples), synthesized in vitro (*e.g.,* using T7 or SP6 polymerase and PCR products or a cloned cDNA), or chemically synthesized.

To form a long dsRNA, for synthetic RNAs, the complement is also transcribed in vitro and hybridized to form a dsRNA. If a naturally occurring RNA population is used, the RNA complements are also provided (*e.g.,* to form dsRNA for digestion by *E. coli* RNAse III or Dicer), *e.g.,* by transcribing cDNAs corresponding to the RNA population, or by using RNA polymerases. The precursor RNAs are then hybridized to form double stranded RNAs for digestion. The dsRNAs can be directly administered to a subject or can be digested in vitro prior to administration.

Methods for isolating RNA, synthesizing RNA, hybridizing nucleic acids, making and screening cDNA libraries, and performing PCR are well known in the art (see, *e.g.,* Gubler and Hoffman, Gene, 25:263-269 (1983); Sambrook et al., supra; Ausubel et al., supra), as are PCR methods (see, U.S. Pat. Nos. 4,683,195 and 4,683,202; PCR Protocols: A Guide to Methods and Applications (Innis et al., eds, 1990)). Expression libraries are also well known to those of skill in the art. Additional basic texts disclosing the general methods of use in this invention include Sambrook et al., Molecular Cloning, A Laboratory Manual (2nd ed. 1989); Kriegler, Gene Transfer and Expression: A Laboratory Manual (1990); and Current Protocols in Molecular Biology (Ausubel et al., eds., 1994).

Preferably, siRNA are chemically synthesized. The oligonucleotides that comprise the siRNA molecules of the invention can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). The synthesis of oligonucleotides makes use of common nucleic acid protecting and coupling groups, such as dimethoxytrityl at the 5'-end and phosphoramidites at the 3'-end. As a non-limiting example, small scale syntheses can be conducted on an Applied Biosystems synthesizer using a 0.2 µmol scale protocol. Alternatively, syntheses at the 0.2 µmol scale can be performed on a 96-well plate synthesizer from Protogene (Palo Alto, Calif.). However, a larger or smaller scale of synthesis is also within the scope of this invention. Suitable reagents for oligonucleotide synthesis, methods for RNA deprotection, and methods for RNA purification are known to those of skill in the art.

siRNA molecules can also be synthesized via a tandem synthesis technique, wherein both strands are synthesized as a single continuous oligonucleotide fragment or strand separated by a cleavable linker that is subsequently cleaved to provide separate fragments or strands that hybridize to form the siRNA duplex. The linker can be a polynucleotide linker or a non-nucleotide linker. The tandem synthesis of siRNA can be readily adapted to both multiwell/multiplate synthesis platforms as well as large scale synthesis platforms employing batch reactors, synthesis columns, and the like. Alternatively, siRNA molecules can be assembled from two distinct oligonucleotides, wherein one oligonucleotide comprises the sense strand and the other comprises the antisense strand of the siRNA. For example, each strand can be synthesized separately and joined together by hybridization or ligation following synthesis and/or deprotection. In certain other instances, siRNA molecules can be synthesized as a single continuous oligonucleotide fragment, where the self-complementary sense and antisense regions hybridize to form an siRNA duplex having hairpin secondary structure.

### Modifying siRNA Sequences

In certain aspects, siRNA molecules comprise a duplex having two strands and at least one modified nucleotide in the double-stranded region, wherein each strand is about 15 to about 60 nucleotides in length. Advantageously, the modified siRNA is less immunostimulatory than a corresponding unmodified siRNA sequence, but retains the capability of silencing the expression of a target sequence. In preferred embodiments, the degree of chemical modifications introduced into the siRNA molecule strikes a balance between reduction or abrogation of the immunostimulatory properties of the siRNA and retention of RNAi activity. As a non-limiting example, an siRNA molecule that targets a gene of interest can be minimally modified (*e.g.,* less than about 30%, 25%, 20%, 15%, 10%, or 5% modified) at selective uridine and/or guanosine nucleotides within the siRNA duplex to eliminate the immune response generated by the siRNA while retaining its capability to silence target gene expression.

Examples of modified nucleotides suitable for use in the invention include, but are not limited to, ribonucleotides having a 2'-O-methyl (2'OMe), 2'-deoxy-2'-fluoro (2'F), 2'-deoxy, 5-C-methyl, 2'-O-(2-methoxyethyl) (MOE), 4'-thio, 2'-amino, or 2'-C-allyl group. Modified nucleotides having a Northern conformation such as those described in, *e.g.,* Saenger, Principles of Nucleic Acid Structure, Springer-Verlag Ed. (1984), are also suitable for use in siRNA molecules. Such modified nucleotides include, without limitation, locked nucleic acid (LNA) nucleotides (*e.g.,* 2'-O, 4'-C-methylene-(D-ribofuranosyl) nucleotides), 2'-O-(2-methoxyethyl) (MOE) nucleotides, 2'-methyl-thio-ethyl nucleotides, 2'-deoxy-2'-fluoro (2'F) nucleotides, 2'-deoxy-2'-chloro (2'Cl) nucleotides, and 2'-azido nucleotides. In certain instances, the siRNA molecules described herein include one or more G-clamp nucleotides. A G-clamp nucleotide refers to a modified cytosine analog wherein the modifications confer the ability to hydrogen bond both Watson-Crick and Hoogsteen faces of a complementary guanine nucleotide within a duplex (see, *e.g.,* Lin et al., J. Am. Chem. Soc., 120:8531-8532 (1998)). In addition, nucleotides having a nucleotide base analog such as, for example, C-phenyl, C-naphthyl, other aromatic derivatives, inosine, azole carboxamides, and nitroazole derivatives such as 3-nitropyrrole, 4-nitroindole, 5-nitroindole, and 6-nitroindole (see, *e.g.,* Loakes, Nucl. Acids Res., 29:2437-2447 (2001)) can be incorporated into siRNA molecules.

In certain embodiments, siRNA molecules may further comprise one or more chemical modifications such as terminal cap moieties, phosphate backbone modifications, and the like. Examples of terminal cap moieties include, without limitation, inverted deoxy abasic residues, glyceryl modifications, 4',5'-methylene nucleotides, 1-(β-D-erythrofuranosyl) nucleotides, 4'-thio nucleotides, carbocyclic nucleotides, 1,5-anhydrohexitol nucleotides, L-nucleotides, α-nucleotides, modified base nucleotides, threo-pentofuranosyl nucleotides, acyclic 3',4'-seco nucleotides, acyclic 3,4-dihydroxybutyl nucleotides, acyclic 3,5-dihydroxypentyl nucleotides, 3'-3'-inverted nucleotide moieties, 3'-3'-inverted abasic moieties, 3'-2'-inverted nucleotide moieties, 3'-2'-inverted abasic moieties, 5'-5'-inverted nucleotide moieties, 5'-5'-inverted abasic moieties, 3'-5'-inverted deoxy abasic moieties, 5'-amino-alkyl phosphate, 1,3-diamino-2-propyl phosphate, 3-aminopropyl phosphate, 6-aminohexyl phosphate, 1,2-aminododecyl phosphate, hydroxypropyl phosphate, 1,4-butanediol phosphate, 3'-phosphoramidate, 5'-phosphoramidate, hexylphosphate, aminohexyl phosphate, 3'-phosphate, 5'-amino, 3'-phosphorothioate, 5'-phosphorothioate, phosphorodithioate, and bridging or non-bridging methylphosphonate or 5'-mercapto moieties (see, *e.g.,* U.S. Pat. No. 5,998,203; Beaucage et al., Tetrahedron 49:1925 (1993)). Non-limiting examples of phosphate backbone modifications (i.e., resulting in modified internucleotide linkages) include phosphorothioate, phosphorodithioate, methylphosphonate, phosphotriester, morpholino, amidate, carbamate, carboxymethyl, acetamidate, polyamide, sulfonate, sulfonamide, sulfamate, formacetal, thioformacetal, and alkylsilyl substitutions (see, *e.g.,* Hunziker et al., Nucleic Acid Analogues: Synthesis and Properties, in Modern Synthetic Methods, VCH, 331-417 (1995); Mesmaeker et al., Novel Backbone Replacements for Oligonucleotides, in Carbohydrate Modifications in Antisense Research, ACS, 24-39 (1994)). Such chemical modifications can occur at the 5'-end and/or 3'-end of the sense strand, antisense strand, or both strands of the siRNA.

In some embodiments, the sense and/or antisense strand of the siRNA molecule can further comprise a 3'-terminal overhang having about 1 to about 4 (*e.g.,* 1, 2, 3, or 4) 2'-deoxy ribonucleotides and/or any combination of modified and unmodified nucleotides. Additional examples of modified nucleotides and types of chemical modifications that can be introduced into siRNA molecules are described, *e.g.,* in UK Patent No. GB 2,397,818 B and U.S. Patent Publication Nos. 20040192626, 20050282188, and 20070135372.

The siRNA molecules described herein can optionally comprise one or more non-nucleotides in one or both strands of the siRNA. As used herein, the term "non-nucleotide" refers to any group or compound that can be incorporated into a nucleic acid chain in the place of one or more nucleotide units, including sugar and/or phosphate substitutions, and allows the remaining bases to exhibit their activity. The group or compound is abasic in that it does not contain a commonly recognized nucleotide base such as adenosine, guanine, cytosine, uracil, or thymine and therefore lacks a base at the l'-position.

In other embodiments, chemical modification of the siRNA comprises attaching a conjugate to the siRNA molecule. The conjugate can be attached at the 5' and/or 3'-end of the sense and/or antisense strand of the siRNA via a covalent attachment such as, *e.g.,* a biodegradable linker. The conjugate can also be attached to the siRNA, *e.g.,* through a carbamate group or other linking group (see, *e.g.,* U.S. Patent Publication Nos. 20050074771, 20050043219, and 20050158727). In certain instances, the conjugate is a molecule that facilitates the delivery of the siRNA into a cell. Examples of conjugate molecules suitable for attachment to siRNA include, without limitation, steroids such as cholesterol, glycols such as polyethylene glycol (PEG), human serum albumin (HSA), fatty acids, carotenoids, terpenes, bile acids, folates (*e.g.,* folic acid, folate analogs and derivatives thereof), sugars (*e.g.,* galactose, galactosamine, N-acetyl galactosamine, glucose, mannose, fructose, fucose, etc.), phospholipids, peptides, ligands for cellular receptors capable of mediating cellular uptake, and combinations thereof (see, *e.g.,* U.S. Patent Publication Nos. 20030130186, 20040110296, and 20040249178; U.S. Pat. No. 6,753,423). Other examples include the lipophilic moiety, vitamin, polymer, peptide, protein, nucleic acid, small molecule, oligosaccharide, carbohydrate cluster, intercalator, minor groove binder, cleaving agent, and cross-linking agent conjugate molecules described in U.S. Patent Publication Nos. 20050119470 and 20050107325. Yet other examples include the 2'-O-alkyl amine, 2'-β-alkoxyalkyl amine, polyamine, C5-cationic modified pyrimidine, cationic peptide, guanidinium group, amidininium group, cationic amino acid conjugate molecules described in U.S. Patent Publication No. 20050153337. Additional examples include the hydrophobic group, membrane active compound, cell penetrating compound, cell targeting signal, interaction modifier, and steric stabilizer conjugate molecules described in U.S. Patent Publication No. 20040167090. Further examples include the conjugate molecules described in U.S. Patent Publication No. 20050239739. The type of conjugate used and the extent of conjugation to the siRNA molecule can be evaluated for improved pharmacokinetic profiles, bioavailability, and/or stability of the siRNA while retaining RNAi activity. As such, one skilled in the art can screen siRNA molecules having various conjugates attached thereto to identify ones having improved properties and full RNAi activity using any of a variety of well-known in vitro cell culture or in vivo animal models.

### Target Genes

In certain embodiments, the nucleic acid component (*e.g.,* siRNA) of the lipid nanoparticles described herein can be used to downregulate or silence the translation (i.e., expression) of a gene of interest. Genes of interest include, but are not limited to, genes associated with viral infection and survival, genes associated with metabolic diseases and disorders (*e.g.,* liver diseases and disorders), genes associated with tumorigenesis and cell transformation (*e.g.,* cancer), angiogenic genes, immunomodulator genes such as those associated with inflammatory and autoimmune responses, ligand receptor genes, and genes associated with neurodegenerative disorders. In certain embodiments, the gene of interest is expressed in hepatocytes.

Genes associated with viral infection and survival include those expressed by a virus in order to bind, enter, and replicate in a cell. Of particular interest are viral sequences associated with chronic viral diseases. Viral sequences of particular interest include sequences of Filoviruses such as Ebola virus and Marburg virus (see, *e.g.,* Geisbert et al., J. Infect. Dis., 193:1650-1657 (2006)); Arenaviruses such as Lassa virus, Junin virus, Machupo virus, Guanarito virus, and Sabia virus (Buchmeier et al., Arenaviridae: the viruses and their replication, In: FIELDS VIROLOGY, Knipe et al. (eds.), 4th ed., Lippincott-Raven, Philadelphia, (2001)); Influenza viruses such as Influenza A, B, and C viruses, (see, *e.g.,* Steinhauer et al., Annu Rev Genet., 36:305-332 (2002); and Neumann et al., J Gen Virol., 83:2635-2662 (2002)); Hepatitis viruses (see, *e.g.,* Hamasaki et al., FEBS Lett., 543:51 (2003); Yokota et al., EMBO Rep., 4:602 (2003); Schlomai et al., Hepatology, 37:764 (2003); Wilson et al., Proc. Natl. Acad. Sci. USA, 100:2783 (2003); Kapadia et al., Proc. Natl. Acad. Sci. USA, 100:2014 (2003); and FIELDS VIROLOGY, Knipe et al. (eds.), 4th ed., Lippincott-Raven, Philadelphia (2001)); Human Immunodeficiency Virus (HIV) (Banerjea et al., Mol. Ther., 8:62 (2003); Song et al., J. Virol., 77:7174 (2003); Stephenson, JAMA, 289:1494 (2003); Qin et al., Proc. Natl. Acad. Sci. USA, 100:183 (2003)); Herpes viruses (Jia et al., J. Virol., 77:3301 (2003)); and Human Papilloma Viruses (HPV) (Hall et al., J. Virol., 77:6066 (2003); Jiang et al., Oncogene, 21:6041 (2002)).

Exemplary Filovirus nucleic acid sequences that can be silenced include, but are not limited to, nucleic acid sequences encoding structural proteins (*e.g.,* VP30, VP35, nucleoprotein (NP), polymerase protein (L-pol)) and membrane-associated proteins (*e.g.,* VP40, glycoprotein (GP), VP24). Complete genome sequences for Ebola virus are set forth in, *e.g.,* Genbank Accession Nos. NC_002549; AY769362; NC_006432; NC_004161; AY729654; AY354458; AY142960; AB050936; AF522874; AF499101; AF272001; and AF086833. Ebola virus VP24 sequences are set forth in, *e.g.,* Genbank Accession Nos. U77385 and AY058897. Ebola virus L-pol sequences are set forth in, *e.g.,* Genbank Accession No. X67110. Ebola virus VP40 sequences are set forth in, *e.g.,* Genbank Accession No. AY058896. Ebola virus NP sequences are set forth in, *e.g.,* Genbank Accession No. AY058895. Ebola virus GP sequences are set forth in, *e.g.,* Genbank Accession No. AY058898; Sanchez et al., Virus Res., 29:215-240 (1993); Will et al., J. Virol., 67:1203-1210 (1993); Volchkov et al., FEBS Lett., 305:181-184 (1992); and U.S. Pat. No. 6,713,069. Additional Ebola virus sequences are set forth in, *e.g.,* Genbank Accession Nos. L11365 and X61274. Complete genome sequences for Marburg virus are set forth in, *e.g.,* Genbank Accession Nos. NC_001608; AY430365; AY430366; and AY358025. Marburg virus GP sequences are set forth in, *e.g.,* Genbank Accession Nos. AF005734; AF005733; and AF005732. Marburg virus VP35 sequences are set forth in, *e.g.,* Genbank Accession Nos. AF005731 and AF005730. Additional Marburg virus sequences are set forth in, *e.g.,* Genbank Accession Nos. X64406; Z29337; AF005735; and Z12132. Non-limiting examples of siRNA molecules targeting Ebola virus and Marburg virus nucleic acid sequences include those described in U.S. Patent Publication No. 20070135370.

Exemplary Influenza virus nucleic acid sequences that can be silenced include, but are not limited to, nucleic acid sequences encoding nucleoprotein (NP), matrix proteins (M1 and M2), nonstructural proteins (NS1 and NS2), RNA polymerase (PA, PB1, PB2), neuraminidase (NA), and haemagglutinin (HA). Influenza A NP sequences are set forth in, *e.g.,* Genbank Accession Nos. NC_004522; AY818138; AB166863; AB188817; AB189046; AB189054; AB189062; AY646169; AY646177; AY651486; AY651493; AY651494; AY651495; AY651496; AY651497; AY651498; AY651499; AY651500; AY651501; AY651502; AY651503; AY651504; AY651505; AY651506; AY651507; AY651509; AY651528; AY770996; AY790308; AY818138; and AY818140. Influenza A PA sequences are set forth in, e.g., Genbank Accession Nos. AY818132; AY790280; AY646171; AY818132; AY818133; AY646179; AY818134; AY551934; AY651613; AY651610; AY651620; AY651617; AY651600; AY651611; AY651606; AY651618; AY651608; AY651607; AY651605; AY651609; AY651615; AY651616; AY651640; AY651614; AY651612; AY651621; AY651619; AY770995; and AY724786. Non-limiting examples of siRNA molecules targeting Influenza virus nucleic acid sequences include those described in U.S. Patent Publication No. 20070218122.

Exemplary hepatitis virus nucleic acid sequences that can be silenced include, but are not limited to, nucleic acid sequences involved in transcription and translation (*e.g.,* En1, En2, X, P) and nucleic acid sequences encoding structural proteins (*e.g.,* core proteins including C and C-related proteins, capsid and envelope proteins including S, M, and/or L proteins, or fragments thereof) (see, *e.g.,* FIELDS VIROLOGY, supra). Exemplary Hepatitis C virus (HCV) nucleic acid sequences that can be silenced include, but are not limited to, the 5'-untranslated region (5'-UTR), the 3'-untranslated region (3'-UTR), the polyprotein translation initiation codon region, the internal ribosome entry site (IRES) sequence, and/or nucleic acid sequences encoding the core protein, the E1 protein, the E2 protein, the p7 protein, the NS2 protein, the NS3 protease/helicase, the NS4A protein, the NS4B protein, the NS5A protein, and/or the NS5B RNA-dependent RNA polymerase. HCV genome sequences are set forth in, *e.g.,* Genbank Accession Nos. NC_004102 (HCV genotype 1a), AJ238799 (HCV genotype 1b), NC_009823 (HCV genotype 2), NC_009824 (HCV genotype 3), NC_009825 (HCV genotype 4), NC_ 009826 (HCV genotype 5), and NC_009827 (HCV genotype 6). Hepatitis A virus nucleic acid sequences are set forth in, *e.g.,* Genbank Accession No. NC_001489; Hepatitis B virus nucleic acid sequences are set forth in, *e.g.,* Genbank Accession No. NC_003977; Hepatitis D virus nucleic acid sequence are set forth in, *e.g.,* Genbank Accession No. NC_001653; Hepatitis E virus nucleic acid sequences are set forth in, *e.g.,* Genbank Accession No. NC_001434; and Hepatitis G virus nucleic acid sequences are set forth in, *e.g.,* Genbank Accession No. NC_ 001710. Silencing of sequences that encode genes associated with viral infection and survival can conveniently be used in combination with the administration of conventional agents used to treat the viral condition. Non-limiting examples of siRNA molecules targeting hepatitis virus nucleic acid sequences include those described in U.S. Patent Publication Nos. 20060281175, 20050058982, and 20070149470; U.S. Pat. No. 7,348,314; and U.S. Provisional Application No. 61/162,127, filed Mar. 20, 2009.

Genes associated with metabolic diseases and disorders (*e.g.,* disorders in which the liver is the target and liver diseases and disorders) include, for example, genes expressed in dyslipidemia (*e.g*., liver X receptors such as LXRα and LXRβ (Genback Accession No. NM_ 007121), farnesoid X receptors (FXR) (Genbank Accession No. NM_005123), sterol-regulatory element binding protein (SREBP), site-1 protease (SIP), 3-hydroxy-3-methylglutaryl coenzyme-A reductase (HMG coenzyme-A reductase), apolipoprotein B (ApoB) (Genbank Accession No. NM_000384), apolipoprotein CIII (ApoC3) (Genbank Accession Nos. NM_000040 and NG_ 008949 REGION: 5001.8164), and apolipoprotein E (ApoE) (Genbank Accession Nos. NM_ 000041 and NG_007084 REGION: 5001.8612)); and diabetes (*e.g.,* glucose 6-phosphatase) (see, *e.g.,* Forman et al., Cell, 81:687 (1995); Seol et al., Mol. Endocrinol., 9:72 (1995), Zavacki et al., Proc. Natl. Acad. Sci. USA, 94:7909 (1997); Sakai et al., Cell, 85:1037-1046 (1996); Duncan et al., J. Biol. Chem., 272:12778-12785 (1997); Willy et al., Genes Dev., 9:1033-1045 (1995); Lehmann et al., J. Biol. Chem., 272:3137-3140 (1997); Janowski et al., Nature, 383:728-731 (1996); and Peet et al., Cell, 93:693-704 (1998)). One of skill in the art will appreciate that genes associated with metabolic diseases and disorders (*e.g.,* diseases and disorders in which the liver is a target and liver diseases and disorders) include genes that are expressed in the liver itself as well as and genes expressed in other organs and tissues. Silencing of sequences that encode genes associated with metabolic diseases and disorders can conveniently be used in combination with the administration of conventional agents used to treat the disease or disorder. Non-limiting examples of siRNA molecules targeting the ApoB gene include those described in U.S. Patent Publication No. 20060134189. Non-limiting examples of siRNA molecules targeting the ApoC3 gene include those described in U.S. Provisional Application No. 61/147,235, filed Jan. 26, 2009.

Examples of gene sequences associated with tumorigenesis and cell transformation (*e.g.,* cancer or other neoplasia) include mitotic kinesins such as Eg5 (KSP, KIF11; Genbank Accession No. NM_004523); serine/threonine kinases such as polo-like kinase 1 (PLK-1) (Genbank Accession No. NM_005030; Barr et al., Nat. Rev. Mol. Cell. Biol., 5:429-440 (2004)); tyrosine kinases such as WEE1 (Genbank Accession Nos. NM_003390 and NM_ 001143976); inhibitors of apoptosis such as XIAP (Genbank Accession No. NM_001167); COP9 signalosome subunits such as CSN1, CSN2, CSN3, CSN4, CSN5 (JAB1; Genbank Accession No. NM_006837); CSN6, CSN7A, CSN7B, and CSN8; ubiquitin ligases such as COP1 (RFWD2; Genbank Accession Nos. NM_022457 and NM_001001740); and histone deacetylases such as HDAC1, HDAC2 (Genbank Accession No. NM_001527), HDAC3, HDAC4, HDAC5, HDAC6, HDAC7, HDAC8, HDAC9, etc. Non-limiting examples of siRNA molecules targeting the Eg5 and XIAP genes include those described in U.S. patent application Ser. No. 11/807,872, filed May 29, 2007. Non-limiting examples of siRNA molecules targeting the PLK-1 gene include those described in U.S. Patent Publication Nos. 20050107316 and 20070265438; and U.S. patent application Ser. No. 12/343,342, filed Dec. 23, 2008. Non-limiting examples of siRNA molecules targeting the CSN5 gene include those described in U.S. Provisional Application No. 61/045,251, filed Apr. 15, 2008.

Additional examples of gene sequences associated with tumorigenesis and cell transformation include translocation sequences such as MLL fusion genes, BCR-ABL (Wilda et al., Oncogene, 21:5716 (2002); Scherr et al., Blood, 101:1566 (2003)), TEL-AML1, EWS-FLI1, TLS-FUS, PAX3-FKHR, BCL-2, AML1-ETO, and AML1-MTG8 (Heidenreich et al., Blood, 101:3157 (2003)); overexpressed sequences such as multidrug resistance genes (Nieth et al., FEBS Lett., 545:144 (2003); Wu et al, Cancer Res. 63:1515 (2003)), cyclins (Li et al., Cancer Res., 63:3593 (2003); Zou et al., Genes Dev., 16:2923 (2002)), beta-catenin (Verma et al., Clin Cancer Res., 9:1291 (2003)), telomerase genes (Kosciolek et al., Mol Cancer Ther., 2:209 (2003)), c-MYC, N-MYC, BCL-2, growth factor receptors (*e.g*., EGFR/ErbB1 (Genbank Accession Nos. NM_005228, NM_201282, NM_201283, and NM_201284; see also, Nagy et al. Exp. Cell Res., 285:39-49 (2003), ErbB2/HER-2 (Genbank Accession Nos. NM_004448 and NM_001005862), ErbB3 (Genbank Accession Nos. NM_001982 and NM_001005915), and ErbB4 (Genbank Accession Nos. NM_005235 and NM_001042599); and mutated sequences such as RAS (reviewed in Tuschl and Borkhardt, Mol. Interventions, 2:158 (2002)). Non-limiting examples of siRNA molecules targeting the EGFR gene include those described in U.S. patent application Ser. No. 11/807,872, filed May 29, 2007.

Silencing of sequences that encode DNA repair enzymes find use in combination with the administration of chemotherapeutic agents (Collis et al., Cancer Res., 63:1550 (2003)). Genes encoding proteins associated with tumor migration are also target sequences of interest, for example, integrins, selectins, and metalloproteinases. The foregoing examples are not exclusive. Those of skill in the art will understand that any whole or partial gene sequence that facilitates or promotes tumorigenesis or cell transformation, tumor growth, or tumor migration can be included as a template sequence.

Angiogenic genes are able to promote the formation of new vessels. Of particular interest is vascular endothelial growth factor (VEGF) (Reich et al., Mol. Vis., 9:210 (2003)) or VEGFR. siRNA sequences that target VEGFR are set forth in, *e.g.,* GB 2396864; U.S. Patent Publication No. 20040142895; and CA 2456444.

Anti-angiogenic genes are able to inhibit neovascularization. These genes are particularly useful for treating those cancers in which angiogenesis plays a role in the pathological development of the disease. Examples of anti-angiogenic genes include, but are not limited to, endostatin (see, *e.g.,* U.S. Pat. No. 6,174,861), angiostatin (see, *e.g.,* U U.S. Pat. No. 5,639,725), and VEGFR2 (see, *e.g.,* Decaussin et al., J. Pathol., 188: 369-377 (1999)). Immunomodulator genes are genes that modulate one or more immune responses. Examples of immunomodulator genes include, without limitation, cytokines such as growth factors (*e.g.,* TGF-α, TGF-β, EGF, FGF, IGF, NGF, PDGF, CGF, GM-CSF, SCF, etc.), interleukins (*e.g*., IL-2, IL-4, IL-12 (Hill et al., J. Immunol., 171:691 (2003)), IL-15, IL-18, IL-20, etc.), interferons (*e.g.,* IFN-α, IFN-β, IFN-γ, etc.) and TNF. Fas and Fas ligand genes are also immunomodulator target sequences of interest (Song et al., Nat. Med., 9:347 (2003)). Genes encoding secondary signaling molecules in hematopoietic and lymphoid cells are also included in the present invention, for example, Tec family kinases such as Bruton's tyrosine kinase (Btk) (Heinonen et al., FEES Lett., 527:274 (2002)).

Cell receptor ligands include ligands that are able to bind to cell surface receptors (*e.g.,* insulin receptor, EPO receptor, G-protein coupled receptors, receptors with tyrosine kinase activity, cytokine receptors, growth factor receptors, etc.), to modulate (*e.g.,* inhibit, activate, etc.) the physiological pathway that the receptor is involved in (*e.g.,* glucose level modulation, blood cell development, mitogenesis, etc.). Examples of cell receptor ligands include, but are not limited to, cytokines, growth factors, interleukins, interferons, erythropoietin (EPO), insulin, glucagon, G-protein coupled receptor ligands, etc. Templates coding for an expansion of trinucleotide repeats (*e.g.,* CAG repeats) find use in silencing pathogenic sequences in neurodegenerative disorders caused by the expansion of trinucleotide repeats, such as spinobulbular muscular atrophy and Huntington's Disease (Caplen et al., Hum. Mol. Genet., 11:175 (2002)).

Certain other target genes, which may be targeted by a nucleic acid (*e.g.,* by siRNA) to downregulate or silence the expression of the gene, include but are not limited to, Actin, Alpha 2, Smooth Muscle, Aorta (ACTA2), Alcohol dehydrogenase 1A (ADH1A), Alcohol dehydrogenase 4 (ADH4), Alcohol dehydrogenase 6 (ADH6), Afamin (AFM), Angiotensinogen (AGT), Serine-pyruvate aminotransferase (AGXT), Alpha-2-HS-glycoprotein (AHSG), Aldo-keto reductase family 1 member C4 (AKR1C4), Serum albumin (ALB), alpha-1-microglobulin/bikunin precursor (AMBP), Angiopoietin-related protein 3 (ANGPTL3), Serum amyloid P-component (APCS), Apolipoprotein A-II (APOA2), Apolipoprotein B-100 (APOB), Apolipoprotein C3 (APOC3), Apolipoprotein C-IV (APOC4), Apolipoprotein F (APOF), Beta-2-glycoprotein 1 (APOH), Aquaporin-9 (AQP9), Bile acid-CoA:amino acid N-acyltransferase (BAAT), C4b-binding protein beta chain (C4BPB), Putative uncharacterized protein encoded by LINC01554 (C5orf27), Complement factor 3 (C3), Complement Factor 5 (C5), Complement component C6 (C6), Complement component C8 alpha chain (C8A), Complement component C8 beta chain (C8B), Complement component C8 gamma chain (C8G), Complement component C9 (C9), Calmodulin Binding Transcription Activator 1 (CAMTA1), CD38 (CD38), Complement Factor B (CFB), Complement factor H-related protein 1 (CFHR1), Complement factor H-related protein 2 (CFHR2), Complement factor H-related protein 3 (CFHR3), Cannabinoid receptor 1 (CNR1), ceruloplasmin (CP), carboxypeptidase B2 (CPB2), Connective tissue growth factor (CTGF), C-X-C motif chemokine 2 (CXCL2), Cytochrome P450 1A2 (CYP1A2), Cytochrome P450 2A6 (CYP2A6), Cytochrome P450 2C8 (CYP2C8), Cytochrome P450 2C9 (CYP2C9), Cytochrome P450 Family 2 Subfamily D Member 6 (CYP2D6), Cytochrome P450 2E1 (CYP2E1), Phylloquinone omega-hydroxylase CYP4F2 (CYP4F2), 7-alpha-hydroxycholest-4-en-3-one 12-alpha-hydroxylase (CYP8B1), Dipeptidyl peptidase 4 (DPP4), coagulation factor 12 (F12), coagulation factor II (thrombin) (F2), coagulation factor IX (F9), fibrinogen alpha chain (FGA), fibrinogen beta chain (FGB), fibrinogen gamma chain (FGG), fibrinogen-like 1 (FGL1), flavin containing monooxygenase 3 (FMO3), flavin containing monooxygenase 5 (FMO5), group-specific component (vitamin D binding protein) (GC), Growth hormone receptor (GHR), glycine N-methyltransferase (GNMT), hyaluronan binding protein 2 (HABP2), hepcidin antimicrobial peptide (HAMP), hydroxyacid oxidase (glycolate oxidase) 1 (HAO1), HGF activator (HGFAC), haptoglobin-related protein; haptoglobin (HPR), hemopexin (HPX), histidine-rich glycoprotein (HRG), hydroxysteroid (11-beta) dehydrogenase 1 (HSD11B1), hydroxysteroid (17-beta) dehydrogenase 13 (HSD17B13), Inter-alpha-trypsin inhibitor heavy chain H1 (ITIH1), Inter-alpha-trypsin inhibitor heavy chain H2 (ITIH2), Inter-alpha-trypsin inhibitor heavy chain H3 (ITIH3), Inter-alpha-trypsin inhibitor heavy chain H4 (ITIH4), Prekallikrein (KLKB1), Lactate dehydrogenase A (LDHA), liver expressed antimicrobial peptide 2 (LEAP2), leukocyte cell-derived chemotaxin 2 (LECT2), Lipoprotein (a) (LPA), mannan-binding lectin serine peptidase 2 (MASP2), S-adenosylmethionine synthase isoform type-1 (MAT1A), NADPH Oxidase 4 (NOX4), Poly [ADP-ribose] polymerase 1 (PARP1), paraoxonase 1 (PON1), paraoxonase 3 (PON3), Vitamin K-dependent protein C (PROC), Retinol dehydrogenase 16 (RDH16), serum amyloid A4, constitutive (SAA4), serine dehydratase (SDS), Serpin Family A Member 1 (SERPINA1), Serpin A11 (SERPINA11), Kallistatin (SERPINA4), Corticosteroid-binding globulin (SERPINA6), Antithrombin-III (SERPINC1), Heparin cofactor 2 (SERPIND1), Serpin Family H Member 1 (SERPINH1), Solute Carrier Family 5 Member 2 (SLC5A2), Sodium/bile acid cotransporter (SLC10A1), Solute carrier family 13 member 5 (SLC13A5), Solute carrier family 22 member 1 (SLC22A1), Solute carrier family 25 member 47 (SLC25A47), Solute carrier family 2, facilitated glucose transporter member 2 (SLC2A2), Sodium-coupled neutral amino acid transporter 4 (SLC38A4), Solute carrier organic anion transporter family member 1B1 (SLCO1B1), Sphingomyelin Phosphodiesterase 1 (SMPD1), Bile salt sulfotransferase (SULT2A1), tyrosine aminotransferase (TAT), tryptophan 2,3-dioxygenase (TDO2), UDP glucuronosyltransferase 2 family, polypeptide B10 (UGT2B10), UDP glucuronosyltransferase 2 family, polypeptide B15 (UGT2B15), UDP glucuronosyltransferase 2 family, polypeptide B4 (UGT2B4) and vitronectin (VTN).

In addition to its utility in silencing the expression of any of the above-described genes for therapeutic purposes, certain nucleic acids (*e.g.,* siRNA) described herein are also useful in research and development applications as well as diagnostic, prophylactic, prognostic, clinical, and other healthcare applications. As a non-limiting example, certain nucleic acids (*e.g.,* siRNA) can be used in target validation studies directed at testing whether a gene of interest has the potential to be a therapeutic target. Certain nucleic acids (*e.g.,* siRNA) can also be used in target identification studies aimed at discovering genes as potential therapeutic targets.

### CRISPR

Targeted genome editing has progressed from being a niche technology to a method used by many biological researchers. This progression has been largely fueled by the emergence of the clustered, regularly interspaced, short palindromic repeat (CRISPR) technology (*see, e.g.,* Sander et al., Nature Biotechnology, 32(4), 347-355, including Supplementary Information (2014) and International Publication Numbers WO 2016/197132 and WO 2016/197133). Accordingly, provided herein are improvements (*e.g.,* lipid nanoparticles and formulations thereof) that can be used in combination with CRISPR technology to treat diseases, such as HBV. Regarding the targets for using CRISPR, the guide RNA (gRNA) utilized in the CRISPR technology can be designed to target specifically identified sequences, *e.g.,* target genes, *e.g.,* of the HBV genome. Examples of such target sequences are provided in International Publication Number WO 2016/197132. Further, International Publication Number WO 2013/151665 (*e.g., see* Table 6) describes about 35,000 mRNA sequences, claimed in the context of an mRNA expression construct. Certain embodiments of the present invention utilize CRISPR technology to target the expression of any of these sequences. Certain embodiments of the present invention may also utilize CRISPR technology to target the expression of a target gene discussed herein.

### aiRNA

Like siRNA, asymmetrical interfering RNA (aiRNA) can recruit the RNA-induced silencing complex (RISC) and lead to effective silencing of a variety of genes in mammalian cells by mediating sequence-specific cleavage of the target sequence between nucleotide 10 and 11 relative to the 5' end of the antisense strand (Sun et al., Nat. Biotech., 26:1379-1382 (2008)). Typically, an aiRNA molecule comprises a short RNA duplex having a sense strand and an antisense strand, wherein the duplex contains overhangs at the 3' and 5' ends of the antisense strand. The aiRNA is generally asymmetric because the sense strand is shorter on both ends when compared to the complementary antisense strand. In some aspects, aiRNA molecules may be designed, synthesized, and annealed under conditions similar to those used for siRNA molecules. As a non-limiting example, aiRNA sequences may be selected and generated using the methods described above for selecting siRNA sequences.

In another embodiment, aiRNA duplexes of various lengths (*e.g.,* about 10-25, 12-20, 12-19, 12-18, 13-17, or 14-17 base pairs, more typically 12, 13, 14, 15, 16, 17, 18, 19, or base pairs) may be designed with overhangs at the 3' and 5' ends of the antisense strand to target an mRNA of interest. In certain instances, the sense strand of the aiRNA molecule is about 10-25, 12-20, 12-19, 12-18, 13-17, or 14-17 nucleotides in length, more typically 12, 13, 14, 15, 16, 17, 18, 19, or 20 nucleotides in length. In certain other instances, the antisense strand of the aiRNA molecule is about 15-60, 15-50, or 15-40 nucleotides in length, more typically about 15-30, 15-25, or 19-25 nucleotides in length, and is preferably about 20-24, 21-22, or 21-23 nucleotides in length.

In some embodiments, the 5' antisense overhang contains one, two, three, four, or more nontargeting nucleotides (*e.g.,* "AA", "UU", "dTdT", etc.). In other embodiments, the 3' antisense overhang contains one, two, three, four, or more nontargeting nucleotides (*e.g.,* "AA", "UU", "dTdT", etc.). In certain aspects, the aiRNA molecules described herein may comprise one or more modified nucleotides, *e.g*., in the double-stranded (duplex) region and/or in the antisense overhangs. As a non-limiting example, aiRNA sequences may comprise one or more of the modified nucleotides described above for siRNA sequences. In a preferred embodiment, the aiRNA molecule comprises 2'OMe nucleotides such as, for example, 2'OMe-guanosine nucleotides, 2'OMe-uridine nucleotides, or mixtures thereof.

In certain embodiments, aiRNA molecules may comprise an antisense strand which corresponds to the antisense strand of an siRNA molecule, *e.g.,* one of the siRNA molecules described herein. In other embodiments, aiRNA molecules may be used to silence the expression of any of the target genes set forth above, such as, *e.g.,* genes associated with viral infection and survival, genes associated with metabolic diseases and disorders, genes associated with tumorigenesis and cell transformation, angiogenic genes, immunomodulator genes such as those associated with inflammatory and autoimmune responses, ligand receptor genes, and genes associated with neurodegenerative disorders.

### miRNA

Generally, microRNAs (miRNA) are single-stranded RNA molecules of about 21-23 nucleotides in length which regulate gene expression. miRNAs are encoded by genes from whose DNA they are transcribed, but miRNAs are not translated into protein (non-coding RNA); instead, each primary transcript (a pri-miRNA) is processed into a short stem-loop structure called a pre-miRNA and finally into a functional mature miRNA. Mature miRNA molecules are either partially or completely complementary to one or more messenger RNA (mRNA) molecules, and their main function is to downregulate gene expression. The identification of miRNA molecules is described, *e.g.,* in Lagos-Quintana et al., Science, 294:853-858; Lau et al., Science, 294:858-862; and Lee et al., Science, 294:862-864.

The genes encoding miRNA are much longer than the processed mature miRNA molecule. miRNA are first transcribed as primary transcripts or pri-miRNA with a cap and poly-A tail and processed to short, ~70-nucleotide stem-loop structures known as pre-miRNA in the cell nucleus. This processing is performed in animals by a protein complex known as the Microprocessor complex, consisting of the nuclease Drosha and the double-stranded RNA binding protein Pasha (Denli et al., Nature, 432:231-235 (2004)). These pre-miRNA are then processed to mature miRNA in the cytoplasm by interaction with the endonuclease Dicer, which also initiates the formation of the RNA-induced silencing complex (RISC) (Bernstein et al., Nature, 409:363-366 (2001). Either the sense strand or antisense strand of DNA can function as templates to give rise to miRNA.

When Dicer cleaves the pre-miRNA stem-loop, two complementary short RNA molecules are formed, but only one is integrated into the RISC complex. This strand is known as the guide strand and is selected by the argonaute protein, the catalytically active RNase in the RISC complex, on the basis of the stability of the 5' end (Preall et al., Curr. Biol., 16:530-535 (2006)). The remaining strand, known as the anti-guide or passenger strand, is degraded as a RISC complex substrate (Gregory et al., Cell, 123:631-640 (2005)). After integration into the active RISC complex, miRNAs base pair with their complementary mRNA molecules and induce target mRNA degradation and/or translational silencing.

Mammalian miRNA molecules are usually complementary to a site in the 3' UTR of the target mRNA sequence. In certain instances, the annealing of the miRNA to the target mRNA inhibits protein translation by blocking the protein translation machinery. In certain other instances, the annealing of the miRNA to the target mRNA facilitates the cleavage and degradation of the target mRNA through a process similar to RNA interference (RNAi). miRNA may also target methylation of genomic sites which correspond to targeted mRNA. Generally, miRNA function in association with a complement of proteins collectively termed the miRNP.

In certain aspects, the miRNA molecules described herein are about 15-100, 15-90, 15-80, 15-75, 15-70, 15-60, 15-50, or 15-40 nucleotides in length, more typically about 15-30, 15-25, or 19-25 nucleotides in length, and are preferably about 20-24, 21-22, or 21-23 nucleotides in length. In certain other aspects, miRNA molecules may comprise one or more modified nucleotides. As a non-limiting example, miRNA sequences may comprise one or more of the modified nucleotides described above for siRNA sequences. In a preferred embodiment, the miRNA molecule comprises 2'OMe nucleotides such as, for example, 2'OMe-guanosine nucleotides, 2'OMe-uridine nucleotides, or mixtures thereof.

In some embodiments, miRNA molecules may be used to silence the expression of any of the target genes set forth above, such as, *e.g.,* genes associated with viral infection and survival, genes associated with metabolic diseases and disorders, genes associated with tumorigenesis and cell transformation, angiogenic genes, immunomodulator genes such as those associated with inflammatory and autoimmune responses, ligand receptor genes, and genes associated with neurodegenerative disorders.

In other embodiments, one or more agents that block the activity of a miRNA targeting an mRNA of interest are administered using a lipid particle of the invention (*e.g.,* a lipid nanoparticle). Examples of blocking agents include, but are not limited to, steric blocking oligonucleotides, locked nucleic acid oligonucleotides, and Morpholino oligonucleotides. Such blocking agents may bind directly to the miRNA or to the miRNA binding site on the target mRNA.

### Antisense Oligonucleotides

In one embodiment, the nucleic acid is an antisense oligonucleotide directed to a target gene or sequence of interest. The terms "antisense oligonucleotide" or "antisense" include oligonucleotides that are complementary to a targeted polynucleotide sequence. Antisense oligonucleotides are single strands of DNA or RNA that are complementary to a chosen sequence. Antisense RNA oligonucleotides prevent the translation of complementary RNA strands by binding to the RNA. Antisense DNA oligonucleotides can be used to target a specific, complementary (coding or non-coding) RNA. If binding occurs, this DNA/RNA hybrid can be degraded by the enzyme RNase H. In a particular embodiment, antisense oligonucleotides comprise from about 10 to about 60 nucleotides, more preferably from about 15 to about 30 nucleotides. The term also encompasses antisense oligonucleotides that may not be exactly complementary to the desired target gene. Thus, the invention can be utilized in instances where non-target specific-activities are found with antisense, or where an antisense sequence containing one or more mismatches with the target sequence is the most preferred for a particular use.

Antisense oligonucleotides have been demonstrated to be effective and targeted inhibitors of protein synthesis, and, consequently, can be used to specifically inhibit protein synthesis by a targeted gene. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (see, U.S. Pat. Nos. 5,739,119 and 5,759,829). Furthermore, examples of antisense inhibition have been demonstrated with the nuclear protein cyclin, the multiple drug resistance gene (MDR1), ICAM-1, E-selectin, STK-1, striatal GABAA receptor, and human EGF (see, Jaskulski et al., Science, 240:1544-6 (1988); Vasanthakumar et al., Cancer Commun., 1:225-32 (1989); Penis et al., Brain Res Mol Brain Res., 15; 57:310-20 (1998); and U.S. Pat. Nos. 5,801,154; 5,789,573; 5,718,709 and 5,610,288). Moreover, antisense constructs have also been described that inhibit and can be used to treat a variety of abnormal cellular proliferations, *e.g.*, cancer (see, U.S. Pat. Nos. 5,747,470; 5,591,317; and 5,783,683).

Methods of producing antisense oligonucleotides are known in the art and can be readily adapted to produce an antisense oligonucleotide that targets any polynucleotide sequence. Selection of antisense oligonucleotide sequences specific for a given target sequence is based upon analysis of the chosen target sequence and determination of secondary structure, Tₘ, binding energy, and relative stability. Antisense oligonucleotides may be selected based upon their relative inability to form dimers, hairpins, or other secondary structures that would reduce or prohibit specific binding to the target mRNA in a host cell. Highly preferred target regions of the mRNA include those regions at or near the AUG translation initiation codon and those sequences that are substantially complementary to 5' regions of the mRNA. These secondary structure analyses and target site selection considerations can be performed, for example, using v.4 of the OLIGO primer analysis software (Molecular Biology Insights) and/or the BLASTN 2.0.5 algorithm software (Altschul et al., Nucleic Acids Res., 25:3389-402 (1997)).

### Ribozymes

According to another embodiment of the invention, lipid nanoparticles are associated with ribozymes. Ribozymes are RNA-protein complexes having specific catalytic domains that possess endonuclease activity (see, Kim et al., Proc. Natl. Acad. Sci. USA., 84:8788-92 (1987); and Forster et al., Cell, 49:211-20 (1987)). For example, a large number of ribozymes accelerate phosphoester transfer reactions with a high degree of specificity, often cleaving only one of several phosphoesters in an oligonucleotide substrate (see, Cech et al., Cell, 27:487-96 (1981); Michel et al., J. Mol. Biol., 216:585-610 (1990); Reinhold-Hurek et al., Nature, 357:173-6 (1992)). This specificity has been attributed to the requirement that the substrate bind via specific base-pairing interactions to the internal guide sequence ("IGS") of the ribozyme prior to chemical reaction.

At least six basic varieties of naturally-occurring enzymatic RNA molecules are known presently. Each can catalyze the hydrolysis of RNA phosphodiester bonds in trans (and thus can cleave other RNA molecules) under physiological conditions. In general, enzymatic nucleic acids act by first binding to a target RNA. Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nucleic acid molecule may be formed in a hammerhead, hairpin, hepatitis δ virus, group I intron or RNaseP RNA (in association with an RNA guide sequence), or *Neurospora* VS RNA motif, for example. Specific examples of hammerhead motifs are described in, *e.g.,* Rossi et al., Nucleic Acids Res., 20:4559-65 (1992). Examples of hairpin motifs are described in, *e.g.,* EP 0360257, Hampel et al., Biochemistry, 28:4929-33 (1989); Hampel et al., Nucleic Acids Res., 18:299-304 (1990); and U.S. Pat. No. 5,631,359. An example of the hepatitis δ virus motif is described in, *e.g.,* Perrotta et al., Biochemistry, 31:11843-52 (1992). An example of the RNaseP motif is described in, *e.g.,* Guerrier-Takada et al., Cell, 35:849-57 (1983). Examples of the *Neurospora* VS RNA ribozyme motif is described in, *e.g.,* Saville et al., Cell, 61:685-96 (1990); Saville et al., Proc. Natl. Acad. Sci. USA, 88:8826-30 (1991); Collins et al., Biochemistry, 32:2795-9 (1993). An example of the Group I intron is described in, *e.g.,* U.S. Pat. No. 4,987,071. Important characteristics of enzymatic nucleic acid molecules used according to the invention are that they have a specific substrate binding site which is complementary to one or more of the target gene DNA or RNA regions, and that they have nucleotide sequences within or surrounding that substrate binding site which impart an RNA cleaving activity to the molecule. Thus, the ribozyme constructs need not be limited to specific motifs mentioned herein.

Methods of producing a ribozyme targeted to any polynucleotide sequence are known in the art. Ribozymes may be designed as described in, *e.g.,* PCT Publication Nos. WO 93/23569 and WO 94/02595, and synthesized to be tested in vitro and/or in vivo as described therein.

Ribozyme activity can be optimized by altering the length of the ribozyme binding arms or chemically synthesizing ribozymes with modifications that prevent their degradation by serum ribonucleases (see, *e.g.,* PCT Publication Nos. WO 92/07065, WO 93/15187, WO 91/03162, and WO 94/13688; EP 92110298.4; and U.S. Pat. No. 5,334,711, which describe various chemical modifications that can be made to the sugar moieties of enzymatic RNA molecules), modifications which enhance their efficacy in cells, and removal of stem II bases to shorten RNA synthesis times and reduce chemical requirements.

### Immunostimulatory Oligonucleotides

Nucleic acids associated with lipid particles of the present invention may be immunostimulatory, including immunostimulatory oligonucleotides (ISS; single- or double-stranded) capable of inducing an immune response when administered to a subject, which may be a mammal such as a human. ISS include, *e.g*., certain palindromes leading to hairpin secondary structures (see, Yamamoto et al., J. Immunol., 148:4072-6 (1992)), or CpG motifs, as well as other known ISS features (such as multi-G domains; see; PCT Publication No. WO 96/11266).

Immunostimulatory nucleic acids are considered to be non-sequence specific when it is not required that they specifically bind to and reduce the expression of a target sequence in order to provoke an immune response. Thus, certain immunostimulatory nucleic acids may comprise a sequence corresponding to a region of a naturally-occurring gene or mRNA, but they may still be considered non-sequence specific immunostimulatory nucleic acids.

In one embodiment, the immunostimulatory nucleic acid or oligonucleotide comprises at least one CpG dinucleotide. The oligonucleotide or CpG dinucleotide may be unmethylated or methylated. In another embodiment, the immunostimulatory nucleic acid comprises at least one CpG dinucleotide having a methylated cytosine. In one embodiment, the nucleic acid comprises a single CpG dinucleotide, wherein the cytosine in the CpG dinucleotide is methylated. In an alternative embodiment, the nucleic acid comprises at least two CpG dinucleotides, wherein at least one cytosine in the CpG dinucleotides is methylated. In a further embodiment, each cytosine in the CpG dinucleotides present in the sequence is methylated. In another embodiment, the nucleic acid comprises a plurality of CpG dinucleotides, wherein at least one of the CpG dinucleotides comprises a methylated cytosine. Examples of immunostimulatory oligonucleotides suitable for use in the compositions and methods of the present invention are described in PCT Application No. PCT/US08/88676, filed Dec. 31, 2008, PCT Publication Nos. WO 02/069369 and WO 01/15726, U.S. Pat. No. 6,406,705, and Raney et al., J. Pharm. Exper. Ther., 298:1185-92 (2001). In certain embodiments, the oligonucleotides used in the compositions and methods of the invention have a phosphodiester ("PO") backbone or a phosphorothioate ("PS") backbone, and/or at least one methylated cytosine residue in a CpG motif.

### mRNA

Certain embodiments of the invention provide compositions which are useful in methods that can be used to express one or more mRNA molecules in a living cell (*e.g.,* cells within a human body). The mRNA molecules encode one or more polypeptides to be expressed within the living cells. In some embodiments, the polypeptides are expressed within a diseased organism (*e.g.,* mammal, such as a human being), and expression of the polypeptide ameliorates one or more symptoms of the disease. The compositions of the invention are particularly useful for treating human diseases caused by the absence, or reduced levels, of a functional polypeptide within the human body. Accordingly, in certain embodiments, an LNP may comprise one or more nucleic acid molecules, such as one or more mRNA molecules (*e.g,* a cocktail of mRNA molecules).

In some embodiments, the mRNA(s) are fully encapsulated in the nucleic acid-lipid particle (*e.g.,* LNP). With respect to formulations comprising an mRNA cocktail, the different types of mRNA species present in the cocktail (*e.g.,* mRNA having different sequences) may be co-encapsulated in the same particle, or each type of mRNA species present in the cocktail may be encapsulated in a separate particle. The mRNA cocktail may be formulated in the particles described herein using a mixture of two or more individual mRNAs (each having a unique sequence) at identical, similar, or different concentrations or molar ratios. In one embodiment, a cocktail of mRNAs (corresponding to a plurality of mRNAs with different sequences) is formulated using identical, similar, or different concentrations or molar ratios of each mRNA species, and the different types of mRNAs are co-encapsulated in the same particle. In another embodiment, each type of mRNA species present in the cocktail is encapsulated in different particles at identical, similar, or different mRNA concentrations or molar ratios, and the particles thus formed (each containing a different mRNA payload) are administered separately (*e.g.,* at different times in accordance with a therapeutic regimen), or are combined and administered together as a single unit dose (*e.g.,* with a pharmaceutically acceptable carrier). The particles described herein are serum-stable, are resistant to nuclease degradation, and are substantially non-toxic to mammals such as humans.

### Modifications to mRNA

mRNA used in the practice of the present invention can include one, two, or more than two nucleoside modifications. In some embodiments, the modified mRNA exhibits reduced degradation in a cell into which the mRNA is introduced, relative to a corresponding unmodified mRNA.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1 -carboxymethyl-pseudouridine, 5-propynyl-uridine, 1 -propynyl-pseudouridine, 5-taurinomethyluridine, 1-taurinomethyl-pseudouridine, 5-taurinomethyl-2-thio-uridine, l -taurinomethyl-4-thio-uridine, 5-methyluridine, 1 -methy 1-pseudouridine, 4-thio- 1 -methy 1-pseudouridine, 2-thio- 1 -methy 1-pseudouridine, 1 -methy 1- 1 -deaza-pseudouridine, 2-thio- 1 -methyl- 1 -deaza-pseudouridine, dihy drouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1 -methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio- 1 -methyl-pseudoisocytidine, 4-thio- 1 -methyl- 1 -deaza-pseudoisocytidine, 1 -methyl- 1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy- 1 -methyl-pseudoisocytidine.

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1 -methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In specific embodiments, a modified nucleoside is 5 '-0-(l -Thiophosphate)- Adenosine, 5 ' -0-( 1 -Thiophosphate)-Cytidine, 5 ' -0-( 1 -Thiophosphate)-Guanosine, 5 '-0-( 1 - Thiophosphate)-Uridine or 5'-0-(l -Thiophosphate)-Pseudouridine. The α-thio substituted phosphate moiety is provided to confer stability to RNA polymers through the unnatural phosphorothioate backbone linkages. Phosphorothioate RNA have increased nuclease resistance and subsequently a longer half-life in a cellular environment. Phosphorothioate linked nucleic acids are expected to also reduce the innate immune response through weaker binding/activation of cellular innate immune molecules.

In certain embodiments it is desirable to intracellularly degrade a modified nucleic acid introduced into the cell, for example if precise timing of protein production is desired. Thus, the invention provides a modified nucleic acid containing a degradation domain, which is capable of being acted on in a directed manner within a cell.

In other embodiments, modified nucleosides include inosine, 1 -methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1 -methylguanosine, N2-methylguanosine, N2,N2-dimethylguanosine, 8-oxo-guanosine, 7-methyl-8-oxo-guanosine, 1 -methyl-6-thio-guanosine, N2-methyl-6-thio-guanosine, and N2,N2-dimethyl-6-thio-guanosine.

### Optional Components of the Modified Nucleic Acids

In further embodiments, the modified nucleic acids may include other optional components, which can be beneficial in some embodiments. These optional components include, but are not limited to, untranslated regions, kozak sequences, intronic nucleotide sequences, internal ribosome entry site (IRES), caps and polyA tails. For example, a 5' untranslated region (UTR) and/or a 3 ' UTR may be provided, wherein either or both may independently contain one or more different nucleoside modifications. In such embodiments, nucleoside modifications may also be present in the translatable region. Also provided are nucleic acids containing a Kozak sequence.

Additionally, provided are nucleic acids containing one or more intronic nucleotide sequences capable of being excised from the nucleic acid.

### Untranslated Regions (UTRs)

Untranslated regions (UTRs) of a gene are transcribed but not translated. The 5'UTR starts at the transcription start site and continues to the start codon but does not include the start codon; whereas, the 3'UTR starts immediately following the stop codon and continues until the transcriptional termination signal. There is growing body of evidence about the regulatory roles played by the UTRs in terms of stability of the nucleic acid molecule and translation. The regulatory features of a UTR can be incorporated into the mRNA used in the present invention to increase the stability of the molecule. The specific features can also be incorporated to ensure controlled down-regulation of the transcript in case they are misdirected to undesired organs sites.

### 5 ' Capping

The 5' cap structure of an mRNA is involved in nuclear export, increasing mRNA stability and binds the mRNA Cap Binding Protein (CBP), which is responsible for mRNA stability in the cell and translation competency through the association of CBP with poly(A) binding protein to form the mature cyclic mRNA species. The cap further assists the removal of 5' proximal introns removal during mRNA splicing.

Endogenous mRNA molecules may be 5'-end capped generating a 5'-ppp-5'-triphosphate linkage between a terminal guanosine cap residue and the 5'-terminal transcribed sense nucleotide of the mRNA molecule. This 5'-guanylate cap may then be methylated to generate an N7-methyl-guanylate residue. The ribose sugars of the terminal and/or anteterminal transcribed nucleotides of the 5' end of the mRNA may optionally also be 2'-0-methylated. 5'-decapping through hydrolysis and cleavage of the guanylate cap structure may target a nucleic acid molecule, such as an mRNA molecule, for degradation.

### IRES Sequences

mRNA containing an internal ribosome entry site (IRES) are also useful in the practice of the present invention. An IRES may act as the sole ribosome binding site, or may serve as one of multiple ribosome binding sites of an mRNA. An mRNA containing more than one functional ribosome binding site may encode several peptides or polypeptides that are translated independently by the ribosomes ("multicistronic mRNA"). When mRNA are provided with an IRES, further optionally provided is a second translatable region. Examples of IRES sequences that can be used according to the invention include without limitation, those from picornaviruses (*e.g.* FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (S1V) or cricket paralysis viruses (CrPV).

### Poly-A tails

During RNA processing, a long chain of adenine nucleotides (poly-A tail) may be added to a polynucleotide such as an mRNA molecules in order to increase stability. Immediately after transcription, the 3' end of the transcript may be cleaved to free a 3' hydroxyl. Then poly-A polymerase adds a chain of adenine nucleotides to the RNA. The process, called polyadenylation, adds a poly-A tail that can be between 100 and 250 residues long.

Generally, the length of a poly-A tail is greater than 30 nucleotides in length. In another embodiment, the poly-A tail is greater than 35 nucleotides in length (*e.g.,* at least or greater than about 35, 40, 45, 50, 55, 60, 70, 80, 90, 100, 120, 140, 160, 180, 200, 250, 300, 350, 400, 450, 500, 600, 700, 800, 900, 1000, 1100, 1200, 1300, 1400, 1500, 1600, 1700, 1800, 1900, 2,000, 2,500, and 3,000 nucleotides).

In this context the poly-A tail may be 10, 20, 30, 40, 50, 60, 70, 80, 90, or 100% greater in length than the modified mRNA. The poly-A tail may also be designed as a fraction of modified nucleic acids to which it belongs. In this context, the poly-A tail may be 10, 20, 30, 40, 50, 60, 70, 80, or 90% or more of the total length of the modified mRNA or the total length of the modified mRNA minus the poly-A tail.

### Generating mRNA Molecules

Methods for isolating RNA, synthesizing RNA, hybridizing nucleic acids, making and screening cDNA libraries, and performing PCR are well known in the art (*see, e.g.,* Gubler and Hoffman, Gene, 25:263-269 (1983); Sambrook et al., Molecular Cloning, A Laboratory Manual (2nd ed. 1989)); as are PCR methods (*see,* U.S. Patent Nos. 4,683,195 and 4,683,202; PCR Protocols: A Guide to Methods and Applications (Innis et al., eds, 1990)). Expression libraries are also well known to those of skill in the art. Additional basic texts disclosing the general methods of use in this invention include Kriegler, Gene Transfer and Expression: A Laboratory Manual (1990); and Current Protocols in Molecular Biology (Ausubel et al., eds., 1994).

### Encoded Polypeptides

The mRNA component of a lipid nanoparticle described herein can be used to express a polypeptide of interest. Certain diseases in humans are caused by the absence or impairment of a functional protein in a cell type where the protein is normally present and active. The functional protein can be completely or partially absent due, *e.g.,* to transcriptional inactivity of the encoding gene or due to the presence of a mutation in the encoding gene that renders the protein completely or partially non-functional. Examples of human diseases that are caused by complete or partial inactivation of a protein include X-linked severe combined immunodeficiency (X-SCID) and adrenoleukodystrophy (X-ALD). X-SCID is caused by one or more mutations in the gene encoding the common gamma chain protein that is a component of the receptors for several interleukins that are involved in the development and maturation of B and T cells within the immune system. X-ALD is caused by one or more mutations in a peroxisomal membrane transporter protein gene called ABCD1. Individuals afflicted with X-ALD have very high levels of long chain fatty acids in tissues throughout the body, which causes a variety of symptoms that may lead to mental impairment or death.

Attempts have been made to use gene therapy to treat some diseases caused by the absence or impairment of a functional protein in a cell type where the protein is normally present and active. Gene therapy typically involves introduction of a vector that includes a gene encoding a functional form of the affected protein, into a diseased person, and expression of the functional protein to treat the disease. Thus far, gene therapy has met with limited success. Additionally, certain aspects of delivering mRNA using LNPs have been described, *e.g*., in International Publication Numbers WO 2018/006052 and WO 2015/011633.

As such, there is a continuing need for improvement for expressing a functional form of a protein within a human who suffers from a disease caused by the complete or partial absence of the functional protein, and there is a need for improved delivery of nucleic acids *(e.g.,* mRNA) via a methods and compositions, *e.g*., that can trigger less of an immune response to the therapy. Certain embodiments of the present invention are useful in this context. Thus, in certain embodiments, expression of the polypeptide ameliorates one or more symptoms of a disease or disorder. Certain compositions and methods of the invention may be useful for treating human diseases caused by the absence, or reduced levels, of a functional polypeptide within the human body. In other embodiments, certain compositions and methods of the invention may be useful for expressing a vaccine antigen, *e.g.,* for treating cancer. Certain embodiments provide the use of a lipid nanoparticle described herein as a vaccine, *e.g.,* for use in delivering a therapeutic mRNA to a subject in need thereof.

### Self-Amplifying RNA

In certain embodiments, the nucleic acid is one or more self-amplifying RNA molecules. Self-amplifying RNA (sa-RNA) may also be referred to as self-replicating RNA, replication-competent RNA, replicons or RepRNA. RepRNA, referred to as self-amplifying mRNA when derived from positive-strand viruses, is generated from a viral genome lacking at least one structural gene; it can translate and replicate (hence "self-amplifying") without generating infectious progeny virus. In certain embodiments, the RepRNA technology may be used to insert a gene cassette encoding a desired antigen of interest. For example, the alphaviral genome is divided into two open reading frames (ORFs): the first ORF encodes proteins for the RNA dependent RNA polymerase (replicase), and the second ORF encodes structural proteins. In sa-RNA vaccine constructs, the ORF encoding viral structural proteins may be replaced with any antigen of choice, while the viral replicase remains an integral part of the vaccine and drives intracellular amplification of the RNA after immunization.

### Preparation of Lipid Particles

In certain embodiments, the present invention provides for LNP produced via a continuous mixing method, *e.g.,* a process that includes providing an aqueous solution comprising a nucleic acid in a first reservoir, providing an organic lipid solution in a second reservoir, and mixing the aqueous solution with the organic lipid solution such that the organic lipid solution mixes with the aqueous solution so as to substantially instantaneously produce a liposome encapsulating the nucleic acid (e.g., interfering RNA or mRNA). This process and the apparatus for carrying this process are described in detail in U.S. Patent Publication No. 20040142025.

The action of continuously introducing lipid and buffer solutions into a mixing environment, such as in a mixing chamber, causes a continuous dilution of the lipid solution with the buffer solution, thereby producing a liposome substantially instantaneously upon mixing. As used herein, the phrase "continuously diluting a lipid solution with a buffer solution" (and variations) generally means that the lipid solution is diluted sufficiently rapidly in a hydration process with sufficient force to effectuate vesicle generation. By mixing the aqueous solution comprising a nucleic acid with the organic lipid solution, the organic lipid solution undergoes a continuous stepwise dilution in the presence of the buffer solution (i.e., aqueous solution) to produce a lipid nanoparticle.

The LNP formed using the continuous mixing method typically have a size of from about 40 nm to about 150 nm, from about 40 nm to about 80 nm, from about 40 nm to about 60 nm, from about 50 nm to about 60 nm, from about 50 nm to about 150 nm, from about 60 nm to about 130 nm, from about 70 nm to about 110 nm, or from about 70 nm to about 90 nm. The particles thus formed do not aggregate and are optionally sized to achieve a uniform particle size.

In another embodiment, the present invention provides for LNP produced via a direct dilution process that includes forming a liposome solution and immediately and directly introducing the liposome solution into a collection vessel containing a controlled amount of dilution buffer. In preferred aspects, the collection vessel includes one or more elements configured to stir the contents of the collection vessel to facilitate dilution. In one aspect, the amount of dilution buffer present in the collection vessel is substantially equal to the volume of liposome solution introduced thereto. As a non-limiting example, a liposome solution in 45% ethanol when introduced into the collection vessel containing an equal volume of dilution buffer will advantageously yield smaller particles.

In yet another embodiment, the present invention provides for LNP produced via a direct dilution process in which a third reservoir containing dilution buffer is fluidly coupled to a second mixing region. In this embodiment, the liposome solution formed in a first mixing region is immediately and directly mixed with dilution buffer in the second mixing region. In preferred aspects, the second mixing region includes a T-connector arranged so that the liposome solution and the dilution buffer flows meet as opposing 180° flows; however, connectors providing shallower angles can be used, *e.g.,* from about 27° to about 180°. A pump mechanism delivers a controllable flow of buffer to the second mixing region. In one aspect, the flow rate of dilution buffer provided to the second mixing region is controlled to be substantially equal to the flow rate of liposome solution introduced thereto from the first mixing region. This embodiment advantageously allows for more control of the flow of dilution buffer mixing with the liposome solution in the second mixing region, and therefore also the concentration of liposome solution in buffer throughout the second mixing process. Such control of the dilution buffer flow rate advantageously allows for small particle size formation at reduced concentrations.

These processes and the apparatuses for carrying out these direct dilution processes are described in detail in U.S. Patent Publication No. 20070042031.

The LNP formed using the direct dilution process typically have a size of from about 40 nm to about 150 nm, from about 40 nm to about 80 nm, from about 40 nm to about 60 nm, from about 50 nm to about 60 nm, from about 50 nm to about 150 nm, from about 60 nm to about 130 nm, from about 70 nm to about 110 nm, or from about 70 nm to about 90 nm. The particles thus formed do not aggregate and are optionally sized to achieve a uniform particle size.

If needed, the lipid particles of the invention (*e.g.,* LNP) can be sized by any of the methods available for sizing liposomes. The sizing may be conducted in order to achieve a desired size range and relatively narrow distribution of particle sizes.

Several techniques are available for sizing the particles to a desired size. One sizing method, used for liposomes and equally applicable to the present particles, is described in U.S. Pat. No. 4,737,323. Sonicating a particle suspension either by bath or probe sonication produces a progressive size reduction down to particles of less than about 50 nm in size. Homogenization is another method which relies on shearing energy to fragment larger particles into smaller ones. In a typical homogenization procedure, particles are recirculated through a standard emulsion homogenizer until selected particle sizes, typically between about 60 and about 80 nm, are observed. In both methods, the particle size distribution can be monitored by conventional laser-beam particle size discrimination, or QELS.

Extrusion of the particles through a small-pore polycarbonate membrane or an asymmetric ceramic membrane is also an effective method for reducing particle sizes to a relatively well-defined size distribution. Typically, the suspension is cycled through the membrane one or more times until the desired particle size distribution is achieved. The particles may be extruded through successively smaller-pore membranes, to achieve a gradual reduction in size.

In some embodiments, the nucleic acids in the LNP are precondensed as described in, *e.g.,* U.S. patent application Ser. No. 09/744,103.

In other embodiments, the methods will further comprise adding non-lipid polycations which are useful to effect the lipofection of cells using the present compositions. Examples of suitable non-lipid polycations include, hexadimethrine bromide (sold under the brandname POLYBRENE^{®}, from Aldrich Chemical Co., Milwaukee, Wis., USA) or other salts of hexadimethrine. Other suitable polycations include, for example, salts of poly-L-ornithine, poly-L-arginine, poly-L-lysine, poly-D-lysine, polyallylamine, and polyethyleneimine. Addition of these salts is preferably after the particles have been formed.

### Administration of Lipid Particles

Once formed, the lipid particles of the invention (*e.g.,* LNP) are useful for the introduction of nucleic acidsinto cells. Accordingly, the present invention is useful in methods for introducing a nucleic acid such as a nucleic acid (*e.g.,* interfering RNA or mRNA) into a cell. The methods are carried out *in vitro* or *in vivo* by first forming the particles as described above and then contacting the particles with the cells for a period of time sufficient for delivery of the nucleic acid to the cells to occur.

The lipid particles of the invention (*e.g.,* LNP) can be adsorbed to almost any cell type with which they are mixed or contacted. Once adsorbed, the particles can either be endocytosed by a portion of the cells, exchange lipids with cell membranes, or fuse with the cells. Transfer or incorporation of the nucleic acid (*e.g.,* nucleic acid) portion of the particle can take place via any one of these pathways. In particular, when fusion takes place, the particle membrane is integrated into the cell membrane and the contents of the particle combine with the intracellular fluid.

The lipid particles of the invention (*e.g.,* LNP) can be administered either alone or in a mixture with a pharmaceutically acceptable carrier (*e.g.,* physiological saline or phosphate buffer) selected in accordance with the route of administration and standard pharmaceutical practice. Generally, normal buffered saline (*e.g.,* 135-150 mM NaCl) will be employed as the pharmaceutically acceptable carrier. Other suitable carriers include, e.g., water, buffered water, 0.4% saline, 0.3% glycine, and the like, including glycoproteins for enhanced stability, such as albumin, lipoprotein, globulin, etc. Additional suitable carriers are described in, *e.g.,* REMINGTON'S PHARMACEUTICAL SCIENCES, Mack Publishing Company, Philadelphia, Pa., 17th ed. (1985). As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The phrase "pharmaceutically-acceptable" refers to molecular entities and compositions that do not produce an allergic or similar untoward reaction when administered to a human.

The pharmaceutically acceptable carrier is generally added following particle formation. Thus, after the particle is formed, the particle can be diluted into pharmaceutically acceptable carriers such as normal buffered saline.

The concentration of particles in the pharmaceutical formulations can vary widely, i.e., from less than about 0.05%, usually at or at least about 2 to 5%, to as much as about 10 to 90% by weight, and will be selected primarily by fluid volumes, viscosities, etc., in accordance with the particular mode of administration selected. For example, the concentration may be increased to lower the fluid load associated with treatment. This may be particularly desirable in patients having atherosclerosis-associated congestive heart failure or severe hypertension. Alternatively, particles composed of irritating lipids may be diluted to low concentrations to lessen inflammation at the site of administration.

The pharmaceutical compositions of the present invention may be sterilized by conventional, well-known sterilization techniques. Aqueous solutions can be packaged for use or filtered under aseptic conditions and lyophilized, the lyophilized preparation being combined with a sterile aqueous solution prior to administration. The compositions can contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, and calcium chloride. Additionally, the particle suspension may include lipid-protective agents which protect lipids against free-radical and lipid-peroxidative damages on storage. Lipophilic free-radical quenchers, such as alphatocopherol and water-soluble iron-specific chelators, such as ferrioxamine, are suitable.

### In Vivo Administration

Systemic delivery for in vivo therapy, *e.g*., delivery of a therapeutic nucleic acid to a distal target cell via body systems such as the circulation, has been achieved using nucleic acid-lipid particles such as those described in PCT Publication Nos. WO 05/007196, WO 05/121348, WO 05/120152, and WO 04/002453. The present invention also provides fully encapsulated lipid particles that protect the nucleic acid from nuclease degradation in serum, are nonimmunogenic, are small in size, and are suitable for repeat dosing.

For *in vivo* administration, administration can be in any manner known in the art, *e.g.,* by injection, oral administration, inhalation (*e.g*., intransal or intratracheal), transdermal application, or rectal administration. Administration can be accomplished via single or divided doses. The pharmaceutical compositions can be administered parenterally, i.e., intraarticularly, intravenously, intraperitoneally, subcutaneously, or intramuscularly. In some embodiments, the pharmaceutical compositions are administered intravenously or intraperitoneally by a bolus injection (see, *e.g.,* U.S. Pat. No. 5,286,634). Intracellular nucleic acid delivery has also been discussed in Straubringer et al., Methods Enzymol., 101:512 (1983); Mannino et al., Biotechniques, 6:682 (1988); Nicolau et al., Crit. Rev. Ther. Drug Carrier Syst., 6:239 (1989); and Behr, Acc. Chem. Res., 26:274 (1993). Still other methods of administering lipid-based therapeutics are described in, for example, U.S. Pat. Nos. 3,993,754; 4,145,410; 4,235,871; 4,224,179; 4,522,803; and 4,588,578. The lipid particles can be administered by direct injection at the site of disease or by injection at a site distal from the site of disease (see, *e.g.,* Culver, HUMAN GENE THERAPY, MaryAnn Liebert, Inc., Publishers, New York. pp. 70-71 (1994)).

The compositions of the present invention, either alone or in combination with other suitable components, can be made into aerosol formulations (i.e., they can be "nebulized") to be administered via inhalation (*e.g.,* intranasally or intratracheally) (see, Brigham et al., Am. J. Sci., 298:278 (1989)). Aerosol formulations can be placed into pressurized acceptable propellants, such as dichlorodifluoromethane, propane, nitrogen, and the like.

In certain embodiments, the pharmaceutical compositions may be delivered by intranasal sprays, inhalation, and/or other aerosol delivery vehicles. Methods for delivering nucleic acid compositions directly to the lungs via nasal aerosol sprays have been described, *e.g*., in U.S. Pat. Nos. 5,756,353 and 5,804,212. Likewise, the delivery of drugs using intranasal microparticle resins and lysophosphatidyl-glycerol compounds (U.S. Pat. No. 5,725,871) are also well-known in the pharmaceutical arts. Similarly, transmucosal drug delivery in the form of a polytetrafluoroetheylene support matrix is described in U.S. Pat. No. 5,780,045.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions are preferably administered, for example, by intravenous infusion, orally, topically, intraperitoneally, intravesically, or intrathecally.

Generally, when administered intravenously, the lipid particle formulations are formulated with a suitable pharmaceutical carrier. Many pharmaceutically acceptable carriers may be employed in the compositions and methods of the present invention. Suitable formulations for use in the present invention are found, for example, in REMINGTON'S PHARMACEUTICAL SCIENCES, Mack Publishing Company, Philadelphia, Pa., 17th ed. (1985). A variety of aqueous carriers may be used, for example, water, buffered water, 0.4% saline, 0.3% glycine, and the like, and may include glycoproteins for enhanced stability, such as albumin, lipoprotein, globulin, etc. Generally, normal buffered saline (135-150 mM NaCl) will be employed as the pharmaceutically acceptable carrier, but other suitable carriers will suffice. These compositions can be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc. These compositions can be sterilized using the techniques referred to above or, alternatively, they can be produced under sterile conditions. The resulting aqueous solutions may be packaged for use or filtered under aseptic conditions and lyophilized, the lyophilized preparation being combined with a sterile aqueous solution prior to administration.

In certain applications, the lipid particles disclosed herein may be delivered via oral administration to the individual. The particles may be incorporated with excipients and used in the form of ingestible tablets, buccal tablets, troches, capsules, pills, lozenges, elixirs, mouthwash, suspensions, oral sprays, syrups, wafers, and the like (see, *e.g.,* U.S. Pat. Nos. 5,641,515, 5,580,579, and 5,792,451). These oral dosage forms may also contain the following: binders, gelatin; excipients, lubricants, and/or flavoring agents. When the unit dosage form is a capsule, it may contain, in addition to the materials described above, a liquid carrier. Various other materials may be present as coatings or to otherwise modify the physical form of the dosage unit. Of course, any material used in preparing any unit dosage form should be pharmaceutically pure and substantially non-toxic in the amounts employed.

Typically, these oral formulations may contain at least about 0.1% of the lipid particles or more, although the percentage of the particles may, of course, be varied and may conveniently be between about 1% or 2% and about 60% or 70% or more of the weight or volume of the total formulation. Naturally, the amount of particles in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

Formulations suitable for oral administration can consist of: (a) liquid solutions, such as an effective amount of a packaged therapeutic agent such as nucleic acid (e.g., interfering RNA or mRNA) suspended in diluents such as water, saline, or PEG 400; (b) capsules, sachets, or tablets, each containing a predetermined amount of a therapeutic agent such as nucleic acid *(e.g.,* interfering RNA or mRNA), as liquids, solids, granules, or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise a therapeutic agent such as nucleic acid (e.g., interfering RNA or mRNA) in a flavor, *e.g.*, sucrose, as well as pastilles comprising the therapeutic agent in an inert base, such as gelatin and glycerin or sucrose and acacia emulsions, gels, and the like containing, in addition to the therapeutic agent, carriers known in the art.

In another example of their use, lipid particles can be incorporated into a broad range of topical dosage forms. For instance, a suspension containing nucleic acid-lipid particles such as LNP can be formulated and administered as gels, oils, emulsions, topical creams, pastes, ointments, lotions, foams, mousses, and the like.

When preparing pharmaceutical preparations of the lipid particles of the invention, it is preferable to use quantities of the particles which have been purified to reduce or eliminate empty particles or particles with therapeutic agents such as nucleic acid associated with the external surface.

The methods described herein may be practiced in a variety of hosts. Preferred hosts include mammalian species, such as primates (e.g., humans and chimpanzees as well as other nonhuman primates), canines, felines, equines, bovines, ovines, caprines, rodents (e.g., rats and mice), lagomorphs, and swine.

The amount of particles administered will depend upon the ratio of therapeutic agent *(e.g.,* nucleic acid) to lipid, the particular therapeutic agent *(e.g.,* nucleic acid) used, the disease or disorder being treated, the age, weight, and condition of the patient, and the judgment of the clinician, but will generally be between about 0.01 and about 50 mg per kilogram of body weight, preferably between about 0.1 and about 5 mg/kg of body weight, or about 10⁸-10¹⁰ particles per administration (e.g., injection).

### In Vitro Administration

For in vitro applications, the delivery of therapeutic agents such as nucleic acids (*e.g.,* interfering RNA or mRNA) can be to any cell grown in culture, whether of plant or animal origin, vertebrate or invertebrate, and of any tissue or type. In preferred embodiments, the cells are animal cells, more preferably mammalian cells, and most preferably human cells.

Contact between the cells and the lipid particles, when carried out in vitro, takes place in a biologically compatible medium. The concentration of particles varies widely depending on the particular application, but is generally between about 1 µmol and about 10 mmol. Treatment of the cells with the lipid particles is generally carried out at physiological temperatures (about 37° C.) for periods of time of from about 1 to 48 hours, preferably of from about 2 to 4 hours.

In one group of preferred embodiments, a lipid particle suspension is added to 60-80% confluent plated cells having a cell density of from about 10³ to about 10⁵ cells/ml, more preferably about 2×10⁴ cells/ml. The concentration of the suspension added to the cells is preferably of from about 0.01 to 0.2 µg/ml, more preferably about 0.1 µg/ml.

Using an Endosomal Release Parameter (ERP) assay, the delivery efficiency of the LNP or other lipid particle of the invention can be optimized. An ERP assay is described in detail in U.S. Patent Publication No. 20030077829. More particularly, the purpose of an ERP assay is to distinguish the effect of various cationic lipids and helper lipid components of LNP based on their relative effect on binding/uptake or fusion with/destabilization of the endosomal membrane. This assay allows one to determine quantitatively how each component of the LNP or other lipid particle affects delivery efficiency, thereby optimizing the LNP or other lipid particle. Usually, an ERP assay measures expression of a reporter protein (*e.g.,* luciferase, β-galactosidase, green fluorescent protein (GFP), etc.), and in some instances, a LNP formulation optimized for an expression plasmid will also be appropriate for encapsulating an interfering RNA or mRNA. In other instances, an ERP assay can be adapted to measure downregulation of transcription or translation of a target sequence in the presence or absence of an interfering RNA (*e.g.,* siRNA). In other instances, an ERP assay can be adapted to measure the expression of a target protein in the presence or absence of an mRNA. By comparing the ERPs for each of the various LNP or other lipid particles, one can readily determine the optimized system, *e.g*., the LNP or other lipid particle that has the greatest uptake in the cell.

### Cells for Delivery of Lipid Particles

The compositions of the present invention are used to treat a wide variety of cell types, *in vivo* and *in vitro.* Suitable cells include, *e.g.,* hematopoietic precursor (stem) cells, fibroblasts, keratinocytes, hepatocytes, endothelial cells, skeletal and smooth muscle cells, osteoblasts, neurons, quiescent lymphocytes, terminally differentiated cells, slow or noncycling primary cells, parenchymal cells, lymphoid cells, epithelial cells, bone cells, and the like. In one embodiment, an nucleic acid, such as one or more nucleic acid molecules (e.g, an interfering RNA (*e.g.,* siRNA) or mRNA) is delivered to cancer cells such as, *e.g.,* lung cancer cells, colon cancer cells, rectal cancer cells, anal cancer cells, bile duct cancer cells, small intestine cancer cells, stomach (gastric) cancer cells, esophageal cancer cells, gallbladder cancer cells, liver cancer cells, pancreatic cancer cells, appendix cancer cells, breast cancer cells, ovarian cancer cells, cervical cancer cells, prostate cancer cells, renal cancer cells, cancer cells of the central nervous system, glioblastoma tumor cells, skin cancer cells, lymphoma cells, choriocarcinoma tumor cells, head and neck cancer cells, osteogenic sarcoma tumor cells, and blood cancer cells.

In vivo delivery of lipid particles such as LNP encapsulating one or more nucleic acid molecules (*e.g.,* interfering RNA (*e.g.,* siRNA) or mRNA) is suited for targeting cells of any cell type. The methods and compositions can be employed with cells of a wide variety of vertebrates, including mammals, such as, *e.g*., canines, felines, equines, bovines, ovines, caprines, rodents (*e.g.,* mice, rats, and guinea pigs), lagomorphs, swine, and primates (*e.g.* monkeys, chimpanzees, and humans).

To the extent that tissue culture of cells may be required, it is well-known in the art. For example, Freshney, Culture of Animal Cells, a Manual of Basic Technique, 3rd Ed., Wiley-Liss, New York (1994), Kuchler et al., Biochemical Methods in Cell Culture and Virology, Dowden, Hutchinson and Ross, Inc. (1977), and the references cited therein provide a general guide to the culture of cells. Cultured cell systems often will be in the form of monolayers of cells, although cell suspensions are also used.

### Detection of Lipid Particles

In some embodiments, the lipid particles of the present invention (*e.g.,* LNP) are detectable in the subject at about 1, 2, 3, 4, 5, 6, 7, 8 or more hours. In other embodiments, the lipid particles of the present invention (e.g., LNP) are detectable in the subject at about 8, 12, 24, 48, 60, 72, or 96 hours, or about 6, 8, 10, 12, 14, 16, 18, 19, 22, 24, 25, or 28 days after administration of the particles. The presence of the particles can be detected in the cells, tissues, or other biological samples from the subject. The particles may be detected, *e.g.,* by direct detection of the particles, detection of a therapeutic nucleic acid, such as an interfering RNA (*e.g*., siRNA) sequence or mRNA sequence, detection of a target sequence of interest (i.e., by detecting changes in expression of the sequence of interest), or a combination thereof.

### Detection of Particles

Lipid particles of the invention such as LNP can be detected using any method known in the art. For example, a label can be coupled directly or indirectly to a component of the lipid particle using methods well-known in the art. A wide variety of labels can be used, with the choice of label depending on sensitivity required, ease of conjugation with the lipid particle component, stability requirements, and available instrumentation and disposal provisions. Suitable labels include, but are not limited to, spectral labels such as fluorescent dyes (*e.g.,* fluorescein and derivatives, such as fluorescein isothiocyanate (FITC) and Oregon Green^{™}; rhodamine and derivatives such Texas red, tetrarhodimine isothiocynate (TRITC), etc., digoxigenin, biotin, phycoerythrin, AMCA, CyDyes^{™}, and the like; radiolabels such as ³H, ¹²⁵I, ³⁵S, ¹⁴C, ³²P, ³³P, etc.; enzymes such as horse radish peroxidase, alkaline phosphatase, etc.; spectral colorimetric labels such as colloidal gold or colored glass or plastic beads such as polystyrene, polypropylene, latex, etc. The label can be detected using any means known in the art.

### Detection of Nucleic Acids

Nucleic acids (*e.g.,* interfering RNA or mRNA) are detected and quantified herein by any of a number of means well-known to those of skill in the art. The detection of nucleic acids may proceed by well-known methods such as Southern analysis, Northern analysis, gel electrophoresis, PCR, radiolabeling, scintillation counting, and affinity chromatography. Additional analytic biochemical methods such as spectrophotometry, radiography, electrophoresis, capillary electrophoresis, high performance liquid chromatography (HPLC), thin layer chromatography (TLC), and hyperdiffusion chromatography may also be employed.

The selection of a nucleic acid hybridization format is not critical. A variety of nucleic acid hybridization formats are known to those skilled in the art. For example, common formats include sandwich assays and competition or displacement assays. Hybridization techniques are generally described in, *e.g.,* "Nucleic Acid Hybridization, A Practical Approach," Eds. Hames and Higgins, IRL Press (1985).

The sensitivity of the hybridization assays may be enhanced through use of a nucleic acid amplification system which multiplies the target nucleic acid being detected. In vitro amplification techniques suitable for amplifying sequences for use as molecular probes or for generating nucleic acid fragments for subsequent subcloning are known. Examples of techniques sufficient to direct persons of skill through such in vitro amplification methods, including the polymerase chain reaction (PCR) the ligase chain reaction (LCR), Qβ-replicase amplification and other RNA polymerase mediated techniques (e.g., NASBA^{™}) are found in Sambrook et al., In Molecular Cloning: A Laboratory Manual, Cold Spring Harbor Laboratory Press (2000); and Ausubel et al., SHORT PROTOCOLS IN MOLECULAR BIOLOGY, eds., Current Protocols, Greene Publishing Associates, Inc. and John Wiley & Sons, Inc. (2002); as well as U.S. Pat. No. 4,683,202; PCR Protocols, A Guide to Methods and Applications (Innis et al. eds.) Academic Press Inc. San Diego, Calif. (1990); Arnheim & Levinson (Oct. 1, 1990), C&EN 36; The Journal Of NIH Research, 3:81 (1991); Kwoh et al., Proc. Natl. Acad. Sci. USA, 86:1173 (1989); Guatelli et al., Proc. Natl. Acad. Sci. USA, 87:1874 (1990); Lomell et al., J. Clin. Chem., 35:1826 (1989); Landegren et al., Science, 241:1077 (1988); Van Brunt, Biotechnology, 8:291 (1990); Wu and Wallace, Gene, 4:560 (1989); Barringer et al., Gene, 89:117 (1990); and Sooknanan and Malek, Biotechnology, 13:563 (1995). Improved methods of cloning in vitro amplified nucleic acids are described in U.S. Pat. No. 5,426,039. Other methods described in the art are the nucleic acid sequence based amplification (NASBA^{™}, Cangene, Mississauga, Ontario) and Qβ-replicase systems. These systems can be used to directly identify mutants where the PCR or LCR primers are designed to be extended or ligated only when a select sequence is present. Alternatively, the select sequences can be generally amplified using, for example, nonspecific PCR primers and the amplified target region later probed for a specific sequence indicative of a mutation.

Nucleic acids for use as probes, *e.g.,* in in vitro amplification methods, for use as gene probes, or as inhibitor components are typically synthesized chemically according to the solid phase phosphoramidite triester method described by Beaucage et al., Tetrahedron Letts., 22:1859 1862 (1981), *e.g.,* using an automated synthesizer, as described in Needham VanDevanter et al., Nucleic Acids Res., 12:6159 (1984). Purification of polynucleotides, where necessary, is typically performed by either native acrylamide gel electrophoresis or by anion exchange HPLC as described in Pearson et al., J. Chrom., 255:137 149 (1983). The sequence of the synthetic polynucleotides can be verified using the chemical degradation method of Maxam and Gilbert (1980) in Grossman and Moldave (eds.) Academic Press, New York, Methods in Enzymology, 65:499.

An alternative means for determining the level of transcription is in situ hybridization. In situ hybridization assays are well-known and are generally described in Angerer et al., Methods Enzymol., 152:649 (1987). In an in situ hybridization assay, cells are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters.

### Examples

PEG2000-C-DMA (US 8,936,942) has been used in lipid nanoparticle (LNP) formulations that have entered human clinical trials in applications as diverse as oncology, vaccines, antivirals and metabolic diseases. These LNP formulations have been used to deliver therapeutic payloads including but not limited to nucleic acids such as plasmid DNA, siRNA, mRNA and self-replicating RNA. PEG-2000-C-DMA is a close structural analog of PEG-C-DOMG, used in the FDA-approved Onpattro.

The nature (*e.g.,* size, number, saturation level) of the alkyl domain of PEG lipids can have effects on the biological performance of LNP *in vivo.* This alkyl domain serves as an "anchor" that allows formulation and incorporation into the LNP resulting in surface presentation of the PEG domain. In addition to allowing formulation of small stable particles and masking these particles *in vivo* to avoid innate immune responses, PEG lipids are also important for biological activity. PEG lipids diffuse away from the LNP following administration *in vivo,* and in the case of liver-directed formulations, are concomitantly substituted by APOE facilitating hepatocyte uptake via the LDL receptor. Consequently, the rate at which PEG lipids are removed from the LNP, which in turn is directly related to the efficacy of the alkyl domain anchor, is an important property.

As part of a strategy to optimize and improve LNP formulations for nucleic acid delivery, a series of PEG lipids was designed and synthesized in which the two alkyl chains, of the same length, were attached to a single atom (either carbon or nitrogen). This contrasts with the design motif in C-DMA and DOMG in which the alkyl domains are attached via glycidol linkers. Several chemical functionalities were designed and investigated to couple the PEG domain to the dialkyl domain. These novel PEG lipids performed generally as well or better than the benchmark PEG-2000-C-DMA when comparing the same length alkyl chains (*e.g.,* C₁₄). It was discovered that certain motifs (*e.g.,* carbamate and urea) surprisingly produced more efficacious particles than other related compounds.

General synthesis of symmetrical dialkylamines exemplified by the synthesis of ditetradecylamine **(5)**

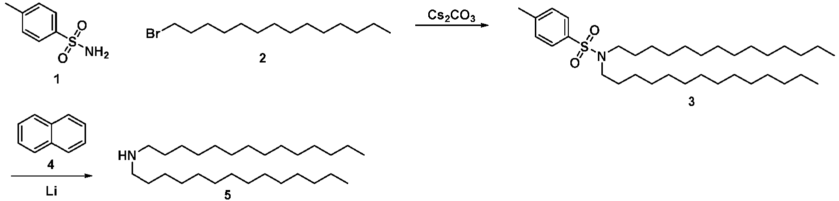

### a. Synthesis of 4-methyl-N,N-ditetradecylbenzenesulfonamide (3)

K₂CO₃ (20.18 g, 146.0 mmol), p-toluenesulfonamide **(1)** (5.00 g, 29.2 mmol) and 1-bromotetradecane **(2)** (21.7 ml, 73.0 mmol) were heated at reflux in DMF (75 ml) for 20 h. The reaction was cooled to RT, diluted with Et₂O (100 ml) and filtered. The filtrate was washed with H₂O (50 ml) and the aqueous back extracted with Et₂O (100 ml). The combined organics were dried (Na₂SO₄), filtered, concentrated *in-vacuo* and the residue purified by automated flash chromatography (DCM/hexane 40/60) to give N,N-ditetradecyl-p-toluenesulfonamide **(3)** (15.86 g, 96 %).

### b. Synthesis of ditetradecylamine (5)

4-Methyl-N,N-ditetradecylbenzenesulfonamide **(3)** (10 g, 17.7 mmol) in THF (5 ml) was added to a solution of lithium napthalenide (prepared from stirring naphthalene **(4)** (11.36 g, 88.7 mmol) and lithium metal (0.92 g, 133.0 mmol) in THF (40 ml) at RT for 1 h) at 0°C and the reaction stirred for 1 h at RT. MeOH (4 ml) was added, followed by H₂O (50 ml) and the mixture extracted with ether (100 ml). The organics were dried (Na₂SO₄), filtered and concentrated *in-vacuo.* The residue was purified by automated flash chromatography (EtOAc/Hex (50:50)) to give ditetradecylamine **(5)** (4.85 g, 66.7 %).

### Example 1. General Synthesis of Dialkylcarbamate PEG Lipids Exemplified by Compound(9)

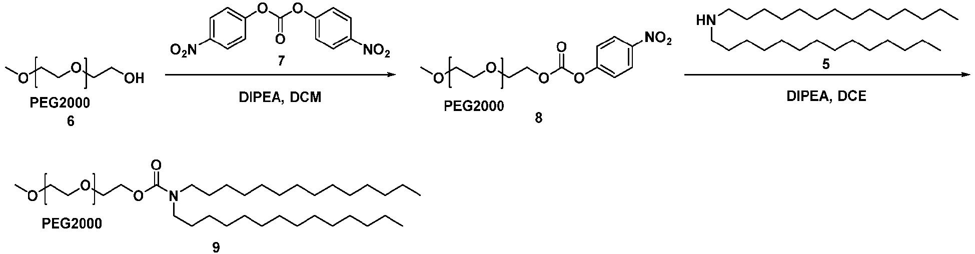

### a. Synthesis of PEG2000 p-nitrocarbonate (8)

Methoxy PEG 2000 OH **(6)** and DIPEA (0.8 ml, 4.6 mmol) were stirred in DCM (100 ml) at RT. Bis(4-nitrophenyl carbonate (7) (1.4 g, 4.6 mmol) was added and the reaction stirred at RT for 16 h. The reaction was washed with NaHCO₃ (3 x 150 ml), brine (150 ml), dried (MgSO₄), filtered and concentrated *in-vacuo.* The residue was purified by automated flash chromatography (0-15% MeOH/DCM) to give PEG2000 p-Nitrocarbonate **(8)**

### b. Synthesis of dialkylcarbamate PEG lipid (9)

PEG2000 p-Nitrocarbonate **(8)** (1g, 0.42 mmol), DIPEA (0.15 ml, 0.85 mmol) and ditetradecylamine **(5)** (0.174 g, 0.42 mmol) were heated in DCE at reflux for 16h. The reaction was cooled, washed with saturated NaHCO₃ (5 x 75 ml), dried (MgSO₄) and concentrated *in-vacuo.* The residue was purified by automated flash chromatography (0-15% MeOH/DCM). This material was further purified with tangential flow ultrafiltration (MWCO = 10000 kDA) with 10 x volume water washes. The recovered aqueous solution was subject to lyophilization to give Compound **(9)** (406 mg, 36 %). ¹H NMR (400 MHz, D6-DMSO) δ 4.21 (m, 3H), 3.62 (m, 136 H), 3.38 (s, 3H), 3.15 (bs, 5H), 1.49 (bs, 4H), 1.24 (bs, 35H), 0.87 (t, J = 8 Hz, 6H).

### Example 2. General synthesis of dialkylamide PEG lipids exemplified by compound (13)

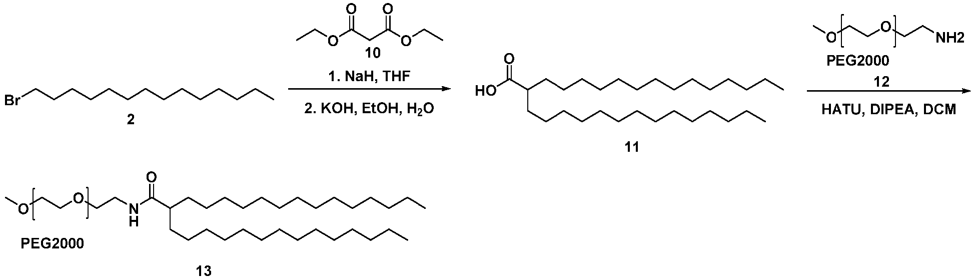

### a. Synthesis of 2-tetradecylhexadecanoic acid (11)

Diethyl malonate **(10)** (10.0 g, 9.5 mmol) was added dropwise to a suspension of NaH (60% dispersion in mineral oil) (2.75 g, 68.7 mmol) in dry THF at 0°C. 1-bromotetradecane **(2)** (19.5 ml, 65.6 mmol) was added and the reaction refluxed for 18 h. The reaction was cooled, quenched with MeOH, concentrated *in-vacuo* and the residue partitioned between hexane and H₂O. The organic layer was washed with H₂O and brine, dried (MgSO₄), filtered and concentrated *in-vacuo.* The residue was purified by automated flash chromatography (5% EtOAc/Hex). This material was subsequently subjected to a second alkylation step in an identical fashion as above. The intermediate diethyl-2,2,dimyristal-malonate was dissolved in EtOH (250 ml), KOH (24.5 g, 437 mmol) in H₂O (250 ml) added and the reaction stirred at reflux for 18 h. The reaction was cooled, poured onto ice and acidified with 3 M HCl. The aqueous was extracted with EtOAc, the organics dried (MgSO₄) and concentrated *in-vacuo.* The residue was heated without solvent at 170°C under vacuum until gas evolution ceased. After cooling, the crude material was purified by automated flash chromatography (5% MeOH, DCM) to give 2-tetradecylhexadecanoic acid **(11)** (7.24 g, 24.4%)

### b. Synthesis of dialkylamide PEG lipid (13)

2-Tetradecylhexadecanoic acid **(11)** (0.142 g, 0.31 mmol), methoxy PEG 2000 amine **(12)** (500 mg, 0.25 mmol), HATU (0.143 g, 0.375 mmol) and DIPEA (0.13 mL, 0.75 mmol) were stirred in DCM at RT for 18 h. The reaction was diluted with DCM, washed sequentially with saturated NaHCO₃ and brine, dried (MgSO₄), filtered and concentrated *in-vacuo.* The residue was purified by automated flash chromatography (0-15% MeOH/DCM). The pure PEG-lipid was dissolved in water (8mL) and lyophilized to give dialkylamide PEG lipid (13) (365 mg, 60.0 %). 1H NMR (400 MHz, D6-DMSO) δ 3.81 (m, 1H), 3.64 (bs, 179H), 3.53 (m, 5H), 3.45 (m, 1H), 3.35 (m, 3H), 2.8 (s, 6H), 1.31 (bs, 53H), 0.9 (t, J = 8 Hz, 6H).

### Example 3. General Procedure for the Synthesis of Dialkyester PEG Lipids Exemplified by Compound (20)

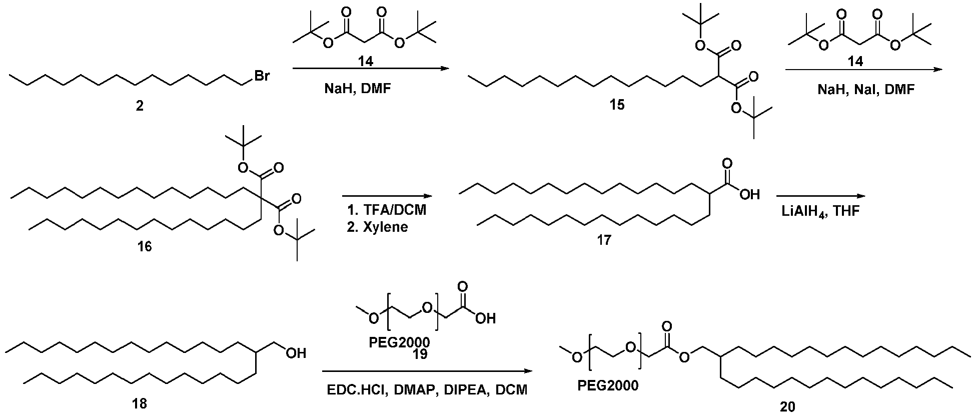

### a. Synthesis of di-tert-butyl 2-tetradecylmalonate (15)

NaH (60% dipersion in mineral oil) (453 mg, 11.3 mmol) was dissolved in DMF (100 mL) and cooled to 0°C. Di-tert-butyl malonate **(14)** (2.2 g, 10.3 mmol) was added and the reaction stirred for 10 min. 1-Bromotetradecane **(2)** (3.0 g, 10.8 mmol) was added and the reaction stirred at rt for 18 h. The reaction was cooled to 0°C, quenched with saturated NH₄Cl solution, and extracted with hexane. The organic layer was washed with brine, dried (MgSO₄), filtered and concentrated *in-vacuo.* The residue was purified by automated flash chromatography (0-10% EtOAc/Hex to give **(15)** (4.1g, 95.7%)

### b. Synthesis of di-tert-butyl 2,2-ditetradecylmalonate (16)

NaH 60% dispersion in mineral oil (433 mg, 10.8 mmol) was dissolved in DMF (100 mL) and cooled to 0°C. Di-tert-butyl 2-tetradecylmalonate () (4.1 g, 9.9 mmol) was added and the mixture stirred for 10 min. 1-bromotetradecane **(2)** (2.87 g, 10.3 mmol) and NaI (0.44 g, 3.0 mmol) was added and the reaction stirred at rt for 3 days. The reaction was cooled to 0°C, quenched with saturated NH₄Cl solution and extracted with EtOAc. The organic layer was washed with brine, dried (MgSO₄), filtered and concentrated *in-vacuo.* The residue was purified by automated flash chromatography (0-5% EtOAc/Hex) to give di-tert-butyl 2,2-ditetradecylmalonate **(16)** (5.0 g, 83.8 %).

### c. Synthesis of 2-tetradecylhexadecanoic acid (17)

Di-tert-butyl 2,2-ditetradecylmalonate **(16)** (5.5 g, 9.1 mmol) was dissolved in DCM (40 mL) and TFA (20 mL) was added at 0°C. The reaction was stirred at RT for 2h, then azeotroped with toluene twice. The residue was dissolved in xylene (30 mL) and stirred at 150°C for 18h. The reaction was cooled to RT and concentrated *in-vacuo.* The residue was dissolved in Hexane (20 ml), heated to 50°C to dissolve and hot loaded onto a flash column. The product was purified by automated flash chromatography (0-30% EtOAc/Hex) to give 2-tetradecylhexadecanoic acid **(17)** (2.2 g, 52.8 %).

### d. Synthesis of 2-tetradecylhexadecan-1-ol (18)

A solution of 2-tetradecylhexadecanoic acid **(17)** (2.2 g, 5.0 mmol) in THF (100 mL) was added to a stirred solution of LiAlH₄ (364 mg, 9.6 mmol) in THF at 0°C. The reaction was warmed to RT and stirred for 18 h. A solution of saturated Na₂SO₄*10H₂O solution was added dropwise with stirring at 0°C. The resultant PPT was removed by filtration through Celite. The filtrate was concentrated *in-vacuo* and the residue purified by automated flash chromatography (0-20% EtOAc/hex) to give 2-tetradecylhexadecan-1-ol **(18)** (1.9 g, 92.1%).

### e, Synthesis of dialkyester PEG lipid (20)

PEG2000-CO₂H **(19)** (1.0 g, 0.45 mmol), 2-tetradecylhexadecan-1-ol **(18)** (0.3 g, 0.68 mmol), EDC.HCl (0.22 g, 1.1 mmol), DMAP (6 mg, 0.05 mmol) and DIPEA (0.18 g, 1.36 mmol) were dissolved in DCM (20 mL) at 0°C. The reaction was warmed to RT and stirred for 3 d. Additional EDC.HCl (0.22 g, 1.13 mmol) was added and the reaction heated at reflux for 18 h. After cooling the reaction was washed with brine, dried (MgSO₄), filtered and the filtrate concentrated *in-vacuo.* The residue was purified by automated flash chromatography (0-10%MeOH/DCM). The recovered product was further purified with tangential flow ultrafiltration (MWCO = 10000 kDA) with 10 x volume water washes. The recovered aqueous solution was subject to lyophilization to give dialkyester PEG lipid **(20)** (187 mg, 15.7 %). 1H NMR (400 MHz, CDCl₃) δ 4.14 (s, 2H), 4.05 (d, J = 4 Hz, 2H), 3.64 (PEG), 3.38 (S, 3H), 1.25 (bs, 54H), 0.88 (t, J = 8 Hz, 6H).

### Example 4. General Procedure for the Synthesis of Dialkyether PEG Lipids Exemplified by Compound (24)

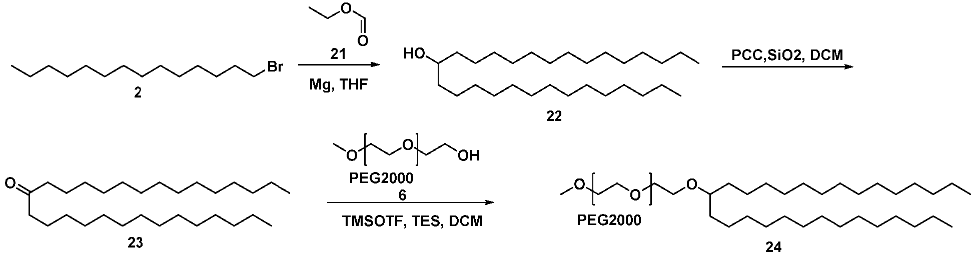

### a. Synthesis of nonacosan-15-ol (22)

Myristyl magnesium bromide was prepared using standard Grignard conditions from 1-Bromotetradecane (2) (20 g, 72.1 mmol) and magnesium turnings (1.75 g, 72.1 mmol) in dry THF. After stirring for 2 h at RT, ethyl formate **(21)** (5.8 ml, 72.1 mmol) was added dropwise and the reaction stirred for 16 h. H₂O (100mL) was added followed by slow addition of 6M HCl until all magnesium was dissolved. The resultant PPT was removed by filtration and the filtrate concentrated *in-vacuo* to give nonacosan-15-ol **(22)** (3.5 g, 22.8 %) which was used subsequently without further purification.

### b. Synthesis of nonacosan-15-one (23)

Nonacosan-15-ol **(22)** (500 mg, 1.2 mmol) was dissolved in DCM (20 mL). PCC on Si (1.5 g, 3.5 mmol) was added and the reaction stirred at RT for 16h. The reaction was filtered and the filtrate concentrated *in-vacuo* to give crude nonacosan-15-one **(23)** which was used in the subsequent without purification.

### c. Synthesis of dialkyether PEG lipid (24)

Methoxy PEG2000-OH **(6)** (1.5 g, 0.75 mmol) was dissolved dry DCM (25 mL) and cooled to 0°C. Trimethylsilyl trifluoromethanesulfonate (0.22 g, 1 mmol) was added and the reaction stirred for 20 min. Nonacosan-15-one **(23)** (211 mg, 0.5 mmol) and triethylsilane (116 mg, 1 mmol) were added sequentially. After 5 mins additional trifluoromethanesulfonate (111 mg, 0.5 mmol) was added. The reaction was stirred at RT for 2 h, quenched with 1M NaOH (10 mL) and diluted with DCM (50 mL). The organics were washed with H₂O (50 mL), brine (50 mL), dried (MgSO₄), filtered and concentrated *in-vacuo.* The residue was purified by automated flash chromatography (0-15% MeOH/DCM) to give dialkyether PEG lipid **(24)** (635 mg, 54 %). 1H NMR (400 MHz, CDCl₃) δ 3.8 (m, 2H), 3.63 (m, 156H), 3.37 (s, 3H), 3.22 (m, 2H), 1.24 (m, 48H), 0.87 )t, J = 8 Hz, 6H).

### Example 5. General Procedure for the Synthesis of Dialkyurea PEG Lipids exemplified by Compound (26)

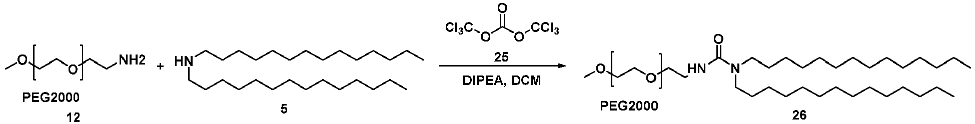

### a. Synthesis of dialkylurea PEG lipid (26)

Methoxy PEG2000 amine **(12)** (500 mg, 0.25 mmol) and DIPEA (0.096 mL, 0.55 mmol) were stirred in DCM (5 mL). Triphosgene **(25)** (27 mg, 0.093 mmol) was added and the reaction stirred at RT for 4 h. ditetradecylamine (5) (102 mg, 0.25 mmol) in DCM (5 mL) was added dropwise and the reaction stirred at RT for 18 h. The reaction was diluted with DCM, washed with brine, dried (MgSO₄), filtered and concentrated *in-vacuo.* The crude material was purified by automated flash chromatography (0-15% MeOH/DCM). The recovered product was further purified by tangential flow-ultrafiltration (10 wash volumes) and lyophilized to give dialkylurea PEG lipid (26) (250 mg, 0.102 mmol, 40.8 %). 1H NMR (400 MHz, CDCl₃) δ 3.63 (m, 146H), 3.54 (m, 6H), 3.37 (s, 3H), 3.15 (t, J = 8Hz, 2H), 1.82 (bs, 6H), 1.51 (bs, 4H), 1.24 (m, 40 H), 0.87 (t, J = 8Hz, 6H).

### Example 6. General procedure for the synthesis of dialkysulfonamide PEG lipids exemplified by (29)

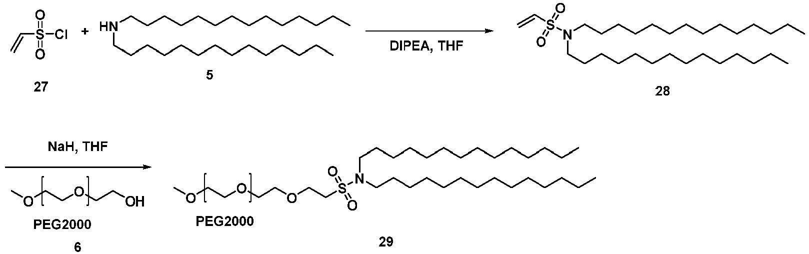

### a. Synthesis of N,N-ditetradecylethenesulfonamide (28)

Ethenesulfonyl chloride (27) (154 mg, 1.2 mmol) was added to ditetradecylamine (5) (0.5 g, 1.2 mmol) and DIPEA (0.34 ml, 2.5 mmol) at RT. The reaction was stirred at RT for 2 hours, concentrated *in-vacuo* and the residue purified by automated flash chromatography (7% EtOAc/Hex) to give N,N-ditetradecylethenesulfonamide **(28)** (190 mg, 31 %). 1H NMR (400 MHz, CDCl₃) δ 6.41 (m, 1H), 6.18 (D, J = 16 Hz, 1H), (5.88, D, J = 12 Hz, 1 H), 3.09 (t, J = 8 Hz, 4H), 1.56 (BS, 8 H), 1.27 (BS, 41 H), 0.87 (t, J = 8Hz, 6H).

### b. Synthesis of dialkysulfonamide PEG lipid (29)

Methoxy PEG 2000 **(6)** (1.2 g, 0.56 mmol) and NaH (60% dispersion in mineral oil)(22 mg, 0.56 mmol) were stirred in dry THF at RT for 30 min. N,N-ditetradecylethenesulfonamide **(28)** (280 mg, 0.56 mmol) was added and the reaction stirred at RT for 16 h. The reaction was concentrated *in-vacuo* and the residue purified by automated flash chromatography (7% MeOH/DCM) to give dialkysulfonamide PEG lipid **(29)** (500 mg, 34 %). 1H NMR (400 MHz, CDCl₃) δ 3.84 (m, 3H), 3.58 (m, 110H), 3.37 (s, 3H), 3.21 (t, J = 8Hz, 2H), 3.13 (t, t, 8 Hz, 3H), 1.72 (BS, 4H), 1.56 (BS, 4H), 1.27 (BS, 34H), 0.87 (t, J = 8 Hz, 6H).

### Example 7. Synthesis of Compound 30

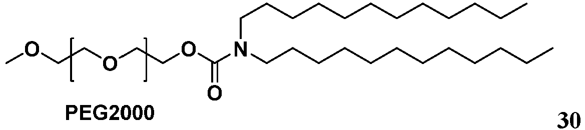

Compound **30** was prepared according to Example 1, 'General Synthesis of Dialkylcarbamate PEG Lipids' . After purification 465 mg of the required product was obtained in 43% yield from the appropriate starting materials. 1H NMR (400 MHz, CDCl₃) δ 4.20 (m, 2H), 3.80 (m, 1H), 3.66-3.45 (m, 154H), 3.37 (S, 3H), 3.17 (BS, 4H), 2.10 (BS, 5H), 1.48 (BS, 4H), 1.24 (BS, 36H), 0.87 (t, 6H, J = 4Hz).

### Example 8. Synthesis of Compound 31

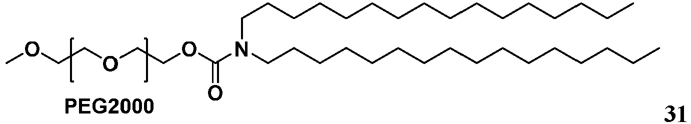

Compound **31** was prepared according to Example 1, 'General Synthesis of Dialkylcarbamate PEG Lipids' . After purification 664 mg of the required product was obtained in 58% yield from the appropriate starting materials. 1H NMR (400 MHz, CDCl₃) δ 4.20 (m, 2H), 3.80 (m, 1H), 3.66-3.45 (m, 167H), 3.37 (S, 3H), 3.17 (BS, 4H), 1.76 (BS, 5H), 1.48 (BS, 4H), 1.24 (BS, 50 H), 0.87 (t, 6H, J = 8Hz)

### Example 9. Preparation of Lipid Nanoparticle Formulations

A lipid solution was prepared with 4 components: a PEG-conjugated lipid (either PEG-C-DMA control, or a PEG-lipid of the present invention), an ionizable lipid ((6Z,16Z)-12-((Z)-dec-4-en-1-yl)docosa-6,16-dien-11-yl 5-(dimethylamino)pentanoate as described in US 9,352,042), cholesterol, and DSPC. The percent molar ratios of the 4 components were 1.6 to 54.6 to 32.8 to 10.9 respectively, and the solution had a total lipid concentration of ~7 mg/mL in 100% ethanol. Firefly luciferase mRNA (TriLink Biotechnologies, L-7202) was diluted in acetate, pH 5 buffer and nuclease free water to achieve a target concentration of 0.366 mg/mL mRNA in 100 mM acetate, pH 5. Equal volumes of the lipid and nucleic acid solutions were blended at a flow rate of 400 mL/min through a T-connector, and diluted with ~4 volumes of PBS, pH 7.4. Formulations were placed in Slide-A-Lyzer dialysis units (MWCO 10,000) and dialyzed overnight against 10 mM Tris, 500 mM NaCl, pH 8 buffer. Following dialysis, the formulations were concentrated to ~ 0.6 mg/mL using VivaSpin concentrator units (MWCO 100,000) and dialyzed overnight against 5 mM Tris, 10% sucrose, pH 8 buffer. Formulations were filtered through a 0.2 µm syringe filter (PES membrane). Nucleic acid concentration was determined by the RiboGreen assay. Particle size and polydispersity were determined using a Malvern Nano Series Zetasizer.

### Example 10. In vivo Administration of LNPs

LNP formulations encapsulating firefly luciferase mRNA were injected intravenously at 0.5 mg/kg to female BALB/c mice (6-8 weeks old). On day of injection, the LNP stocks were filtered and diluted to the required dosing concentration with phosphate buffered saline. At 6 hours post-administration, animals were euthanized with a lethal dosage of ketamine/xylazine. Blood samples were collected into EDTA microtainer tubes and centrifuged at 16,000xg for 5 minutes at 4°C. Plasma samples were removed and stored at -80°C until cytokine analysis performed. Liver samples (left lateral lobe) were collected, weighed, and flash frozen in liquid nitrogen. Liver samples were stored in FastPrep^{®} tubes at -80°C until analyzed for luciferase activity.

### Example 11. Luciferase Activity Analysis

Frozen aliquots of liver were thawed and homogenized in 1mL of 1xCCLR (Cell Culture Lysis Reagent) using a FastPrep^{®} homogenizer. The homogenate was then centrifuged at 16,000 RPM for 10 minutes at 4 °C. Twenty (20) µL of the supernatant was loaded into a 96-well white plate and luminescence measured following addition of luciferase reagent (Promega Luciferase Assay System). Luciferase activity was determined by comparing luminescence of the homogenized samples to luciferase protein standards. To account for any quenching of luminescence by components in the liver homogenate, luciferase was added to the liver homogenates of untreated animals and the resulting luminescence measured. The resulting quench factor was applied to all samples to obtain the corrected luciferase activity and normalized per unit mass of tissue analyzed.

Luciferase activity of 0.5 mg/kg LNP containing firefly luciferase mRNA and various PEG-conjugated lipids was measured at 6 hours following intravenous administration in BALB/c mice (n=4); results are shown in the following Table.

| **Treatment** | **6h post-IV administration** | |
|---|---|---|
| **[LNP mol ratios of 1.6: 54.6: 32.8: 10.9; PEG-lipid: Ionizable lipid: Cholesterol: DSPC]** | Luc Activity (pg/g liver) | Std Dev (pg/g liver) |
| PEG-C-DMA (Benchmark) | 4.91E+07 | 7.95E+06 |
| Compound 9 - Example 1 | 8.26E+07 | 1.44E+07 |
| Compound 13 - Example 2 | 5.98E+07 | 1.22E+07 |
| Compound 20 - Example 3 | 2.81E+07 | 3.16E+06 |
| Compound 24 - Example 4 | 2.88E+07 | 3.84E+06 |
| Compound 26 - Example 5 | 6.65E+07 | 1.81E+07 |
| Compound 29 - Example 6 | 7.25E+07 | 4.03E+07 |

### Example 12. Repeat dose potency and tolerability in rat model

**Preparation of Lipid Nanoparticle Formulations.** These were prepared as described in Example 9, except formulations encapsulated human erythropoietin (EPO) mRNA (TriLink Biotechnologies, L-7209).

**In vivo administration of LNPs.** LNP formulations encapsulating human EPO mRNA were injected intravenously at 0.25 mg/kg to male Sprague Dawley rats (7-8 weeks old). Animals were dosed weekly (Q7D) for a total of 3 doses on Days 0, 7, and 14. On each day of injection, the LNP stocks were filtered and diluted to the required dosing concentration with phosphate buffered saline. Prior to test article administration, at 6 h post-dose, and at the terminal timepoint (6 h post-third dose), blood samples were collected into EDTA microtainer tubes and centrifuged at 16,000 x g for 5 minutes at 4°C. All plasma samples were stored at - 80°C until EPO expression and anti-PEG antibody analyses were performed.

**Anti-PEG antibody analysis.** An ELISA method was used to measure the presence of anti-PEG IgM and IgG antibodies in the rat plasma samples. Millipore Multiscreen-HTS IP Plates (Millipore, MSIPN4510) were coated with 0.5 mg/mL PEG lipid and held at 37°C until dry. After blocking with 10% FBS in PBS and washing once with 1% FBS in PBS, plasma samples were plated at 1/30 dilution onto 2 separate plates. After washing three times, a goat anti-rat IgG conjugated to peroxidase (Cedarlane, 112-036-072) was added to one plate, and a goat anti-rat IgM conjugated to peroxidase (Cedarlane, 112-036-075) was added to the second plate. After incubation and washing, TMB Substrate (BD Biosciences, 555214) was added for color development and the reaction was stopped with 2N sulfuric acid. Content from each well was transferred to a clear 96-well plate for absorbance reading at 450/570 OD.

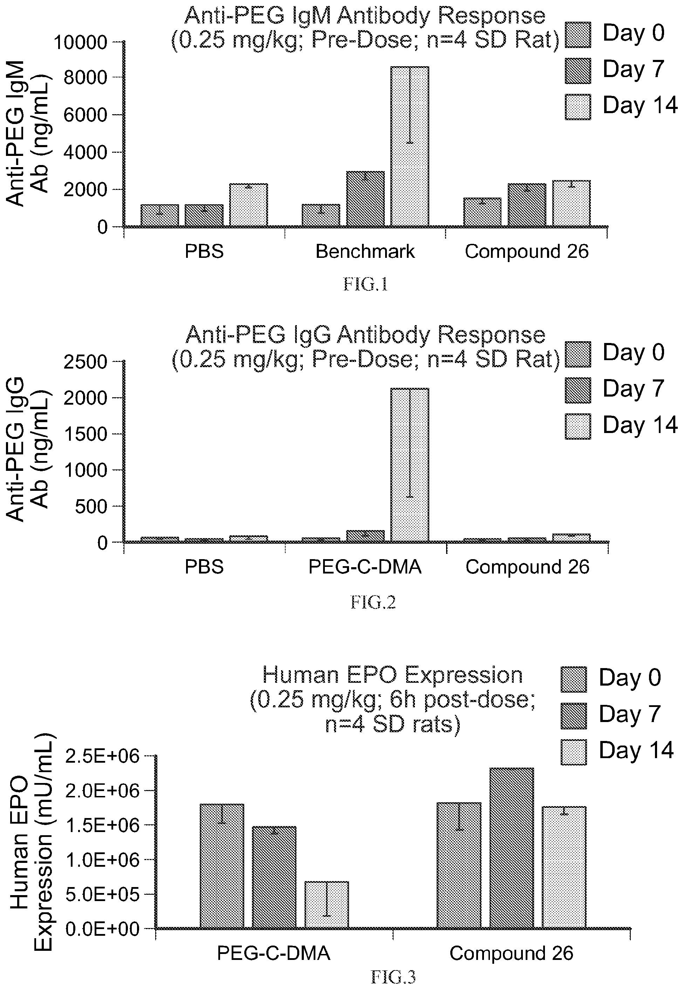

LNP bearing nucleic acid payloads can be immunogenic if they circulate in the blood for a prolonged period. Antibodies (IgG/IgM) recognizing the surface chemistry of the LNP (typically the PEG polymer) are raised and readily detected in the blood. This can be problematic for repeat administrations of LNP, as the anti-PEG antibodies induced by the first dose detect and clear the subsequent doses, causing loss of potency and hypersensitive reactions. The effect is exacerbated by more inflammatory payloads, including mRNA, which cannot be chemically modified to the same degree as other nucleic payloads (e.g., siRNA) as a mitigation strategy. PEG lipids were identified that promoted faster uptake by the liver, and more rapid clearance from the blood. PEG-C-DMA was used as a benchmark, which has been employed in several clinical siRNA-LNP products. Compounds of the present invention, such as Compound 26, exhibited a far lower antibody (IgG/IgM) response than the benchmark PEG-C-DMA (see Figures 1 and 2).

**EPO expression analysis.** Human EPO concentration in rat plasma was determined using an EPO ELISA assay (catalog #DEP00; R&D systems) according to the manufacturer's instructions.

EPO expression was measured at 6 hours following each 0.25 mg/kg intravenous dose in SD rats (n=4). Loss of potency (human EPO expression) was observed upon repeat administration of LNP containing PEG-C-DMA but not Compound **26** (see Figure 3). These data correlate with the levels of anti-PEG antibodies induced by the first dose, which recognize and rapidly clear subsequent doses rendering them ineffective.

### Example 13. Single dose OTC expression in non-human primate model

**Preparation of Lipid Nanoparticle Formulations.** These were prepared as described in Example 9, except formulations encapsulate mRNA encoding human ornithine transcarbamylase (OTC) that was custom synthesized at TriLink Biotechnologies.

**In vivo administration of LNPs.** LNP formulations encapsulating human OTC mRNA were administered at 0.5 mg/kg to naive cynomolgus monkeys (n=4; 2-3 years old) via 60 min intravenous infusion. On day of dosing, the LNP stocks were filtered and diluted to the required dosing concentration with 0.9% saline. Blood samples were collected into EDTA microtainer tubes at various timepoints post-start of infusion and centrifuged at 16,000 x g for 5 minutes at 4°C. All plasma samples were stored at -80°C until lipid analysis was performed. At the terminal timepoint (24 h post-dose), 500 mg of liver from the left lateral lobe was collected and snap-frozen in liquid nitrogen. Liver samples were stored in a freezer set to maintain -80°C until analyzed for human OTC expression by a LC-MS based method.

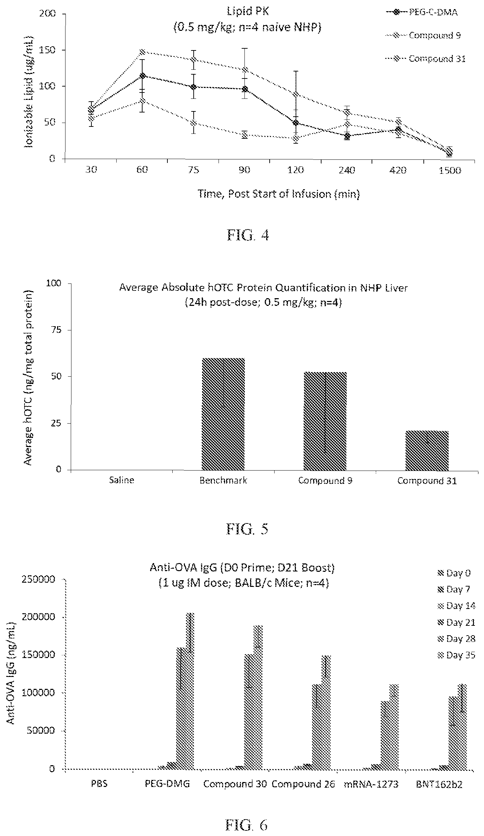

**Lipid clearance.** A LC-MS based method was used to measure the amount of ionizable lipid present in the NHP plasma samples over time. (see Figure 4) Both PEG-C-DMA and Compound **9** comprise two C14 alkyl chains conjugated to a PEG polymer component with a molecular weight of ~2000 g/mol; however, Compound **9** reported much more rapid clearance in NHP. This is consistent with the observation that LNP comprising PEG-lipids of the present invention are less immunogenic than PEG-C-DMA. Compound **31** is an analog of Compound **9** but has a larger lipid anchor holding it into the LNP (two C16 alkyl chains, instead of C14 chains). This suggests that PEG-lipids with larger anchors are more tightly bound to the LNP and promote a longer circulation time.

**OTC protein expression.** A LC-MS based method was used to measure the amount of human OTC protein present in the NHP liver samples 24 h post-dose. (see Figure 5) As expected, the slower pharmacokinetics of Compound **31** resulted in lower protein expression in NHP liver, while both PEG-C-DMA and Compound 9 reported similar levels of human OTC protein.

### Example 14. Immunogenicity in mouse OVA model following intramuscular administration

**Preparation of Lipid Nanoparticle Formulations.** These were prepared as described in Example 9, except formulations encapsulate ovalbumin (OVA) mRNA (TriLink Biotechnologies, L-7210).

**In vivo administration of LNPs.** LNP formulations encapsulating OVA mRNA were administered intramuscularly at 1 µg dose to BALB/c mice (7-8 weeks old) on Day 0 (prime) and Day 21 (boost). Blood samples were collected into EDTA microtainer tubes at pre-dose and at days 7, 14, 21, and 28. Terminal blood was collected on Day 35. Plasma samples were stored in a freezer set to maintain -80°C until analyzed for anti-OVA IgG by ELISA.

**Anti-OVA IgG analysis.** An ELISA method was used to measure the production of anti-OVA IgG antibodies, a measure of efficacy in a vaccine setting. (see Figure 6) Two PEG lipids of the present invention (Compound **26,** Compound **30**) were formulated in a 1.5: 50: 38.5: 10 (PEG-lipid: Ionizable lipid: Cholesterol: DSPC), as well as the benchmark compound PEG-DMG. PEG-DMG is a commercially available PEG lipid comprising two C14 alkyl chains and is used in the Moderna COVID vaccine mRNA-1273, which served as a benchmark in this study. In addition, the LNP compositions were compared with the LNP compositions for the mRNA-1273 and BNT162b2 COVID vaccines. Following two IM doses, LNPs containing either Compound **26** or Compound **30,** reported similar anti-OVA IgG levels to the benchmark comparators (PEG-DMG, mRNA-1273, and BNT162b2).

## Claims

1. A compound of formula (I): 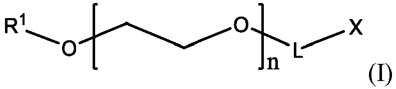 or a salt thereof, wherein:
R¹ is H, (C₁-C₆)alkyl, or (C₁-C₆)alkanoyl;
n is an integer in the range of from about 10 to about 150; and
L is absent and X is -C(=O)NR²R³; or
L is (C₁-C₆)alkyl and X is selected from the group consisting of -N(R⁴)C(=O)CH(R²)(R³), -OCH(R²)(R³), -C(=O)OCH₂CH(R²)(R³), -N(R⁴)C(=O)N(R²)(R³), and -SO₂N(R²)(R³);
R² is (C₁₀-C₂₀)alkyl;
R³ is (C₁₀-C₂₀)alkyl; and
R⁴ is H or (C₁-C₆)alkyl.

2. The compound of claim 1, wherein:
(i) L is absent and X is -C(=O)NR²R³; or
(ii) L is (C₁-C₆)alkyl and X is -N(R4)C(=O)CH(R²)(R³); or
(iii) L is (C₁-C₆)alkyl and X is -OCH(R²)(R³); or
(iv) L is (C₁-C₆)alkyl and X is -C(=O)OCH₂CH(R²)(R³); or
(v) L is (C₁-C₆)alkyl and X is -N(R⁴)C(=O)N(R²)(R³); or
(vi) L is (C₁-C₆)alkyl and X is -SO₂N(R²)(R³).

3. The compound of claim 1 or 2, wherein:
(i) R¹ is H; or
(ii) R¹ is (C₁-C₆)alkyl; or
(iii) R¹ is methyl; or
(iv) R¹ is (C₁-C₆)alkanoyl; or
(v) R¹ is CH₃C(=O)-.

4. The compound of any one of claims 1-3, wherein:
(i) n is an integer in the range of from about 20 to about 100; or
(ii) n is an integer in the range of from about 20 to about 70; or
(iii) n is an integer in the range of from about 20 to about 60; or
(iv) n is an integer in the range of from about 20 to about 50; or
(v) n is an integer in the range of from about 30 to about 100; or
(vi) n is an integer in the range of from about 40 to about 100; or
(vii) n is an integer in the range of from about 40 to about 60; or
(viii) n is an integer in the range of from about 40 to about 50; or
(ix) n is an integer in the range of from about 44 to about 46.

5. The compound of any one of claims 1-4, wherein:
(i) R² is C₁₁-alkyl, C₁₂-alkyl, C₁₃-alkyl, C₁₄-alkyl, C₁₅-alkyl, C₁₆-alkyl, C₁₇-alkyl, C₁₈-alkyl, C₁₉-alkyl, or C₂₀-alkyl; or
(ii) R² is C₁₂-alkyl, C₁₃-alkyl, C₁₄-alkyl, C₁₅-alkyl, C₁₆-alkyl, C₁₇-alkyl, or C₁₈-alkyl; or
(iii) R² is C₁₂-alkyl, C₁₄-alkyl, or C₁₆-alkyl; or
(iv) R² is C₁₄-alkyl.

6. The compound of any one of claims 1-5, wherein:
(i) R³ is C₁₁-alkyl, C₁₂-alkyl, C₁₃-alkyl, C₁₄-alkyl, C₁₅-alkyl, C₁₆-alkyl, C₁₇-alkyl, C₁₈-alkyl, C₁₉-alkyl, or C₂₀-alkyl; or
(ii) R³ is C₁₂-alkyl, C₁₃-alkyl, C₁₄-alkyl, C₁₅-alkyl, C₁₆-alkyl, C₁₇-alkyl, or C₁₈-alkyl; or
(iii) R³ is C₁₂-alkyl, C₁₄-alkyl, or C₁₆-alkyl; or
(iv) R³ is C₁₄-alkyl.

7. The compound of any one of claims 1-6, wherein:
(i) R⁴ is H; or
(ii) R⁴ is (C₁-C₆)alkyl; or
(iii) R⁴ is methyl.

8. The compound of claim 1 that is selected from: 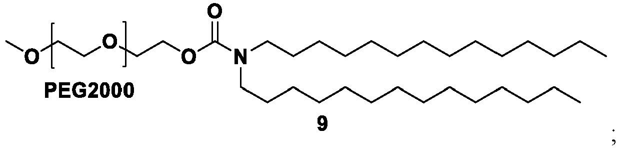 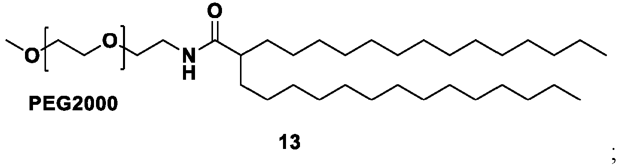 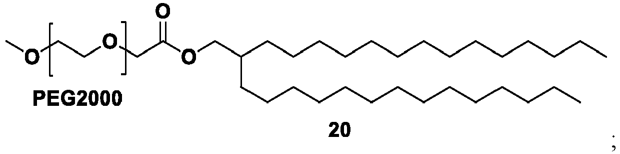 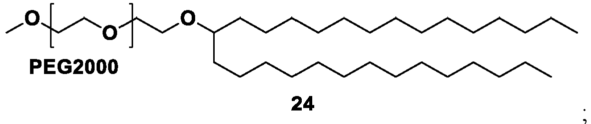 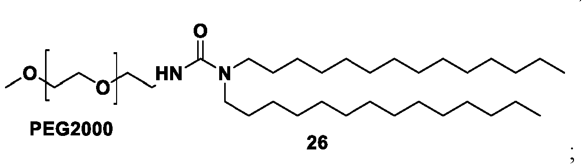 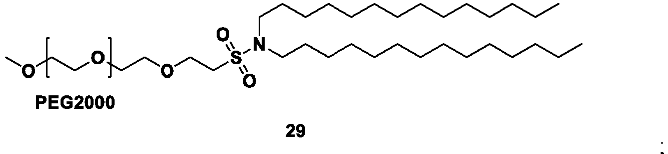 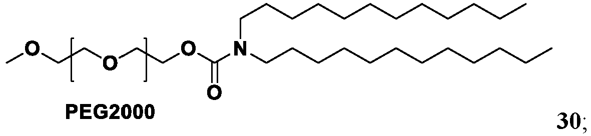 and 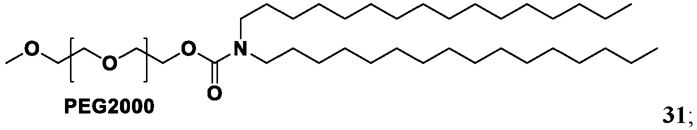 or a salt thereof.

9. A pharmaceutical composition comprising a compound as described in any one of claims 1-8 or a salt thereof, and a pharmaceutically acceptable carrier.

10. A lipid nanoparticle comprising a compound as described in any one of claims 1-8 or a salt thereof, and optionally wherein the lipid nanoparticle further comprises a cationic lipid, a non-cationic lipid (*e.g.,* a phospholipid, *e.g.,* DSPC), cholesterol and a therapeutic agent, and optionally wherein the cationic lipid is selected from: 3-(((6Z,9Z,28Z,31Z)-heptatriaconta-6,9,28,31-tetraen-19-yl)oxy)-N,N-dimethylpropan-1-amine; (6Z,9Z,28Z,31Z)-heptatriaconta-6,9,28,31-tetraen-19-yl 4-(dimethylamino)butanoate; (6Z,16Z)-12-((Z)-dec-4-en-1-yl)docosa-6,16-dien-11-yl 5-(dimethylamino)pentanoate; (6Z,16Z)-12-((Z)-dec-4-en-1-yl)docosa-6,16-dien-11-yl 6-(dimethylamino)hexanoate; N,N-dimethyl-4-(tris(((Z)-dec-4-en-1-yl)oxy)silyl)butan-1-amine; N,N-dimethyl-5-(tris(((Z)-dec-4-en-1-yl)oxy)silyl)pentan-1-amine; N,N-dimethyl-6-(tris(((Z)-dec-4-en-1-yl)oxy)silyl)hexan-1-amine; 2-(methyl(4-(tris(((Z)-dec-4-en-1-yl)oxy)silyl)butyl)amino)ethan-1-ol; (6Z,16Z)-12-((6-(dimethylamino)hexanoyl)oxy)docosa-6,16-dien-11-yl (Z)-undec-5-enoate; N1,N3-bis(4-(bis(((Z)-dec-4-en-1-yl)oxy)(methyl)silyl)butyl)-N1,N3-dimethylpropane-1,3-diamine; N1,N3-dimethyl-N1,N3-bis(4-(tris(((Z)-hept-3-en-1-yl)oxy)silyl)butyl)propane-1,3-diamine; (1r,4r)-N1,N4-bis(4-(bis(((Z)-dec-4-en-1-yl)oxy)(methyl)silyl)butyl)-N1,N4-dimethylcyclohexane-1,4-diamine; 2,8-bis(4-(bis(((Z)-dec-4-en-1-yl)oxy)(methyl)silyl)butyl)-2,8-diazaspiro[4.5]decane; or bis(2-butyloctyl) 10-(N-(3-(dimethylamino)propyl) nonanamido)- nonadecanedioate; di(tridecan-7-yl) 10-(N-(3-(dimethylamino)propyl)octanamido)-nonadecanedioate; di(tridecan-7-yl) 10-(N-decyl-4-(dimethylamino)butanamido)nonadecanedioate; ((4-hydroxybutyl)azanediyl)bis(nonane-9,1-diyl) bis(2-butyloctanoate) or heptadecan-9-yl 8-((2-hydroxyethyl)(8-(nonyloxy)-8-oxooctyl)amino)octanoate; 3-((4,4-bis(octyloxy)butanoyl)oxy)-2-((((3-(diethylamino)propoxy)carbonyl)oxy)methyl)propyl (9Z,12Z)-octadeca-9,12-dienoate or 3,6-bis(4-(bis(2-hydroxydodecyl)amino)butyl)piperazine-2,5-dione; 3,6-bis(4-(bis((9Z,12Z)-2-hydroxyoctadeca-9,12-dien-1-yl)amino)butyl)piperazine-2,5-dione; 1,1'-((2-(1-(2-((2-(bis(2-hydroxydodecyl)amino)ethyl)(2-hydroxydodecyl)amino)ethyl)piperidin-4-yl)ethyl)azanediyl)bis(dodecan-2-ol); tetratridecyl 3,3',3",3‴-((azanediylbis(propane-3,1-diyl))bis(azanetriyl))tetrapropionate or nonyl 8-((8,8-bis(octyloxy)octyl)(2-hydroxyethyl)amino)octanoate or di((Z)-non-2-en-1-yl) 8,8'-((((2-(dimethylamino)ethyl)thio)carbonyl)azanediyl)dioctanoate.

11. The lipid nanoparticle of claim 10 which further comprises a cationic lipid, a non-cationic lipid (*e.g.,* a phospholipid, *e.g.,* DSPC), cholesterol and a therapeutic agent, wherein:
(i) the therapeutic agent is a nucleic acid; or
(ii) the therapeutic agent is DNA, and optionally the DNA is in the form of an antisense molecule, plasmid DNA, pre-condensed DNA, a PCR product, a vector, an expression cassette, a chimeric sequence, or chromosomal DNA, or a combination thereof, or the DNA is in the form of siRNA, asymmetrical interfering RNA (aiRNA), microRNA (miRNA), mRNA, tRNA, rRNA, tRNA, viral RNA (vRNA), or self-amplifying RNA, or a combination thereof; or
(iii) the therapeutic agent is RNA; or
(iv) the therapeutic agent is mRNA; or
(v) the therapeutic agent is siRNA.

12. A pharmaceutical composition comprising a lipid nanoparticle as described in claim 10 or 11, and a pharmaceutically acceptable carrier, and optionally wherein the pharmaceutical composition is formulated for intravenous or intramuscular administration.

13. A lipid nanoparticle as defined in claim 11 wherein the therapeutic agent is mRNA, for use in a method for treating a disease **characterized by** a deficiency of a functional protein, the method comprising administering to a subject having the disease the lipid nanoparticle, wherein the mRNA encodes the functional protein or a protein having the same biological activity as the functional protein.

14. A lipid nanoparticle as defined in claim 11 wherein the therapeutic agent is siRNA, for use in a method for treating a disease **characterized by** overexpression of a polypeptide, the method comprising administering to a subject having the disease the lipid nanoparticle, wherein the siRNA targets expression of the overexpressed polypeptide.

15. A lipid nanoparticle as defined in claim 11 wherein the therapeutic agent is mRNA, or a pharmaceutical composition as defined in claim 12 and which is formulated for intramuscular administration, for use in a method for delivering a vaccine to a subject in need thereof, comprising administering to a subject a therapeutically effective amount of the lipid nanoparticle or the pharmaceutical composition.

## Patentansprüche

1. Verbindung der Formel (I): 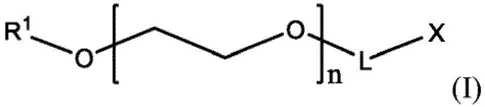 oder ein Salz davon, wobei:
R¹ H, (C₁-C₆)-Alkyl oder (C₁-C₆)-Alkanoyl ist;
n eine Ganzzahl im Bereich von etwa 10 bis etwa 150 ist; und
L nicht vorhanden ist und X -C(=O)NR²R³ ist; oder
L (C₁-C₆)-Alkyl ist und X ausgewählt ist aus der Gruppe bestehend aus - N(R⁴)C(=O)CH(R²)(R³), -OCH(R²)(R³), -C(=O)OCH₂CH(R²)(R³), -N(R⁴)C(=O)N(R²)(R³) und -SO₂N(R²)(R³);
R² (C₁₀-C₂₀)-Alkyl ist;
R³ (C₁₀-C₂₀)-Alkyl ist; und
R⁴ H oder (C₁-C₆)-Alkyl ist.

2. Verbindung nach Anspruch 1, wobei:
(i) L nicht vorhanden ist und X -C(=O)NR²R³ ist; oder
(ii) L (C₁-C₆)-Alkyl ist und X -N(R4)C(=O)CH(R²)(R³) ist; oder
(iii) L (C₁-C₆)-Alkyl ist und X -OCH(R²)(R³) ist; oder
(iv) L (C₁-C₆)-Alkyl ist und X -C(=O)OCH₂CH(R²)(R³) ist; oder
(v) L (C₁-C₆)-Alkyl ist und X -N(R⁴)C(=O)N(R²)(R³); oder
(vi) L (C₁-C₆)-Alkyl ist und X -SO₂N(R²)(R³) ist.

3. Verbindung nach Anspruch 1 oder 2, wobei:
(i) R¹ H ist; oder
(ii) R¹ (C₁-C₆)-Alkyl ist; oder
(iii) R¹ Methyl ist; oder
(iv) R¹ (C₁-C₆)-Alkanoyl ist; oder
(v) R¹ CH₃C(=O)- ist.

4. Verbindung nach einem der Ansprüche 1-3, wobei:
(i) n eine Ganzzahl im Bereich von etwa 20 bis etwa 100 ist; oder
(ii) n eine Ganzzahl im Bereich von etwa 20 bis etwa 70 ist; oder
(iii) n eine Ganzzahl im Bereich von etwa 20 bis etwa 60 ist; oder
(iv) n eine Ganzzahl im Bereich von etwa 20 bis etwa 50 ist; oder
(v) n eine Ganzzahl im Bereich von etwa 30 bis etwa 100 ist; oder
(vi) n eine Ganzzahl im Bereich von etwa 40 bis etwa 100 ist; oder
(vii) n eine Ganzzahl im Bereich von etwa 40 bis etwa 60 ist; oder
(viii) n eine Ganzzahl im Bereich von etwa 40 bis etwa 50 ist; oder
(ix) n eine Ganzzahl im Bereich von etwa 44 bis etwa 46 ist.

5. Verbindung nach einem der Ansprüche 1-4, wobei:
(i) R² C₁₁-Alkyl, C₁₂-Alkyl, C₁₃-Alkyl, C₁₄-Alkyl, C₁₅-Alkyl, C₁₆-Alkyl, C₁₇-Alkyl, C₁₈-Alkyl, C₁₉-Alkyl oder C₂₀-Alkyl ist; oder
(ii) R² C₁₂-Alkyl, C₁₃-Alkyl, C₁₄-Alkyl, C₁₅-Alkyl, C₁₆-Alkyl, C₁₇-Alkyl oder C₁₈-Alkyl ist; oder
(iii) R² C₁₂-Alkyl, C₁₄-Alkyl oder C₁₆-Alkyl ist; oder
(iv) R² C₁₄-Alkyl ist.

6. Verbindung nach einem der Ansprüche 1-5, wobei:
(i) R³ C₁₁-Alkyl, C₁₂-Alkyl, C₁₃-Alkyl, C₁₄-Alkyl, C₁₅-Alkyl, C₁₆-Alkyl, C₁₇-Alkyl, C₁₈-Alkyl, C₁₉-Alkyl oder C₂₀-Alkyl ist; oder
(ii) R³ C₁₂-Alkyl, C₁₃-Alkyl, C₁₄-Alkyl, C₁₅-Alkyl, C₁₆-Alkyl, C₁₇-Alkyl oder C₁₈-Alkyl ist; oder
(iii) R³ C₁₂-Alkyl, C₁₄-Alkyl oder C₁₆-Alkyl ist; oder
(iv) R³ C₁₄-Alkyl ist.

7. Verbindung nach einem der Ansprüche 1-6, wobei:
(i) R⁴ H ist; oder
(ii) R⁴ (C₁-C₆)-Alkyl ist; oder
(iii) R⁴ Methyl ist.

8. Verbindung nach Anspruch 1, die ausgewählt ist aus: 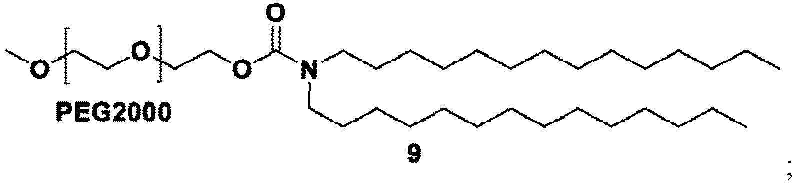 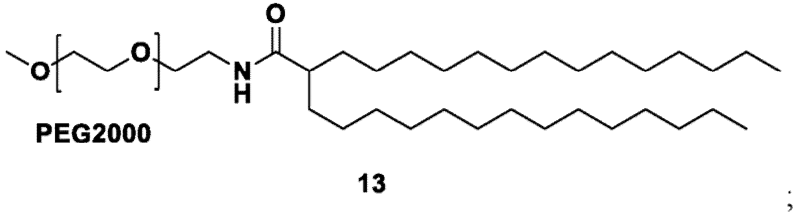 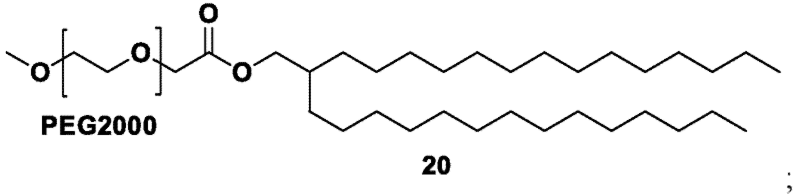 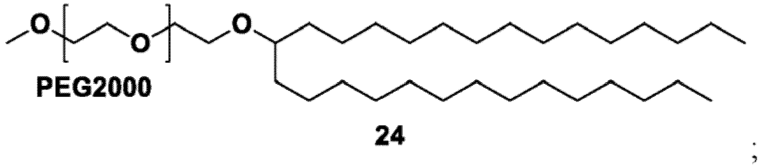 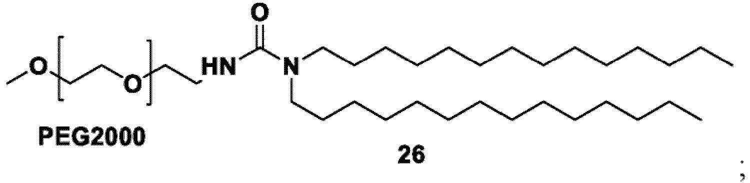 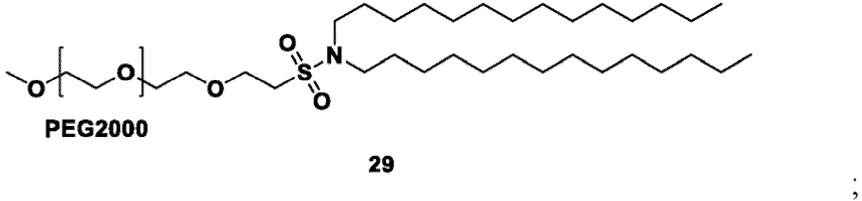 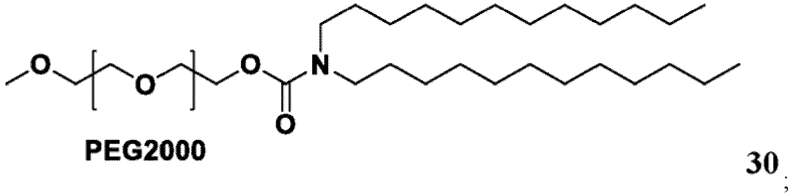 und 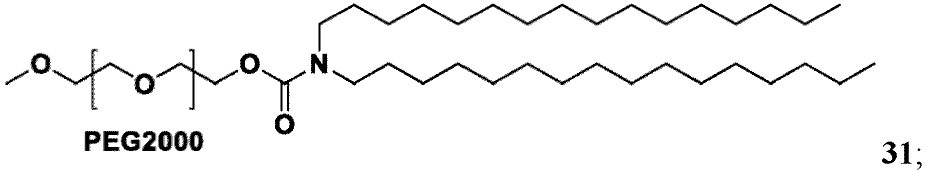 oder einem Salz davon.

9. Pharmazeutische Zusammensetzung, umfassend eine Verbindung wie sie in einem der Ansprüche 1-8 beschrieben ist, oder ein Salz davon, und einen pharmazeutisch annehmbaren Träger.

10. Lipid-Nanopartikel, umfassend eine Verbindung, wie sie in einem der Ansprüche 1-8 beschrieben ist, oder ein Salz davon, und wobei optional das Lipid-Nanopartikel ferner ein kationisches Lipid, ein nicht-kationisches Lipid (*z. B.* ein Phospholipid, *z. B.* DSPC), Cholesterin und ein Therapeutikum umfasst, und wobei optional das kationische Lipid ausgewählt ist aus: 3-(((6Z,9Z,28Z,31Z)-Heptatriaconta-6,9,28,31-tetraen-19-yl)oxy)-N,N-dimethylpropan-1-amin; (6Z,9Z,28Z,31Z)-Heptatriaconta-6,9,28,31-tetraen-19-yl-4-(dimethylamino)butanoat; (6Z,16Z)-12-((Z)-Dec-4-en-1-yl)docosa-6,16-dien-11-yl-5-(dimethylamino)pentanoat; (6Z,16Z)-12-((Z)-Dec-4-en-1-yl)docosa-6,16-dien-11-yl-6-(dimethylamino)hexanoat; N,N-Dimethyl-4-(tris(((Z)-dec-4-en-1-yl)oxy)silyl)butan-1-amin; N,N-Dimethyl-5-(tris(((Z)-dec-4-en-1-yl)oxy)silyl)pentan-1-amin; N,N-Dimethyl-6-(tris(((Z)-dec-4-en-1-yl)oxy)silyl)hexan-1-amin; 2-(Methyl(4-(tris(((Z)-dec-4-en-1-yl)oxy)silyl)butyl)amino)ethan-1-ol ; (6Z,16Z)-12-((6-(Dimethylamino)hexanoyl)oxy)docosa-6,16-dien-11-yl-(Z)-undec-5-enoat; N1,N3-Bis(4-(bis(((Z)-dec-4-en-1-yl)oxy)(methyl)silyl)butyl)-N1,N3-dimethylpropan-1,3-diamin; N1,N3-Dimethyl-N1,N3-bis(4-(tris(((Z)-hept-3-en-1-yl)oxy)silyl)butyl)propan-1,3-diamin; (1r,4r)-N1,N4-Bis(4-(bis(((Z)-dec-4-en-1-yl)oxy)(methyl)silyl)butyl)-N1,N4-dimethylcyclohexan-1,4-diamin; 2,8-Bis(4-(bis(((Z)-dec-4-en-1-yl)oxy)(methyl)silyl)butyl)-2,8-diazaspiro[4.5]decan; oder Bis(2-butyloctyl)-10-(N-(3-(dimethylamino)propyl)nonanamido)-nonadecandioat; Di(tridecan-7-yl) 10-(N-(3-(dimethylamino)propyl)octanamido)-nonadecandioate; Di(tridecan-7-yl)-10-(N-decyl-4-(dimethylamino)butanamido)nonadecandioat; ((4-Hydroxybutyl)azandiyl)bis(nonan-9,1-diyl)-bis(2-butyloctanoat) oder Heptadecan-9-yl-8-((2-hydroxyethyl)(8-(nonyloxy)-8-oxooctyl)amino)octanoat; 3-((4,4-Bis(octyloxy)butanoyl)oxy)-2-((((3-(diethylamino)propoxy)carbonyl)oxy)methyl)propyl-(9Z,12Z)-octadeca-9,12-dienoat oder 3,6-Bis(4-(bis(2-hydroxydodecyl)amino)butyl)piperazin-2,5-dion; 3,6-Bis(4-(bis((9Z,12Z)-2-hydroxyoctadeca-9,12-dien-1-yl)amino)butyl)piperazin-2,5-dion; 1,1'-((2-(1-(2-((2-(Bis(2-hydroxydodecyl)amino)ethyl)(2-hydroxydodecyl)amino)ethyl)piperidin-4-yl)ethyl)azandiyl)bis(dodecan-2-ol); Tetratridecyl-3,3',3",3‴-((azandiylbis(propan-3,1-diyl))bis(azantriyl))tetrapropionat oder Nonyl-8-((8,8-bis(octyloxy)octyl)(2-hydroxyethyl)amino)octanoat oder Di((Z)-non-2-en-1-yl)-8,8'-((((2-(dimethylamino)ethyl)thio)carbonyl)azanediyl)dioctanoat.

11. Lipid-Nanopartikel nach Anspruch 10, das ferner ein kationisches Lipid, ein nicht-kationisches Lipid (*z. B.* ein Phospholipid, *z. B.* DSPC), Cholesterin und ein Therapeutikum umfasst, wobei:
(i) das Therapeutikum eine Nukleinsäure ist; oder
(ii) das Therapeutikum DNA ist und optional die DNA in der Form eines Antisense-Moleküls, Plasmid-DNA, vorkondensierter DNA, eines PCR-Produkts, eines Vektors, einer Expressionskassette, einer chimären Sequenz oder chromosomaler DNA oder einer Kombination davon ist, oder die DNA in der Form von siRNA, asymmetrischer RNA-Interferenz (aiRNA), microRNA (miRNA), mRNA, tRNA, rRNA, tRNA, viraler RNA (vRNA) oder selbstverstärkender RNA oder einer Kombination davon ist; oder
(iii) das Therapeutikum RNA ist; oder
(iv) das Therapeutikum mRNA ist; oder
(v) das Therapeutikum siRNA ist.

12. Pharmazeutische Zusammensetzung, umfassend ein Lipid-Nanopartikel, wie es in Anspruch 10 oder 11 beschrieben ist, und einen pharmazeutischen Träger, und wobei optional die pharmazeutische Zusammensetzung zur intravenösen oder intramuskulären Verabreichung formuliert ist.

13. Lipid-Nanopartikel nach Anspruch 11, wobei das Therapeutikum mRNA ist, zur Verwendung in einem Verfahren zum Behandeln einer Krankheit, **gekennzeichnet durch** einen Mangel eines funktionellen Proteins, wobei das Verfahren das Verabreichen an ein Subjekt, das die Krankheit hat, des Lipid-Nanopartikels umfasst, wobei die mRNA das funktionelle Protein oder ein Protein mit der gleichen biologischen Aktivität wie das funktionelle Protein kodiert.

14. Lipid-Nanopartikel nach Anspruch 11, wobei das Therapeutikum siRNA ist, zur Verwendung in einem Verfahren zum Behandeln einer Krankheit, **gekennzeichnet durch** Überexpression eins Polypeptids, wobei das Verfahren das Verabreichen an ein Subjekt, das die Krankheit hat, des Lipid-Nanopartikels umfasst, wobei die siRNA auf die Expression des überexprimierten Polypeptids abzielt.

15. Lipid-Nanopartikel nach Anspruch 11, wobei das Therapeutikum mRNA ist oder eine pharmazeutische Zusammensetzung nach Anspruch 12, und die formuliert ist zur intramuskulären Verabreichung, zur Verwendung in einem Verfahren zum Zuführen eines Impfstoffs an ein Subjekt, das dessen bedarf, umfassend das Verabreichen an ein Subjekt einer therapeutisch wirksamen Menge des Lipid-Nanopartikels oder der pharmazeutischen Zusammensetzung.

## Revendications

1. Composé répondant à la formule (I) : 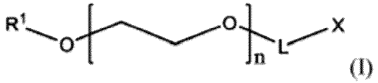 ou sel de celui-ci, où :
R¹ représente H, alkyle en (C₁-C₆) ou alcanoyle en (C₁-C₆) ;
n représente un nombre entier dans la plage allant d'environ 10 à environ 150 ; et
L est absent et X représente -C(=O)NR²R³ ; ou
L représente alkyle en (C₁-C₆) et X est sélectionné dans le groupe constitué de - N(R⁴)C(=O)CH(R²)(R³), -OCH(R²)(R³), -C(=O)OCH₂CH(R²)(R³), -N(R⁴)C(=O)N(R²)(R³), et -SO₂N(R²)(R³) ;
R² représente alkyle en (C₁₀-C₂₀) ;
R³ représente alkyle en (C₁₀-C₂₀) ; et
R⁴ représente H ou alkyle en (C₁-C₆).

2. Composé selon la revendication 1, dans lequel :
(i) L est absent et X représente -C(=O)NR²R³ ; ou
(ii) L représente alkyle en (C₁-C₆) et X représente -N(R⁴)C(=O)CH(R²)(R³) ; ou
(iii) L représente alkyle en (C₁-C₆) et X représente -OCH(R²)(R³) ; ou
(iv) L représente alkyle en (C₁-C₆) et X représente -C(=O)OCH₂CH(R²)(R³) ; ou
(v) L représente alkyle en (C₁-C₆) et X représente -N(R⁴)C(=O)N(R²)(R³) ; ou
(vi) L représente alkyle en (C₁-C₆) et X représente -SO₂N(R²)(R³).

3. Composé selon la revendication 1 ou 2, dans lequel :
(i) R¹ représente H ; ou
(ii) R¹ représente alkyle en (C₁-C₆) ; ou
(iii) R¹ représente méthyle ; ou
(iv) R¹ représente alcanoyle en (C₁-C₆) ; ou
(v) R¹ représente CH₃C(=O)-.

4. Composé selon l'une quelconque des revendications 1 à 3, dans lequel :
(i) n représente un nombre entier dans la plage allant d'environ 20 à environ 100 ; ou
(ii) n représente un nombre entier dans la plage allant d'environ 20 à environ 70 ; ou
(iii) n représente un nombre entier dans la plage allant d'environ 20 à environ 60 ; ou
(iv) n représente un nombre entier dans la plage allant d'environ 20 à environ 50 ; ou
(v) n représente un nombre entier dans la plage allant d'environ 30 à environ 100 ; ou
(vi) n représente un nombre entier dans la plage allant d'environ 40 à environ 100 ; ou
(vii) n représente un nombre entier dans la plage allant d'environ 40 à environ 60 ; ou
(viii) n représente un nombre entier dans la plage allant d'environ 40 à environ 50 ; ou
(ix) n représente un nombre entier dans la plage allant d'environ 44 à environ 46.

5. Composé selon l'une quelconque des revendications 1 à 4, dans lequel :
(i) R² représente alkyle en C₁₁, alkyle en C₁₂, alkyle en C₁₃, alkyle en C₁₄, alkyle en C₁₅, alkyle en C₁₆, alkyle en C₁₇, alkyle en C₁₈, alkyle en C₁₉ ou alkyle en C₂₀ ; ou
(ii) R² représente alkyle en C₁₂, alkyle en C₁₃, alkyle en C₁₄, alkyle en C₁₅, alkyle en C₁₆, alkyle en C₁₇ ou alkyle en C₁₈ ; ou
(iii) R² représente alkyle en C₁₂, alkyle en C₁₄ ou alkyle en C₁₆ ; ou
(iv) R² représente alkyle en C₁₄.

6. Composé selon l'une quelconque des revendications 1 à 5, dans lequel :
(i) R³ représente alkyle en C₁₁, alkyle en C₁₂, alkyle en C₁₃, alkyle en C₁₄, alkyle en C₁₅, alkyle en C₁₆, alkyle en C₁₇, alkyle en C₁₈, alkyle en C₁₉ ou alkyle en C₂₀ ; ou
(ii) R³ représente alkyle en C₁₂, alkyle en C₁₃, alkyle en C₁₄, alkyle en C₁₅, alkyle en C₁₆, alkyle en C₁₇ ou alkyle en C₁₈ ; ou
(iii) R³ représente alkyle en C₁₂, alkyle en C₁₄ ou alkyle en C₁₆ ; ou
(iv) R³ représente alkyle en C₁₄.

7. Composé selon l'une quelconque des revendications 1 à 6, dans lequel :
(i) R⁴ représente H ; ou
(ii) R⁴ représente alkyle en (C₁-C₆) ; ou
(iii) R⁴ représente méthyle.

8. Composé selon revendication 1, sélectionné parmi : 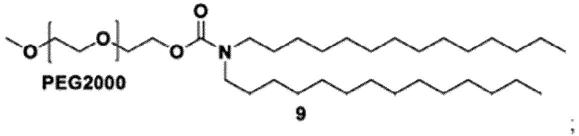 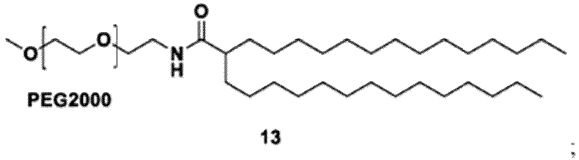 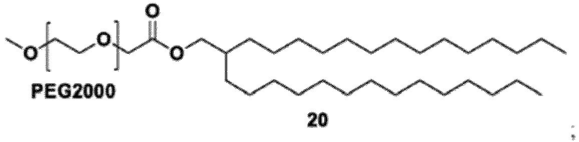 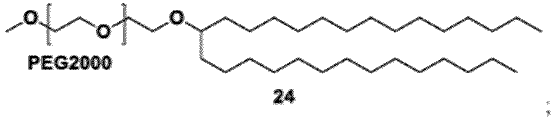 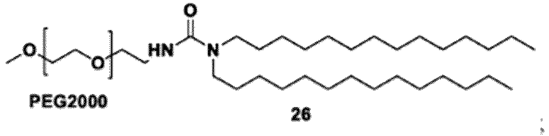 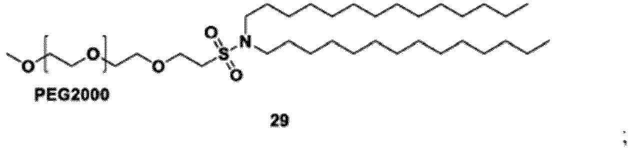 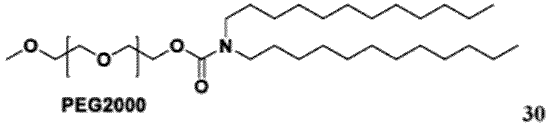 et 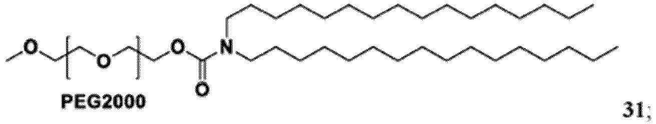 ou sel de celui-ci.

9. Composition pharmaceutique comprenant un composé tel que décrit dans l'une quelconque des revendications 1 à 8 ou un sel de celui-ci ainsi qu'un vecteur pharmaceutiquement acceptable.

10. Nanoparticule lipidique comprenant un composé tel que décrit dans l'une quelconque des revendications 1 à 8 ou un sel de celui-ci, la nanoparticule lipidique comprenant éventuellement en outre un lipide cationique, un lipide non cationique (par exemple un phospholipide, par exemple DSPC), du cholestérol et un agent thérapeutique, ledit lipide cationique étant éventuellement sélectionné parmi : 3-(((6Z,9Z,28Z,31Z)-heptatriaconta-6,9,28,31-tetraén-19-yl)oxy)-N,N-diméthylpropan-1-amine ; 4-(diméthylamino)butanoate de (6Z,9Z,28Z,31Z)-heptatriaconta-6,9,28,31-tetraén-19-yle ; 5-(diméthylamino)pentanoate de (6Z,16Z)-12-((Z)-déc-4-én-1-yl)docosa-6,16-dién-11-yle ; 6-(diméthylamino)hexanoate de (6Z,16Z)-12-((Z)-déc-4-én-1-yl)docosa-6,16-dién-11-yle ; N,N-diméthyl-4-(tris(((Z)-déc-4-én-1-yl)oxy)silyl)butan-1-amine ; N,N-diméthyl-5-(tris(((Z)-déc-4-én-1-yl)oxy)silyl)pentan-1-amine ; N,N-diméthyl-6-(tris(((Z)-déc-4-én-1-yl)oxy)sily)hexan-1-amine ; 2-(méthyl(4-(tris(((Z)-déc-4-én-1-yl)oxy)silyl)butyl)amino)éthan-1-ol ; (Z)-undéc-5-énoate de (6Z,16Z)-12-((6-(diméthylamino)hexanoyl)oxy)docosa-6,16-dién-11-yle ; N1,N3-bis(4-(bis(((Z)-déc-4-én-1-yl)oxy)(méthyl)silyl)butyl)-N1,N3-diméthylpropane-1,3-diamine ; N1,N3-diméthyl-N1,N3-bis(4-(tris(((Z)-hept-3-én-1-yl)oxy)silyl)butyl)propane-1,3-diamine ; (1r,4r)-N1,N4-bis(4-(bis(((Z)-déc-4-én-1-yl)oxy)(méthyl)silyl)butyl)-N1,N4-diméthylcyclohexane-1,4-diamine ; 2,8-bis(4-(bis(((Z)-déc-4-én-1-yl)oxy)(méthyl)silyl)butyl)-2,8-diazaspiro[4.5]décane ; ou 10-(N-(3-(diméthylamino)propyl) nonanamido)-nonadécanedioate de bis(2-butyloctyle) ; 10-(N-(3-(diméthylamino)propyl)octanamido)-nonadécanedioate de di(tridécan-7-yl)e ; 10-(N-décyl-4-(diméthylamino)butanamido)nonadécanedioate de di(tridécan-7-yle) ; bis(2-butyloctanoate) de ((4-hydroxybutyl)azanediyl)bis(nonane-9,1-diyle) ou 8-((2-hydroxyéthyl)(8-(nonyloxy)-8-oxooctyl)amino)octanoate de heptadécan-9-yle ; (9Z,12Z)-octadéca-9,12-diénoate 3-((4,4-bis(octyloxy)butanoyl)oxy)-2-((((3-(diéthylamino)propoxy)carbonyl)oxy)méthyl)propyle ou 3,6-bis(4-(bis(2-hydroxydodécyl)amino)butyl)pipérazine-2,5-dione ; 3,6-bis(4-(bis((9Z,12Z)-2-hydroxyoctadéca-9,12-diène-1-yl)amino)butyl)pipérazine-2,5-dione ; 1,1'-((2-(1-(2-((2-(bis(2-hydroxydodécyl)amino)éthyl)(2-hydroxydodécyl)amino)éthyl)pipéridin-4-yl)éthyl)azanediyl)bis(dodécan-2-ol) ; 3,3',3",3"'-((azanediylbis(propane-3,1-diyl))bis(azanetriyl))tétrapropionate de tétratridécyle ou 8-((8,8-bis(octyloxy)octyl)(2-hydroxyéthyl)amino)octanoate de nonyle ou 8,8'-((((2-(diméthylamino)éthyl)thio)carbonyl)azanediyl)dioctanoate de di((Z)-non-2-én-1-yle).

11. Nanoparticule lipidique selon la revendication 10, comprenant en outre un lipide cationique, un lipide non cationique (par exemple un phospholipide, par exemple DSPC), du cholestérol et un agent thérapeutique, étant entendu que :
(i) l'agent thérapeutique est un acide nucléique ; ou
(ii) l'agent thérapeutique est de l'ADN et, éventuellement, l'ADN se présente sous la forme d'une molécule antisens, d'ADN de plasmide, d'ADN précondensé, d'un produit de PCR, d'un vecteur, d'une cassette d'expression, d'une séquence chimérique ou d'ADN chromosomique, ou d'une combinaison de ceux-ci, ou l'ADN est sous la forme d'un ARNsi, d'un ARN interférent asymétrique (ARNai), d'un microARN (ARNmi), d'un ARNm, d'un ARNt, d'un ARNr, d'un ARNt, d'un ARN viral (ARNv) ou d'un ARN auto-amplifié, ou d'une combinaison de ceux-ci ; ou
(iii) l'agent thérapeutique est de l'ARN ; ou
(iv) l'agent thérapeutique est un ARNm ; ou
(v) l'agent thérapeutique est un ARNsi.

12. Composition pharmaceutique comprenant une nanoparticule lipidique telle que décrite dans la revendication 10 ou 11, et un vecteur pharmaceutiquement acceptable, ladite composition pharmaceutique étant éventuellement formulée pour une administration intraveineuse ou intramusculaire.

13. Nanoparticule lipidique telle que définie dans la revendication 11, dans laquelle l'agent thérapeutique est un ARNm, destinée à être utilisée dans une méthode de traitement d'une maladie **caractérisée par** une déficience en une protéine fonctionnelle, la méthode comprenant l'administration, à un sujet atteint de la maladie, de la nanoparticule de lipide, ledit ARNm codant pour la protéine fonctionnelle ou une protéine ayant la même activité biologique que la protéine fonctionnelle.

14. Nanoparticule lipidique telle que définie dans la revendication 11, dans laquelle l'agent thérapeutique est un ARNsi, destinée à être utilisée dans une méthode de traitement d'une maladie **caractérisée par** une surexpression d'un polypeptide, la méthode comprenant l'administration, à un sujet atteint de la maladie, de la nanoparticule lipidique, ledit ARNsi ciblant l'expression du polypeptide surexprimé.

15. Nanoparticule lipidique telle que définie dans la revendication 11, dans laquelle l'agent thérapeutique est un ARNm, ou composition pharmaceutique telle que définie dans la revendication 12 et qui est formulée pour une administration intramusculaire, destinée à être utilisée dans une méthode d'administration d'un vaccin à un sujet qui en a besoin, comprenant l'administration, à un sujet, d'une quantité thérapeutiquement efficace de la nanoparticule lipidique ou de la composition pharmaceutique.
